(19) Europäisches Patentamt
European Patent Office
Office européen des brevets

(11) Numéro de publication: **0 110 434**
**B1**

# (12) FASCICULE DE BREVET EUROPEEN

(45) Date de publication du fascicule du brevet: **29.10.86**

(51) Int. Cl.[4]: **C 07 J 41/00**, C 07 J 43/00, C 07 J 51/00, C 07 J 71/00

(21) Numéro de dépôt: **83201391.6**

(22) Date de dépôt: **08.01.82**

(60) Numéro de publication de la demande initiale en application de l'article 76 CBE: **0057115**

## (54) Nouveaux produits intermédiaires stéroides substitués en 11-beta et procédé de préparation de ces produits.

(30) Priorité: **09.01.81 FR 8100272**

(43) Date de publication de la demande: **13.06.84 Bulletin 84/24**

(45) Mention de la délivrance du brevet: **29.10.86 Bulletin 86/44**

(84) Etats contractants désignés: **AT BE CH DE FR GB IT LI LU NL SE**

(56) Documents cités: **FR - A - 2 377 417**

(73) Titulaire: **ROUSSEL-UCLAF, 35, boulevard des Invalides, F-75007 Paris (FR)**

(72) Inventeur: **Teutsch, Jean Georges, Résidence Lavoisier Bât. 3 3, rue Lavoisier, F-93500 Pantin (FR)**
Inventeur: **Costerousse, Germain, 10, rue des Réservoirs, F-94410 Saint-Maurice (FR)**
Inventeur: **Philibert, Daniel, 16, rue Chevalier, F-94210-La Varenne Saint-Hilaire (FR)**
Inventeur: **Deraedt, Roger, 23, allée Jean-Baptiste Clément, F-93320-Pavillons-sous-Bois (FR)**

(74) Mandataire: **Bourgouin, André et al, Département des Brevets ROUSSEL UCLAF B.P. no 9, F-93230 Romainville (FR)**

EP 0 110 434 B1

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

**Description**

La présente invention concerne de nouveaux produits intermédiaires 5 substitués en 11 β et le procédé de préparation de ces produits.

Dans le brevet français FR-A 2 377 417 sont décrits des stéroïdes comprenant, en position 3, un radical cétonique bloqué, en position 5α un radical hydroxy, en position 9 (10) une double liaison et en position 11 β un radical $R_1$, $R_1$ étant un radical alcoyle linéaire ou ramifié renfermant de 1 à 12 atomes de carbone ou un radical alcoyle insaturé renfermant de 2 à 8 atomes de carbone éventuellement substitué, un radical aralcoyle renfermant de 7 à 13 atomes de carbone éventuellement substitué ou un radical hétérocyclique renfermant un ou plusieurs atomes de soufre ou d'oxygène.

Cependant, le brevet français précité ne décrit aucun produit dans lequel le substituant $R_1$ comporte un atome d'azote.

L'invention a pour objet les produits de formule II:

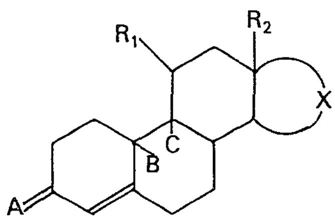

(II)

dans laquelle K représente un groupement cétonique bloqué sous forme de cétal, de thiocétal, d'oxime ou de méthyloxime, $R_1$ représente un radical organique renfermant de 1 à 18 atomes de carbone, contenant au moins un atome d'azote, de phosphore ou de silicium, l'atome immédiatement adjacent au carbone en 11 étant un atome de carbone, $R_2$ représente un radical hydrocarboné renfermant de 1 à 8 atomes de carbone, X représente le reste d'un cycle pentagonal ou hexagonal éventuellement substitué et éventuellement porteur d'insaturation.

$R_2$ représente de préférence un radical alkyle saturé, linéaire ou ramifié, renfermant de 1 à 4 atomes de carbone, par exemple un radical méthyle, éthyle, n-propyle ou butyle.

X représente de préférence le reste d'un cycle pentagonal éventuellement substitué.

L'invention a plus particulièrement pour objet les composés de formule (II), pour lesquels $R_2$ représente un radical méthyle.

L'invention a tout spécialement pour objet les composés de formule (I), pour lesquels X représente le reste d'un cycle de formule:

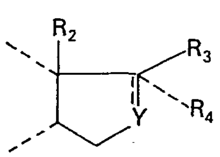

dans lequel $R_2$ conserve la même signification que précédemment, le trait pointillé en 16–17 symbolyse la présence éventuelle d'une double liaison, Y représente un radical

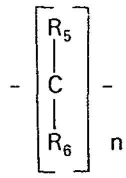

dans lequel n représente le nombre 1 ou 2, $R_5$ représente un atome d'hydrogène, un radical alkyle renfermant de 1 à 8 atomes de carbone, un radical alkényle ou alkynyle renfermant de 2 à 8 atomes de carbone, un radical aryle renfermant de 6 à 14 atomes de carbone ou un radical aralkyle renfermant de 7 à 15 atomes de carbone, $R_6$, identique ou différent de $R_5$, peut prendre l'une des valeurs indiquées pour $R_5$ et peut également représenter un radical hydroxyle, $R_3$ et $R_4$, identiques ou différents, représentent soit un atome d'hydrogène, soit un radical OH, $Oalc_4$, O-CO-$alc_5$, $alc_4$ et $alc_5$ représentant un radical alkyle renfermant de 1 à 8 atomes de carbone ou aralkyle renfermant de 7 à 15 atomes de carbone, soit un radical alkényle ou alkynyle renfermant de 2 à 8 atomes de carbone, soit un radical

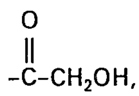

soit un radical $-COCH_2OCOalc_6$, dans lequel $alc_6$ représente un radical alkyle renfermant de 1 à 8 atomes de carbone éventuellement substitué ou un radical aralkyle renfermant de 7 à 15 atomes de carbone, soit un radical $CO-CO_2H$ ou $CO-CO_2alc_7$ dans lequel $alc_7$ représente un radical alkyle renfermant de 1 à 8 atomes de carbone, soit un radical

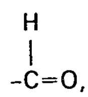

soit un radical

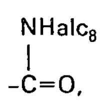

dans lequel $alc_8$ représente un radical alkyle renfermant de 1 à 8 atomes de carbone ou un radical aralkyle renfermant de 7 à 15 atomes de carbone, soit un radical $-C{\equiv}N$, soit $R_3$ et $R_4$ forment ensemble un radical

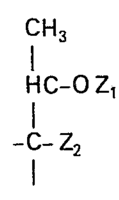

dans lequel $Z_1$ représente un atome d'hydrogène, un radical alkyle ou un radical acyle renfermant

de 1 à 8 atomes de carbone et $Z_2$ un radical alkyle renfermant de 1 à 8 atomes de carbone.

$R_5$ est de préférence différent de $R_6$.

Lorsque $R_5$ ou $R_6$ représente un radical akyle, il s'agit de préférence du radical méthyle ou éthyle.

Lorsque $R_5$ ou $R_6$ représente un radical alkényle, il s'agit de préférence du radical vinyle, isopropényle ou allyle.

Lorsque $R_5$ ou $R_6$ représente un radical alkynyle, il s'agit de préférence du radical éthynyle ou propynyle.

Lorsque $R_5$ ou $R_6$ représente un radical aryle ou aralkyle, il s'agit de préférence du radical phényle ou benzyle.

Lorsque $R_3$ ou $R_4$ représente un radical $Oalc_4$ ou $OCOalc_5$, $alc_4$ et $alc_5$ représentent de préférence un radical méthyle, éthyle, n-propyle, butyle, pentyle, hexyle ou benzyle.

Lorsque $R_3$ ou $R_4$ représente un radical alkényle, il s'agit de préférence du radical vinyle, isopropényle, allyle ou 2-méthylallyle.

Lorsque $R_3$ ou $R_4$ représente un radical alkynyle, il s'agit de préférence du radical $-C{\equiv}CH$, ou $-C{\equiv}C\text{-}alc_9$, $alc_9$ représentant de préférence un radical méthyle, éthyle, propyle, isopropyle, isopropényle, butyle, benzyle ou trifluorométhyle.

$Alc_6$, $alc_7$ et $alc_8$ représentent de préférence une des valeurs préférentielles de $alc_4$ ou $alc_5$.

Les composés préférés sont ceux pour lesquels les radicaux $R_3$ et $R_4$ sont différents sauf dans le cas où $R_3$ et $R_4$ représentent chacun un atome d'hydrogène.

Parmi les valeurs préférées du radical

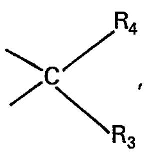
,

on peut citer les radicaux:

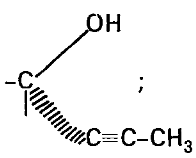
;
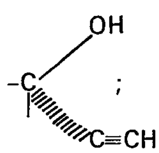
;

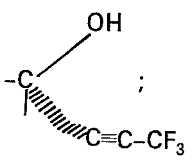
;
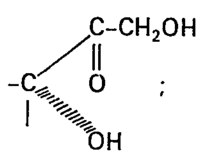
;

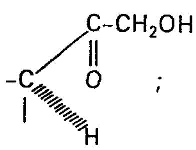
;
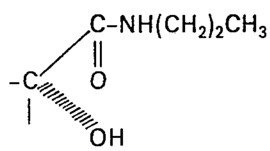
ou

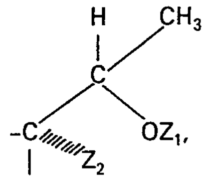

dans lequel $Z_1$ represente un atome d'hydrogène, un radical alkyl renfermant 1 à 8 atomes de carbone ou un radical acyl renfermant de 2 à 8 atomes de carbone, par exemple un radical acétyloxy ou benzoyl et $Z_2$ représente un radical alkyle renfermant de 1 à 8 atomes de carbone, par exemple un radical méthyle.

L'invention a notamment pour objet les composés de formule (II) pour lesquels le cycle D ne porte pas d'insaturation éthylènique, $R_5$ et $R_6$ représentent un atome d'hydrogène et n est égal à 1.

L'invention a notamment pour objet les produits répondant à la formule (V):

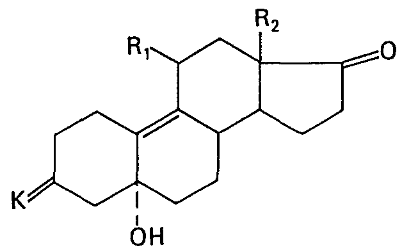
(V)

dans laquelle K, $R_1$ et $R_2$ conservent la même signification que précédemment ou à la formule (II'):

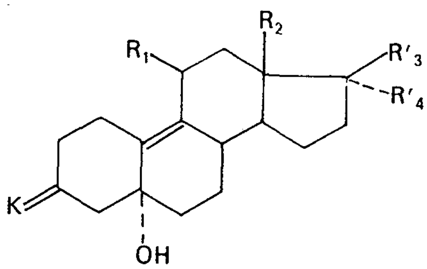
(II')

dans lequel $R_1$, $R_2$ et K sont définis comme précédemment 1, $R'_3$ représente un radical hydroxy ou un radical $OR_e$, dans lequel $R_e$ représente le reste $alc_4$ d'un groupement éther ou $COalc_5$ d'un groupement ester, $alc_4$ et $alc_5$ étant définis comme à la revendication 1 et $R'_4$ représente un atome d'hydrogène ou un radical alkényle ou alkynyle comportant de 2 à 8 atomes de carbone.

L'invention a plus particulièrement pour objet les composés de formule (II) pour lesquels $R_1$ représente un radical hydrocarboné renfermant de 1 à 18 atomes de carbone et contenant au moins un atome d'azote.

Parmi ces composés, on peut citer tout spécialement les composés pour lesquels $R_1$ représente un radical alkyle primaire, secondaire ou tertiaire, renfermant de 1 à 8 atomes de carbone, comportant un ou plusieurs hétéroatomes choisis dans le

groupe constitué par l'oxygène, l'azote et le soufre, dont au moins un atome d'azote, ou substitué par un hétérocycle renfermant au moins un atome d'azote.

Par radical alkyle, on entend de préférence, les radicaux méthyle, éthyle, n-propyle, isopropyle, butyle, isobutyle, terbutyle, pentyle, hexyle, cyclopropyle, cyclobutyle, cyclopentyle ou cyclohexyle.

Par hétérocycle renfermant au moins un atome d'azote, on peut citer les radicaux 3,4- ou 2-pyridyle, les radicaux thiazolyle ou pipéridinyle.

On peut également citer comme composés poréférés de l'invention, les composés pour lesquels $R_1$ représente un radical hétérocyclique comportant au moins un atome d'azote, éventuellement substitué par un radical alkyle renfermant de 1 à 8 atomes de carbone.

Par radical hétérocyclique, on entend de préférence l'un des radicaux mentionnés ci-dessus.

Lorsque $R_1$ représente un radical hétérocyclique comportant au moins un atome d'azote, substitué par un radical alkyle, il s'agit le plus souvent d'un hétérocycle substitué par un radical méthyle, éthyle ou n-propyle.

Parmi les composés préférés de l'invention, on peut citer également les composés, pour lesquels $R_1$ représente un radical aryle ou aralkyle portant une fonction amine

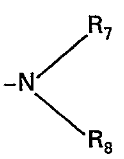 ,

dans laquelle $R_7$ et $R_8$ représentent un radical alkyle renfermant de 1 à 8 atomes de carbone, ou un radical alkyle primaire, secondaire ou tertiaire renfermant de 1 à 8 atomes de carbone, comportant un ou plusieurs hétéroatomes choisis dans le groupe constitué par l'oxygène, l'azote et le soufre, dont au moins un atome d'azote, ou substitué par un hétérocycle comportant au moins un atome d'azote.

Par radical alkyle, on entend de préférence l'un des radicaux mentionnés ci-dessus.

Par radical aryle ou aralkyle, on entend de préférence le radical phényle ou benzyle.

Par radical hétérocyclique, on entend de préférence l'un des radicaux hétérocycliques mentionnés ci-dessus.

L'invention a tout spécialement pour objet les composés pour lesquels $R_1$ représente un radical 2,3- ou 4-pyridyle,

un radical $-(CH_2)_n-$ 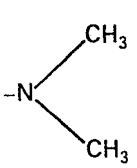 $(\geqslant 3)$ ,

un radical 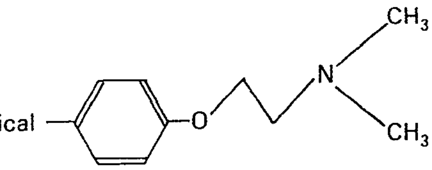

un radical 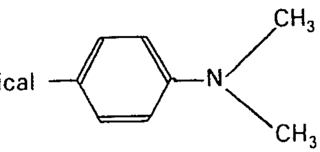

un radical 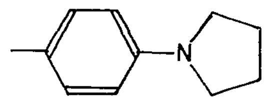 ou

un radical 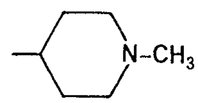

L'invention a également en particulier pour objet les composés pour lesquels $R_1$ représente un radical

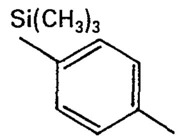

Parmi les composés de l'invention, on peut encore citer les composés dans lesquels $R_1$ comporte un atome d'azote oxydé.

Parmi les composés préférés de l'invention, on peut tout naturellement citer les composés dont la préparation est donnée plus loin dans la partie expérimentale et notamment les composés dont les noms suivent:

- le 11β-[4-(triméthylsilyl) phényl] 3,3-[1,2-éthane diyl bis (oxy)] 17α-(prop-1-ynyl) estr-9-èn 5α,17β-diol,
- le 11β-(4-pyridyl) 3,3-[1,2-éthane diyl bis (oxy)] 17α-(prop-1-ynyl) estr-9-èn 5α,17β-diol,
- le 11β-[3-(N,N-diméthylamino)propyl] 3,3-[1,2-éthane diyl bis (oxy)] 17α-(prop-1-ynyl) estr-9-èn 5α,17β-diol,
- le 11β-(4-diméthylaminophényl) 3,3-[1,2-éthane diyl bis (oxy)] 17α-(prop-1-ynyl) estr-9-èn 5α,17β-diol,
- le 3,3-[éthane diyl bis (oxy)] 11β-[4-(N,N-diméthylaminoéthyloxy) phényl] 17α-(prop-1-ynyl) estr-9-èn 5α,17β-diol,
- le 21-chloro 3,3-[1,2-éthane diyl bis (oxy)] 11β-(4-diméthylaminophényl) (17α) 19-nor pregn-9-èn-20-yn 5α,17β-diol,
- le 11β-(4-diméthylaminophényl) 3,3-[1,2-éthane diyl bis (oxy)] 17α-(prop-2-ynyl) estr-9-èn 5α,17β-diol.

L'invention a également pour objet un procédé de préparation des composés de formule (II), caractérisé en ce que l'on soumet un composé de formule générale (III):

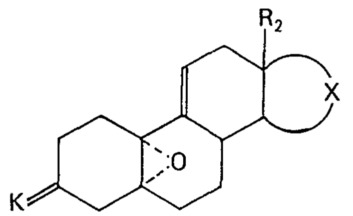

(III)

à l'action d'un composé choisi dans le groupe constitué par les composés de formule $(R_1)_2$ Cu Li, de formule $R_1$Mg Hal et de formule $R_1$Li, dans laquelle $R_1$ conserve la même signification que précédemment et Hal représente un atome d'halogène le cas échéant en présence d'halogénure cuivreux pour obtenir le composé de formule (II) correspondant.

Dans un mode de réalisation préféré du procédé de l'invention:

- la réaction a lieu à la température ambiante,
- on soumet le composé de formule (III) à l'action d'un composé de formule $R_1$Mg Hal en présence de sels cuivreux.

L'invention a également pour objet un procédé de préparation tel que défini précédemment, caractérisé en ce que le produit de départ, répondant à la formule (II'):

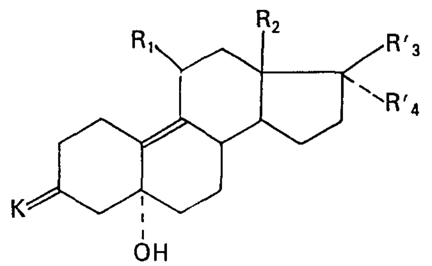

(II')

dans laquelle $R_1$, $R_2$ et K sont définis comme précédemment, $R'_3$ représente un radical hydroxy ou un radical $OR_e$ dans lequel $R_e$ représente le reste $alc_4$ d'un groupement éther ou $COalc_5$ d'un groupement ester, $alc_4$ et $alc_5$ étant définis comme précédemment et $R'_4$ représente un atome d'hydrogène ou un radical alkényle ou alkynyle comportant de 2 à 8 atomes de carbone, est préparé en soumettant un composé de formule (IV):

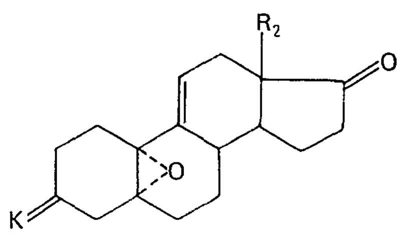

(IV)

à l'action d'un composé choisi dans le groupe constitué par les composés de formule $(R_1)_2$ Cu Li, de formule $R_1$Mg Hal et de formule $R_1$Li, dans laquelle $R_1$ et Hal sont définis comme précédemment, le cas échéant en présence d'halogénure cuivreux, pour obtenir le composé de formule (V):

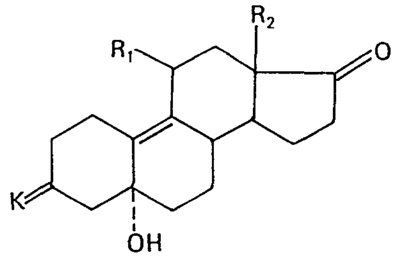

(V)

que l'on soumet soit à l'action d'un agent de réduction, pour obtenir le composé 17-hydroxy correspondant, soit à l'action d'un magnésien approprié, pour obtenir le composé 17β-hydroxy 17α-substitué correspondant, soit à l'action d'un dérivé organométallique tel qu'un lithien ou un dérivé de potassium, pour obtenir le composé 17β-hydroxy 17α-substitué correspondant, soit a) à l'action d'un agent de cyanuration, pour obtenir le composé 17β-cyano 17α-hydroxy correspondant, dont on protège la fonction hydroxy, puis b) à l'action d'un dérivé organométallique tel que décrit précédemment, pour obtenir le composé 17β-hydroxy 17α-substitué correspondant, et que, le cas échéant, l'on soumet l'un ou l'autre des composés 17-hydroxy obtenus ci-dessus, à l'action d'un agent d'estérification ou d'éthérification, et que, le cas échéant, l'on soumet l'un ou l'autre des composés 17-substitués obtenus ci-dessus, dans lesquels le substituant en 17 comporte une triple liaison, à l'action d'un agent de réduction, pour obtenir l'éthylènique correspondant.

Dans un mode de réalisation préféré du procédé de l'invention, la réaction du composé (IV) avec le composé $R_1$Mg Hal, $(R_1)_2$ Cu Li ou $R_1$Li est effectuée dans les conditions déjà décrites précédemment.

Les différents réactifs que l'on fait réagir sur le composé de formule (V) sont bien connus dans la chimie des stéroïdes. La partie expérimentale ci-après, décrit quelques-unes des réactions avec le composé de formule (V).

Les composés de formule (III) et notamment, parmi ceux-ci, les composés de formule (IV), utilisés pour préparer les composés de formule (II) ou (V) sont des produits connus d'une façon générale, qui peuvent être préparés en soumettant les composés Δ5(10), 9(11) correspondants à l'action d'un agent d'époxydation sélectif de la double liaison 5(10). C'est ainsi que l'on peut soumettre les composés Δ5(10) 9(11) par exemple à l'action de l'eau oxygénée utilisée en présence d'hexachloroacétone ou d'hexafluoroacétone, selon le procédé décrit et revendiqué dans le brevet français 2 423 486. Le 3,3-[1,2-éthane diyl bis (oxy)] 17α-(1-propynyl) estr-9 (11)-èn 5α,10α-époxy 17β-ol est un produit non décrit, dont la préparation est donnée plus loin dans la partie expérimentale.

L'invention a donc également pour objet ce produit à titre de produit chimique nouveau et plus particulièrement à titre de produit intermédiaire nécessaire à la mise en œuvre du procédé de l'invention.

Les produits de formule II, objets de la présente invention, sont des intermédiaires nécessaires pour la préparation des produits de formule I'.

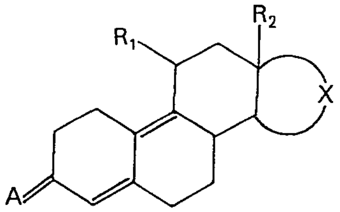

(I')

dans laquelle $R_1$, $R_2$ et X ont la signification indiquée précédemment le groupement C=A en position 3 représente un groupement oxo, libre ou bloqué sous forme de cétal, un groupement

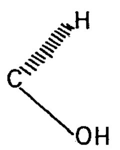
,
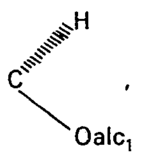
,
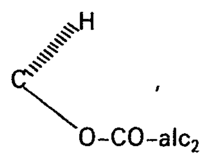
,

un groupement C=NOH, un groupement C=NO-$alc_3$ ou un groupement $CH_2$, $alc_1$, $alc_2$ et $alc_3$ représentant un radical alkyle renfermant de 1 à 8 atomes de carbone ou un groupement aralkyle renfermant de 7 à 15 atomes de carbone et B et C forment ensemble une double liaison ou un pont époxyde.

Le procédé permettant d'obtenir les produits de formule I' à partir des produits de formule II est le suivant.

On soumet un produit de formule II à l'action d'un agent de déshydratation susceptible de libérer la fonction cétone, pour obtenir un composé de formule ($I'_A$):

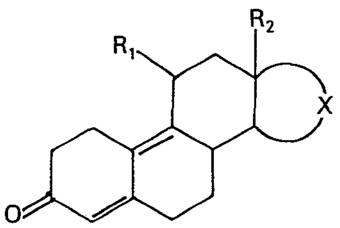

($I'_A$)

que l'on soumet, le cas échéant,
soit à l'action d'un agent de cétalisation pour obtenir le composé de formule ($I'_B$) dans laquelle la fonction cétone en 3 est bloquée sous forme de cétal,

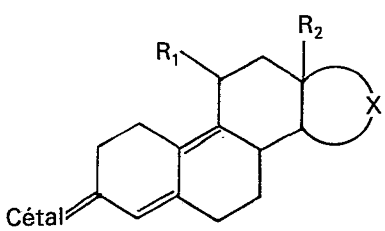

($I'_B$)

soit à l'action de l'hydroxylamine $NH_2OH$ libre, ou bloquée sous forme $NH_2$-O-$alc_3$ dans laquelle $alc_3$ conserve sa signification précédente, pour obtenir le composé de formule ($I'_C$)

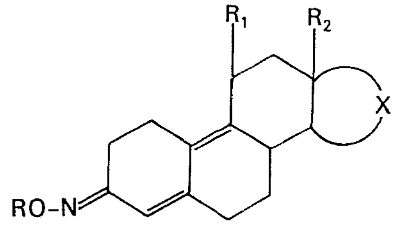

($I'_C$)

dans laquelle R représente un atome d'hydrogène ou un groupement $alc_3$,
soit à l'action d'un agent de réduction capable de réduire sélectivement la fonction cétone en 3, pour obtenir le composé de formule ($I'_D$)

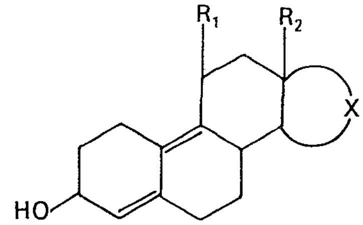

($I'_D$)

que l'on soumet, le cas échéant, ou bien à l'action d'un agent d'éthérification susceptible d'introduire le radical $alc_1$ pour obtenir un composé de formule ($I'_E$)

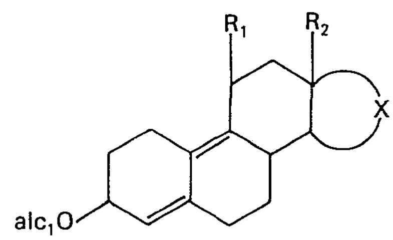

($I'_E$)

ou bien à l'action d'un agent d'estérification susceptible d'introduire le groupement CO $alc_2$ dans lequel $alc_2$ conserve la signification précédente, pour obtenir un composé de formule ($I'_F$)

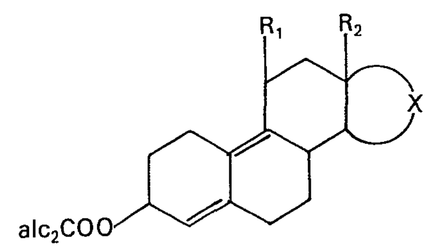

($I'_F$)

ou, composé de formule ($I'_A$), que l'on transforme, le cas échéant, selon les méthodes connues, en dérivé pour lequel C=A représente un groupement $CH_2$ et, composé de formule ($I'_A$), ($I'_B$), ($I'_C$), ($I'_D$), ($I'_E$) ou ($I'_F$) que, le cas échéant, l'on soumet à l'action d'un acide pour obtenir un sel, ou à l'action d'un agent d'oxydation, pour obtenir soit, si le radical $R_1$ comporte un atome d'azote, un dérivé comportant en 11β un radical dont l'atome

d'azote est oxydé et dans lequel les radicaux B et C forment éventuellement un pont époxyde, soit, si le radical $R_1$ ne comporte pas d'atome d'azote, un dérivé dans lequel les radicaux B et C forment un pont époxyde, et, composé dans lequel à la fois le radical $R_1$ comporte un atome d'azote oxydé et B et C forment ensemble un pont époxyde que, le cas échéant, l'on réduit sélectivement au niveau de l'atome d'azote oxydé contenu dans le radical $R_1$ et que, les cas échéant, l'on soumet à l'action d'un acide pour obtenir un sel.

Dans un mode de réalisation préféré du procédé de préparation des produits de formule I' l'agent de déshydratation capable de libérer la fonction cétone est une résine sulfonique (forme acide), par exemple, une résine sulfonique du commerce à support de polystyrène ou à support de polymère styrène/divinylbenzène; mais on peut également utiliser un acide minéral tel que l'acide chlorhydrique ou l'acide sulfurique dans un alcanol inférieur ou l'acide perchlorique dans l'acide acétique, ou un acide sulfonique comme l'acide paratoluène sulfonique.

L'agent de cétalisation est de préférence un alcool ou un dialcool en présence d'un acide organique, comme par exemple l'acide oxalique ou l'acide paratoluènesulfonique.

L'agent de réduction de la fonction cétone est de préférence un hydrure de métal alcaline. (Voir à ce sujet l'article de E.R. Walkis dans Chemical Society Reviews 1976, vol. 5, n° 1, page 23.)

L'agent d'éthérification est de préférence un halogénure d'alcoyle en présence d'une base.

L'agent d'estérification est de préférence un dérivé d'acide carboxylique, par exemple un chlorure ou un anhydride, en présence d'une base telle que la pyridine.

Il va de soi que lorsque l'un des radicaux $R_3$ ou $R_4$ des produits de formule (I') obtenus précédemment représente un radical OH, on peut soumettre ledit radical OH de ces produits de formule (I'), à l'action d'un agent d'éthérification ou d'estérification.

Cet agent d'éthérification ou d'estérification est de préférence l'un de ceux qui ont été mentionnés ci-dessus.

Lorsque $R_3$ ou $R_4$ représentent un radical acyloxy en 17, on peut éventuellement saponifier ce groupement acyloxy. L'agent de saponification utilisé est de préférence une base comme la soude, la potasse, l'amidure de potassium ou le terbutylate de potassium, la réaction de saponification étant réalisée de préférence au sein d'un alcool inférieur comme le méthanol ou l'éthanol, il peut être également l'acétylure de lithium dans l'éthylène diamine.

L'agent d'oxydation est de préférence un peracide comme l'acide métachloroperbenzoïque, l'acide paracétique ou l'acide perphtalique ou encore l'eau oxygénée seule ou en présence d'hexachloro ou d'hexafluoroacétone. Lorsque l'on désire obtenir un composé dans lequel seul l'atome d'azote du radical $R_1$ est oxydé, on utilise un équivalent d'agent d'oxydation. Lorsque l'on désire obtenir un composé dans lequel, en outre, B et C forment un pont époxyde, on utilise deux équivalents d'agent d'oxydation.

L'agent de réduction sélectif de la fonction N-oxyde est de préférence la triphénylphosphine et l'on peut opérer par exemple au sein de l'acide acétique.

Les composés de formule (I') ainsi que leurs sels d'addition avec les acides pharmaceutiquement acceptables sont des produits particulièrement intéressants du point de vue pharmacologique; ils possèdent en particulier une remarquable activité antiglucocorticoïde.

L'étude des produits sur les récepteurs hormonaux a permis de mette en évidence des activités progestomimétiques, androgènes ou anti-androgènes.

Les produits suivants constituent des produits pouvant être obtenus à partir des produits de formule II objets de la présente invention.

A) Les produits de formule:

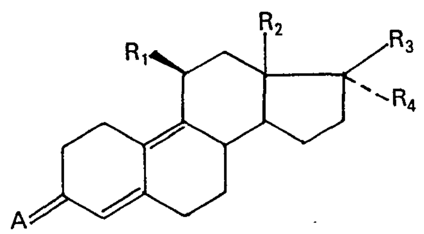

dans laquelle les substituants A, $R_1$, $R_2$, $R_3$ et $R_4$ ont les significations suivantes. (Le sigle " signifie que le substituant est le même que celui qui précède.)

| A | $R_1$ | $R_2$ | $R_3$ | $R_4$ |
|---|---|---|---|---|
| O | 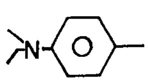 | $CH_3$ | OH | $-C{\equiv}C-H$ |
| O | 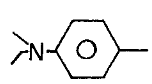 | $CH_3$ | OH | $-C{\equiv}C-CF_3$ |
| O | 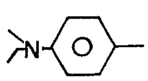 | $CH_3$ | OH | $-C{\equiv}C-CH_2CH_3$ |
| O | 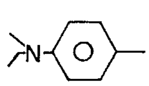 | $CH_3$ | OH | $-CH_2-C{\equiv}C-H$ |

| A | $R_1$ | $R_2$ | $R_3$ | $R_4$ |
|---|---|---|---|---|
| O | 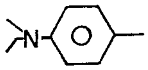 | $CH_3$ | OH | $-C{\equiv}C-SiMe_3$ |
| O | 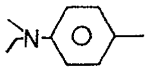 | $CH_3$ | $-C{\equiv}C-H$ | OH |
| O | 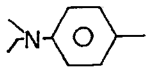 | $CH_3$ | $-C{\equiv}C-SiMe_3$ | OH |
| O | 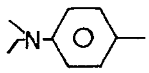 | $CH_2CH_3$ | OH | $-C{\equiv}C-H$ |
| O | 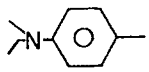 | $CH_2CH_3$ | OH | $-C{\equiv}CH_3$ |
| O | 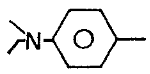 | $CH_2CH_3$ | OH | $-CH_2-C{\equiv}C-H$ |
| O | 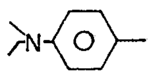 | $CH_3$ | $-C-CH_2OH$<br>‖<br>O | H |
| O | 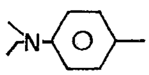 | $CH_3$ | $-C-CH_2OH$<br>‖<br>O | OH |
| HO–N=(E) | 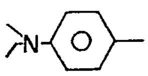 | $CH_3$ | OH | $-C{\equiv}C-H$ |
| HO–N=(E) | 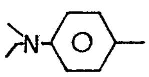 | $CH_3$ | OH | $-C{\equiv}C-CH_3$ |
| HO–N=(E) | 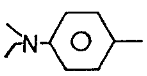 | $CH_3$ | $-C{\equiv}C-H$ | OH |
| HO–N=(E) | 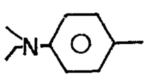 | $CH_3$ | OH | $-CH_2-C{\equiv}C-H$ |
| HO–N=(Z) | 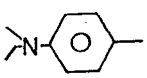 | $CH_3$ | OH | $-C{\equiv}C-H$ |
| HO–N=(Z) | 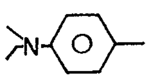 | $CH_3$ | OH | $-C{\equiv}C-CH_3$ |
| HO–N=(Z) | 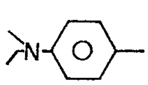 | $CH_3$ | OH | $-CH_2-C{\equiv}C-H$ |
| HO–N=(Z) | 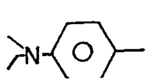 | $CH_3$ | $-C{\equiv}C-H$ | OH |
| O | 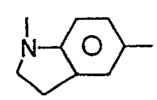 | $CH_3$ | OH | $-C{\equiv}C-H$ |
| O | 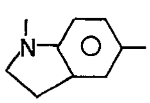 | $CH_3$ | OH | $-C{\equiv}C-CF_3$ |
| O | 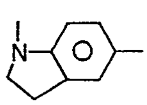 | $CH_3$ | OH | $-CH_2-C{\equiv}C-H$ |

| A | $R_1$ | $R_2$ | $R_3$ | $R_4$ |
|---|---|---|---|---|
| O | 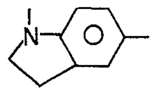 | $CH_3$ | OH | $-C{\equiv}C-CH_2CH_3$ |
| O | 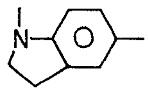 | $CH_3$ | OH | $-C{\equiv}C-Cl$ |
| O | 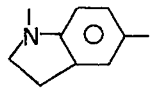 | $CH_3$ | OH | $-C{\equiv}C-SiMe_3$ |
| O | 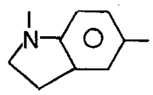 | $CH_3$ | $-C{\equiv}C-H$ | OH |
| O | 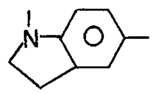 | $CH_3$ | $-C{\equiv}C-SiMe_3$ | OH |
| O | 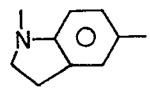 | $CH_2CH_3$ | OH | $-C{\equiv}C-H$ |
| O | 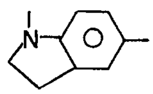 | $CH_2CH_3$ | OH | $-C{\equiv}C-CH_3$ |
| O | 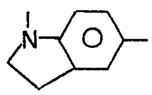 | $CH_2CH_3$ | OH | $-CH_2-C{\equiv}C-H$ |
| O | 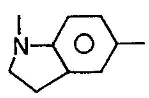 | $CH_3$ | $-C(=O)-CH_2OH$ | -H |
| O | 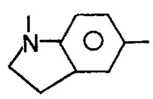 | $CH_3$ | $-C(=O)-CH_2OH$ | -OH |
| O | 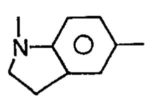 | $CH_3$ | $-C(=O)-CH_3$ | -H |
| HO-N=(E) | 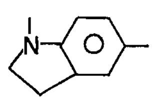 | $CH_3$ | -OH | $-C{\equiv}C-H$ |
| HO-N=(E) | 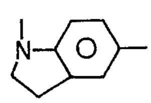 | $CH_3$ | -OH | $-C{\equiv}C-CH_3$ |
| HO-N=(E) | 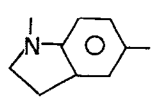 | $CH_3$ | -OH | $-C{\equiv}C-CH_2CH_3$ |
| HO-N=(E) | 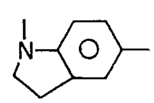 | $CH_3$ | -OH | $-CH_2-C{\equiv}C-H$ |

| A | $R_1$ | $R_2$ | $R_3$ | $R_4$ |
|---|---|---|---|---|
| HO–N=(E) | 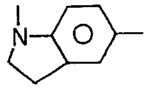 | $CH_3$ | $-C{\equiv}C-H$ | $-OH$ |
| HO–N=(E) | 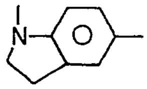 | $CH_3$ | $-\overset{O}{\overset{\|}{C}}-CH_2OH$ | H |
| HO–N=(Z) | 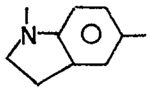 | $CH_3$ | $-\overset{O}{\overset{\|}{C}}-CH_2OH$ | H |
| HO–N=(Z) | 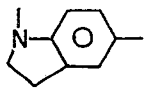 | $CH_3$ | OH | $-C{\equiv}C-H$ |
| HO–N=(Z) | 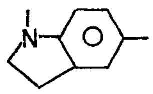 | $CH_3$ | OH | $-C{\equiv}C-CH_3$ |
| HO–N=(Z) | 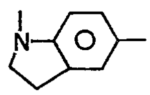 | $CH_3$ | OH | $-C{\equiv}C-CH_2CH_3$ |
| HO–N=(Z) | 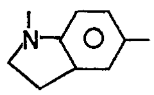 | $CH_3$ | OH | $-CH_2-C{\equiv}C-H$ |
| HO–N=(Z) | 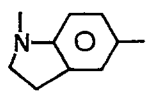 | $CH_3$ | $-C{\equiv}C-H$ | $-OH$ |
| O | 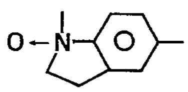 | $CH_3$ | $-C{\equiv}C-H$ | $-OH$ |
| O | 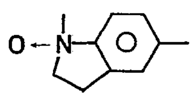 | $CH_3$ | OH | $-C{\equiv}C-H$ |
| O | 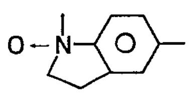 | $CH_3$ | OH | $-C{\equiv}C-CF_3$ |
| O | 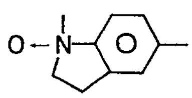 | $CH_3$ | OH | $-C{\equiv}C-Cl$ |
| O | 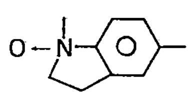 | $CH_3$ | OH | $-C{\equiv}C-CH_2CH_3$ |
| O | 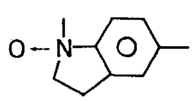 | $CH_3$ | OH | $-CH_2-C{\equiv}C-H$ |
| O | 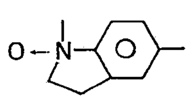 | $CH_3$ | OH | $-C{\equiv}C-SiMe_3$ |

| A | $R_1$ | $R_2$ | $R_3$ | $R_4$ |
|---|---|---|---|---|
| O | 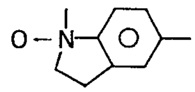 | $CH_3$ | $-C(=O)-CH_2OH$ | $-H$ |
| HO-N=(E) | 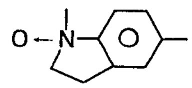 | $CH_3$ | OH | $-C{\equiv}C-H$ |
| HO-N=(E) | 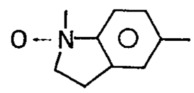 | $CH_3$ | OH | $-C{\equiv}C-CH_3$ |
| HO-N=(E) | 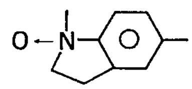 | $CH_3$ | OH | $-CH_2-C{\equiv}C-H$ |
| HO-N=(E) | 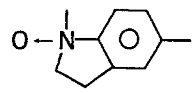 | $CH_3$ | $-C{\equiv}C-H$ | $-OH$ |
| HO-N=(Z) | 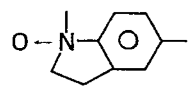 | $CH_3$ | $-C{\equiv}C-H$ | $-OH$ |
| HO-N=(Z) | 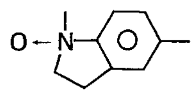 | $CH_3$ | OH | $-C{\equiv}C-H$ |
| HO-N=(Z) | 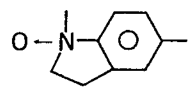 | $CH_3$ | OH | $-C{\equiv}C-CH_3$ |
| HO-N=(Z) | 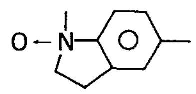 | $CH_3$ | OH | $-CH_2-C{\equiv}C-H$ |
| O | 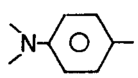 | $CH_3$ | OH | $-C{\equiv}C-CH_2CH_3$ |
| O | 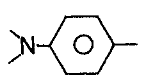 | $CH_3$ | OH | $-C{\equiv}C-CF_3$ |
| O | 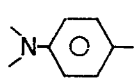 | $CH_3$ | $-C{\equiv}C-SiMe_3$ | $-OH$ |
| O | 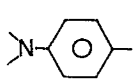 | $CH_3$ | $-C(=O)-CH_2OH$ | $-H$ |
| O | 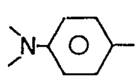 | $CH_3$ | $-C(=O)-CH_2OH$ | $-OH$ |
| O | 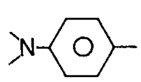 | $CH_3$ | $-C(=O)-CH_3$ | $-H$ |
| O | 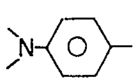 | $CH_2CH_3$ | OH | $-C{\equiv}C-H$ |

| A | $R_1$ | $R_2$ | $R_3$ | $R_4$ |
|---|---|---|---|---|
| O | 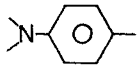 | $CH_2CH_3$ | OH | $-C{\equiv}C-CH_3$ |
| O | 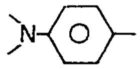 | $CH_2CH_3$ | OH | $-C{\equiv}C-Cl$ |
| O | 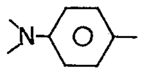 | $CH_2CH_3$ | OH | $-C{\equiv}C-CH_2-CH_3$ |
| O | 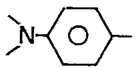 | $CH_2CH_3$ | OH | $-C{\equiv}C-SiMe_3$ |
| O | 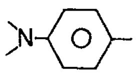 | $CH_2CH_3$ | OH | $-CH_2-C{\equiv}C-H$ |
| O | 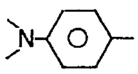 | $CH_2CH_3$ | $-C(=O)-CH_2OH$ | -H |
| HO-N=(E) | 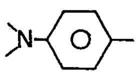 | $CH_3$ | $-C{\equiv}C-H$ | -OH |
| HO-N=(E) | 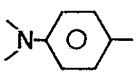 | $CH_3$ | $-C(=O)-CH_2OH$ | -H |
| HO-N=(E) | 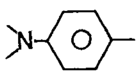 | $CH_3$ | OH | $-CH_2-C{\equiv}C-H$ |
| HO-N=(Z) | 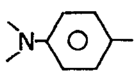 | $CH_3$ | OH | $-CH_2-C{\equiv}C-H$ |
| HO-N=(Z) | 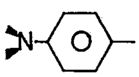 | $CH_3$ | $-C{\equiv}C-H$ | -OH |
| HO-N=(Z) | 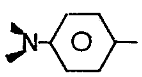 | $CH_3$ | $-C(=O)-CH_2OH$ | -H |
| O | 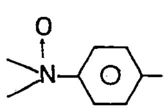 | $CH_3$ | $-C(=O)-CH_2OH$ | -H |
| O | 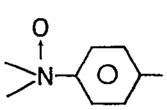 | $CH_3$ | $-C{\equiv}C-H$ | -OH |
| O | 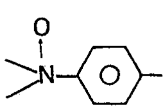 | $CH_3$ | OH | $-CH_2-C{\equiv}C-H$ |
| O | 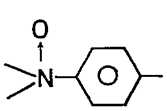 | $CH_3$ | OH | $-C{\equiv}C-CH_2CH_3$ |

| A | $R_1$ | $R_2$ | $R_3$ | $R_4$ |
|---|---|---|---|---|
| O | 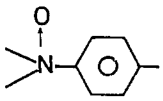 | $CH_3$ | OH | $-C{\equiv}C-CF_3$ |
| O | 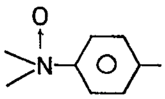 | $CH_3$ | OH | $-C{\equiv}C-H$ |
| O | 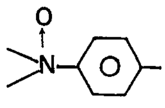 | $CH_3$ | OH | $-C{\equiv}C-SiMe_3$ |
| HO-N=(E) | 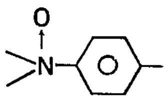 | $CH_3$ | OH | $-C{\equiv}C-H$ |
| HO-N=(E) | 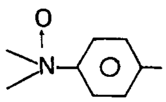 | $CH_3$ | OH | $-C{\equiv}C-CH_3$ |
| HO-N=(E) | 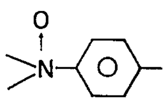 | $CH_3$ | OH | $-C{\equiv}C-CH_2CH_3$ |
| HO-N=(E) | 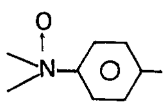 | $CH_3$ | OH | $-C{\equiv}C-Cl$ |
| HO-N=(E) | 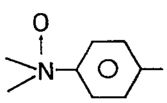 | $CH_3$ | OH | $-C{\equiv}C-SiMe_3$ |
| HO-N=(E) | 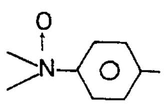 | $CH_3$ | OH | $-CH_2-C{\equiv}C-H$ |
| HO-N=(E) | 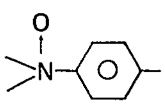 | $CH_3$ | $-C{\equiv}C-H$ | -OH |
| HO-N=(Z) | 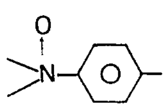 | $CH_3$ | $-C{\equiv}C-H$ | -OH |
| HO-N=(Z) | 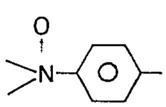 | $CH_3$ | OH | $-C{\equiv}C-H$ |
| HO-N=(Z) | 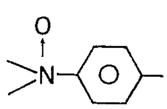 | $CH_3$ | OH | $-C{\equiv}C-CH_3$ |

| A | $R_1$ | $R_2$ | $R_3$ | $R_4$ |
|---|---|---|---|---|
| HO–N=(Z) | 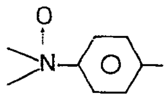 | $CH_3$ | OH | $-C{\equiv}C-CH_2-CH_3$ |
| HO–N=(Z) | 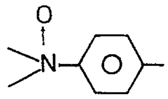 | $CH_3$ | OH | $-C{\equiv}C-Cl$ |
| HO–N=(Z) | 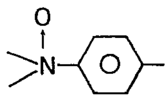 | $CH_3$ | OH | $-C{\equiv}C-SiMe_3$ |
| HO–N=(Z) | 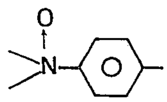 | $CH_3$ | OH | $-CH_2-C{\equiv}C-H$ |
| O | 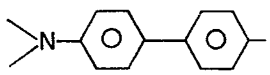 | $CH_3$ | OH | $-C{\equiv}C-H$ |
| O | 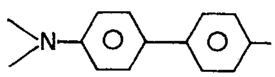 | $CH_3$ | OH | $-C{\equiv}C-CF_3$ |
| O | 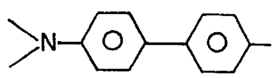 | $CH_3$ | OH | $-C{\equiv}C-CH_3$ |
| O | 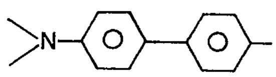 | $CH_3$ | OH | $-C{\equiv}C-Cl$ |
| O | 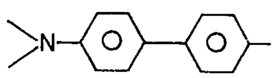 | $CH_3$ | OH | $-CH_2C{\equiv}C-H$ |
| O | 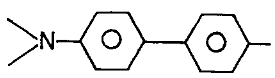 | $CH_3$ | OH | –H |
| O | 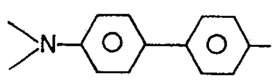 | $CH_3$ | $-C{\equiv}C-H$ | –OH |
| O | 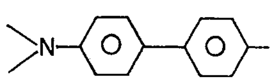 | $CH_3$ | $-\underset{\underset{O}{\|}}{C}-CH_2OH$ | –H |
| O | 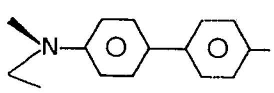 | $CH_3$ | $-\underset{\underset{O}{\|}}{C}-CH_2OH$ | –H |
| O | 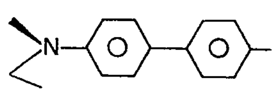 | $CH_3$ | OH | $-C{\equiv}C-H$ |
| O | 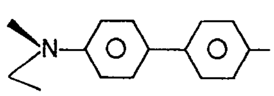 | $CH_3$ | OH | $-C{\equiv}C-CH_3$ |
| O | 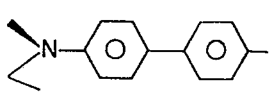 | $CH_3$ | OH | $-C{\equiv}C-Cl$ |

| A | $R_1$ | $R_2$ | $R_3$ | $R_4$ |
|---|---|---|---|---|
| O | 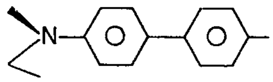 | $CH_3$ | OH | $-CH_2-C{\equiv}C-H$ |
| O | 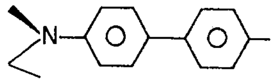 | $CH_3$ | $-C{\equiv}C-H$ | $-OH$ |
| O | 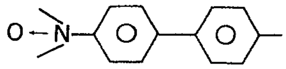 | $CH_3$ | $-C{\equiv}C-H$ | $-OH$ |
| O | 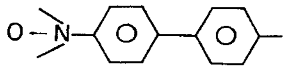 | $CH_3$ | OH | $-C{\equiv}C-H$ |
| O | 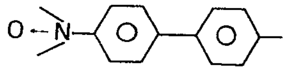 | $CH_3$ | OH | $-C{\equiv}C-CF_3$ |
| O | 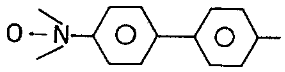 | $CH_3$ | OH | $-C{\equiv}C-CH_3$ |
| O | 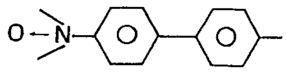 | $CH_3$ | OH | $-CH_2-C{\equiv}C-H$ |
| O | 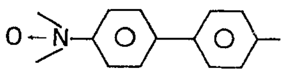 | $CH_3$ | OH | $-H$ |
| O | 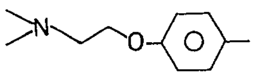 | $CH_3$ | OH | $-C{\equiv}C-H$ |
| O | 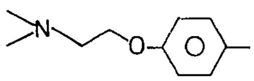 | $CH_3$ | OH | $-CH_2-C{\equiv}C-H$ |
| O | 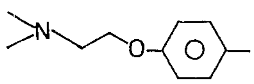 | $CH_3$ | $-C{\equiv}C-H$ | $-OH$ |
| O | 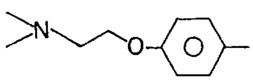 | $CH_3$ | 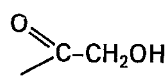 | $-H$ |
| O | 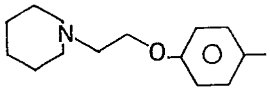 | $CH_3$ | 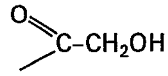 | $-H$ |
| O | 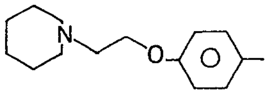 | $CH_3$ | $-C{\equiv}C-H$ | $-OH$ |
| O | 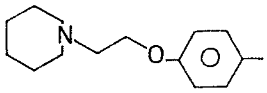 | $CH_3$ | $-OH$ | $-C{\equiv}C-H$ |
| O | 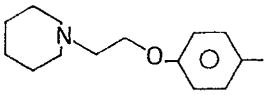 | $CH_3$ | $-OH$ | $-C{\equiv}C-CH_3$ |

| A | $R_1$ | $R_2$ | $R_3$ | $R_4$ |
|---|---|---|---|---|
| O | 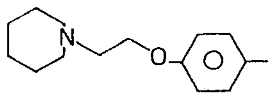 | $CH_3$ | $-OH$ | $-CH_2-C{\equiv}C-H$ |
| O | 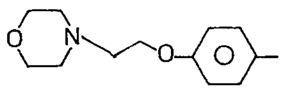 | $CH_3$ | $-OH$ | $-C{\equiv}C-H$ |
| O | 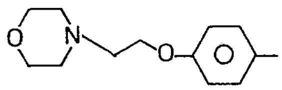 | $CH_3$ | $-OH$ | $-C{\equiv}C-CH_3$ |
| O | 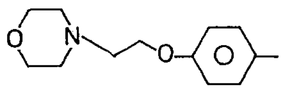 | $CH_3$ | $-OH$ | $-CH_2-C{\equiv}C-H$ |
| O | 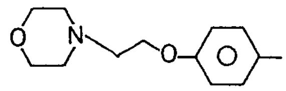 | $CH_3$ | $-C{\equiv}C-H$ | $-OH$ |
| O | 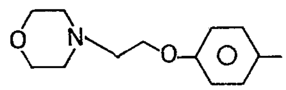 | $CH_3$ | 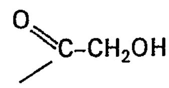 | $-H$ |
| O | 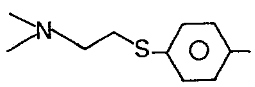 | $CH_3$ | 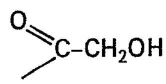 | $-H$ |
| O | 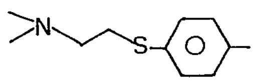 | $CH_3$ | $-C{\equiv}C-H$ | OH |
| O | 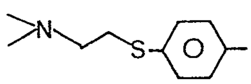 | $CH_3$ | OH | $-C{\equiv}C-CF_3$ |
| O | 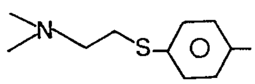 | $CH_3$ | OH | $-C{\equiv}C-H$ |
| O | 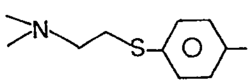 | $CH_3$ | OH | $-CH_2-CH{=}CH_2$ |
| O | 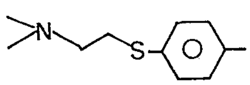 | $CH_3$ | OH | $CH_2-C{\equiv}C-H$ |
| O | 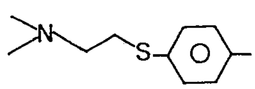 | $CH_3$ | OH | $-CH_2$ |
| O | 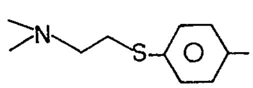 | $CH_3$ | 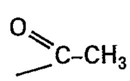 | $-CH_3$ |
| O | 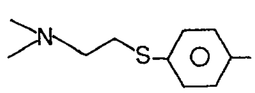 | $CH_3$ | OH | $-CH_2-CN$ |
| HO–N=(E) | 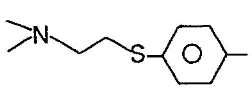 | $CH_3$ | OH | $-C{\equiv}C-H$ |

| A | $R_1$ | $R_2$ | $R_3$ | $R_4$ |
|---|---|---|---|---|
| HO–N=(E) | 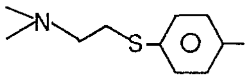 | $CH_3$ | OH | $-C\equiv C-CH_3$ |
| HO–N=(E) | 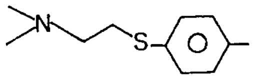 | $CH_3$ | OH | $-C\equiv C-CH_2CH_3$ |
| HO–N=(E) | 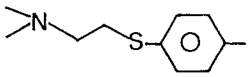 | $CH_3$ | OH | $-C\equiv C-Cl$ |
| HO–N=(E) | 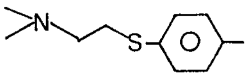 | $CH_3$ | OH | $-CH_2C\equiv C-H$ |
| HO–N=(E) | 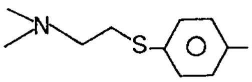 | $CH_3$ | $-C\equiv C-H$ | –OH |
| HO–N=(E) | 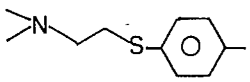 | $CH_3$ | 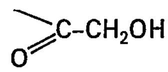 | –H |
| HO–N=(Z) | 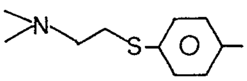 | $CH_3$ | 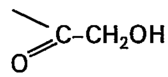 | –H |
| HO–N=(Z) | 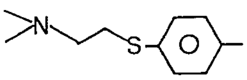 | $CH_3$ | $-C\equiv C-H$ | –OH |
| HO–N=(Z) | 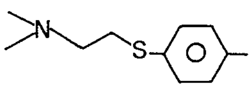 | $CH_3$ | OH | $-C\equiv C-H$ |
| HO–N=(Z) | 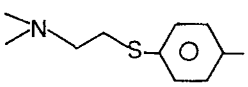 | $CH_3$ | OH | $-C\equiv C-CH_3$ |
| HO–N=(Z) | 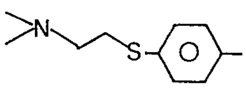 | $CH_3$ | OH | $-C\equiv C-CH_2-CH_3$ |
| HO–N=(Z) | 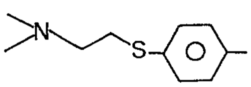 | $CH_3$ | OH | $-C\equiv C-Cl$ |
| HO–N=(Z) | 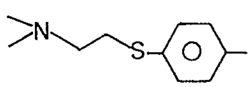 | $CH_3$ | OH | $-CH_2-C\equiv C-H$ |
| O | 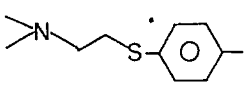 | $CH_2CH_3$ | OH | $-C\equiv C-H$ |
| O | 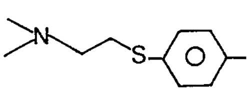 | $CH_2CH_3$ | OH | $-C\equiv C-CH_3$ |
| O | 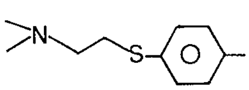 | $CH_2CH_3$ | OH | $-CH_2-C\equiv C-H$ |

| A | $R_1$ | $R_2$ | $R_3$ | $R_4$ |
|---|---|---|---|---|
| O | 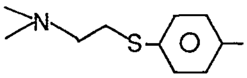 | $CH_2CH_3$ | OH | $-CH_2-CH=CH_2$ |
| O | 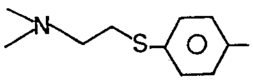 | $CH_2CH_3$ | 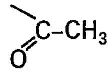 | $-CH_3$ |
| O | 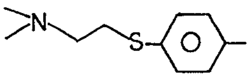 | $CH_2CH_3$ | 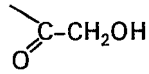 | –H |
| O | 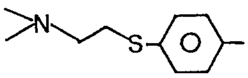 | $CH_2CH_3$ | $-C\equiv C-H$ | –OH |
| O | 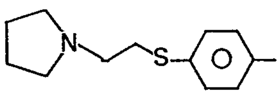 | $CH_3$ | OH | $-C\equiv C-H$ |
| O | 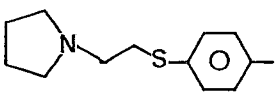 | $CH_3$ | OH | $-C\equiv C-CH_3$ |
| O | 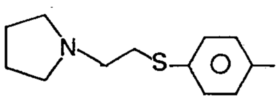 | $CH_3$ | OH | $-C\equiv C-Cl$ |
| O | 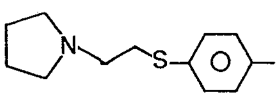 | $CH_3$ | OH | $-C\equiv C-CH_2CH_3$ |
| O | 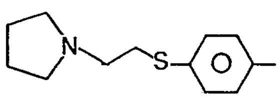 | $CH_3$ | OH | $-CH_2-C\equiv C-H$ |
| O | 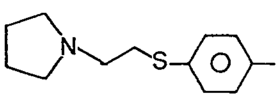 | $CH_3$ | OH | $-CH_2-CH=CH_2$ |
| O | 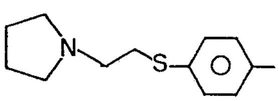 | $CH_3$ | $-C\equiv C-H$ | –OH |
| O | 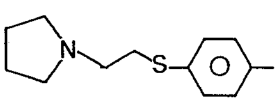 | $CH_3$ | 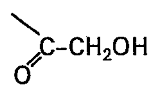 | –H |
| O | 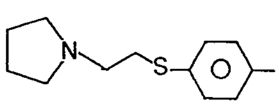 | $CH_3$ | 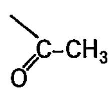 | $-CH_3$ |
| O | 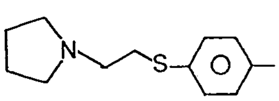 | $CH_3$ | 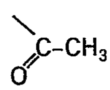 | –H |
| O | 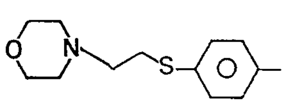 | $CH_3$ | 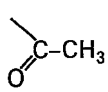 | –H |

| A | $R_1$ | $R_2$ | $R_3$ | $R_4$ |
|---|---|---|---|---|
| O | 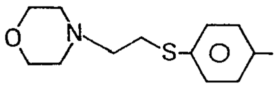 | $CH_3$ | 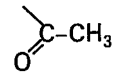 | $-CH_3$ |
| O | 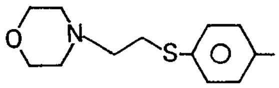 | $CH_3$ | 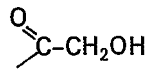 | -H |
| O | 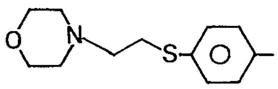 | $CH_3$ | $-C{\equiv}C-H$ | -OH |
| O | 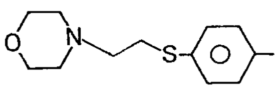 | $CH_3$ | OH | $-C{\equiv}C-H$ |
| O | 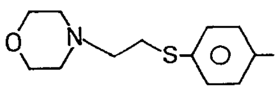 | $CH_3$ | OH | $-C{\equiv}C-CH_3$ |
| O | 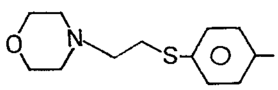 | $CH_3$ | OH | $-C{\equiv}C-Cl$ |
| O | 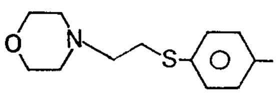 | $CH_3$ | OH | $-C{\equiv}C-CH_2CH_3$ |
| O | 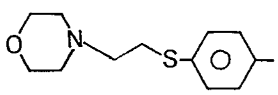 | $CH_3$ | OH | $-CH_2-C{\equiv}C-H$ |
| O | 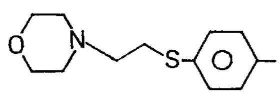 | $CH_3$ | OH | $-CH_2-CH=CH_2$ |
| O | 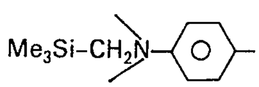 | $CH_3$ | OH | $-C{\equiv}C-H$ |
| O | 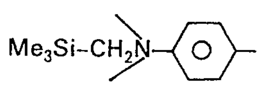 | $CH_3$ | OH | $-C{\equiv}C-CH_3$ |
| O | 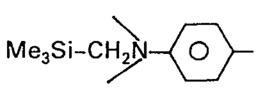 | $CH_3$ | OH | $-CH_2-C{\equiv}C-H$ |
| O | 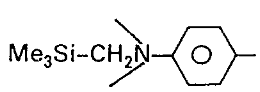 | $CH_3$ | $-C{\equiv}C-H$ | -OH |
| O | 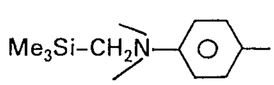 | $CH_3$ | 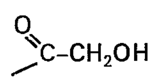 | -H |
| O | 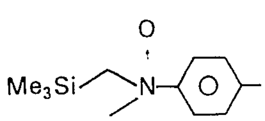 | $CH_3$ | OH | $-C{\equiv}C-H$ |

| A | $R_1$ | $R_2$ | $R_3$ | $R_4$ |
|---|---|---|---|---|
| O | 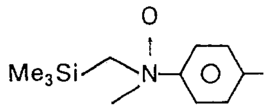 | $CH_3$ | OH | $-C{\equiv}C-CH_3$ |
| O | 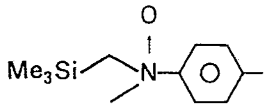 | $CH_3$ | OH | $-CH_2-C{\equiv}C-H$ |
| O | 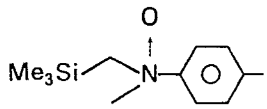 | $CH_3$ | $-C{\equiv}C-H$ | $-OH$ |
| O | 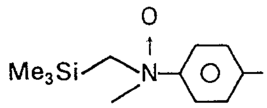 | $CH_3$ | 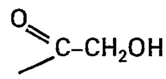 | $-H$ |
| O | 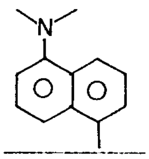 | $CH_3$ | OH | $-C{\equiv}C-CH_3$ |
| O | 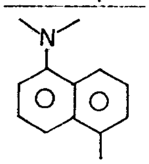 | $CH_3$ | OH | $-CH_2-C{\equiv}C-H$ |
| O | 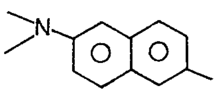 | $CH_3$ | OH | $-C{\equiv}C-H$ |
| O | 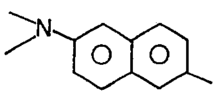 | $CH_3$ | OH | $-C{\equiv}C-CH_3$ |
| O | 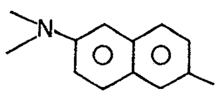 | $CH_3$ | OH | $-C{\equiv}C-Cl$ |
| O | 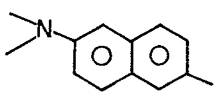 | $CH_3$ | OH | $-CH_2-C{\equiv}C-H$ |
| O | 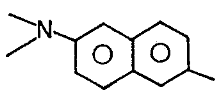 | $CH_3$ | $-C{\equiv}C-H$ | $-OH$ |
| O | 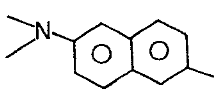 | $CH_3$ | 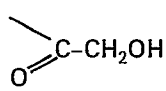 | $-H$ |
| O | 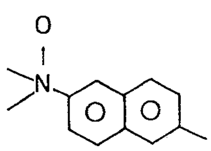 | $CH_3$ | 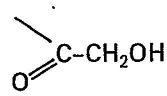 | $-H$ |

| A | $R_1$ | $R_2$ | $R_3$ | $R_4$ |
|---|---|---|---|---|
| O | 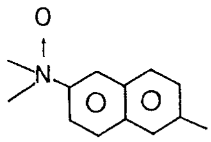 | $CH_3$ | $-C\equiv C-H$ | $-OH$ |
| O | 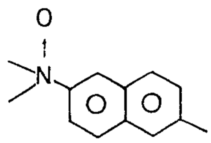 | $CH_3$ | $-OH$ | $-C\equiv C-H$ |
| O | 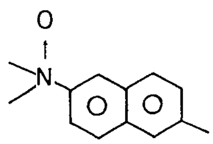 | $CH_3$ | $-OH$ | $-C\equiv C-CH_3$ |
| O | 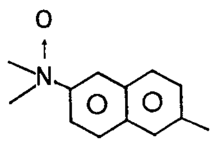 | $CH_3$ | $-OH$ | $-C\equiv C-Cl$ |
| O | 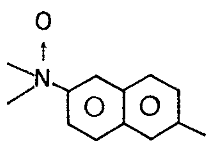 | $CH_3$ | $-OH$ | $-CH_2-C\equiv C-H$ |
| O | $Me_3Si\ CH_2-$ | $CH_3$ | $-OH$ | $-C\equiv C-H$ |
| O | $Me_3Si\ CH_2-$ | $CH_3$ | $-OH$ | $-C\equiv C-CH_3$ |
| O | $Me_3Si\ CH_2-$ | $CH_3$ | $-OH$ | $-CH_2-C\equiv C-H$ |
| O | $Me_3Si\ CH_2-$ | $CH_3$ | $-C\equiv C-H$ | $-OH$ |
| HO–N=(E) | $Me_3Si\ CH_2-$ | $CH_3$ | $-C\equiv C-H$ | $-OH$ |
| HO–N=(E) | $Me_3Si\ CH_2-$ | $CH_3$ | OH | $-C\equiv C-H$ |
| HO–N=(E) | $Me_3Si\ CH_2-$ | $CH_3$ | OH | $-C\equiv C-CH_3$ |
| HO–N=(E) | $Me_3Si\ CH_2-$ | $CH_3$ | OH | $-CH_2-C\equiv C-H$ |
| HO–N=(Z) | $Me_3Si\ CH_2-$ | $CH_3$ | $-C\equiv C-H$ | $-OH$ |
| HO–N=(Z) | $Me_3Si\ CH_2-$ | $CH_3$ | OH | $-C\equiv C-H$ |
| HO–N=(Z) | $Me_3Si\ CH_2-$ | $CH_3$ | OH | $-C\equiv C-CH_3$ |
| HO–N=(Z) | $Me_3Si\ CH_2-$ | $CH_3$ | OH | $-CH_2-C\equiv C-H$ |

| A | $R_1$ | $R_2$ | $R_3$ | $R_4$ |
|---|---|---|---|---|
| O | 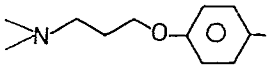 | $CH_3$ | OH | $-C{\equiv}C-CH_3$ |
| O | 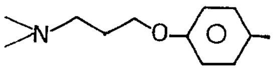 | $CH_3$ | 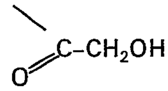 | -H |
| O | 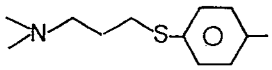 | $CH_3$ | 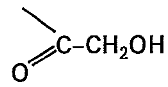 | -H |
| O | 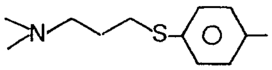 | $CH_3$ | OH | $-C{\equiv}C-H$ |
| O | 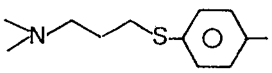 | $CH_3$ | OH | $-C{\equiv}C-CH_3$ |
| O | 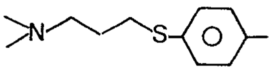 | $CH_3$ | OH | $-CH_2-C{\equiv}C-H$ |
| O | 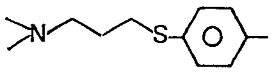 | $CH_3$ | OH | $-CH_2-CH=CH_2$ |
| O | 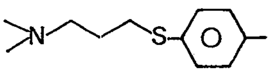 | $CH_3$ | OH | $-CH_2CN$ |
| O | 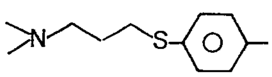 | $CH_3$ | $-C{\equiv}C-H$ | -OH |
| O | 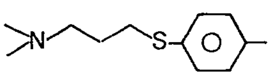 | $CH_3$ | 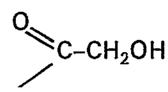 | -H |
| O | 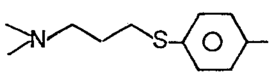 | $CH_3$ | 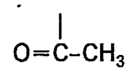 | $-CH_3$ |
| O | 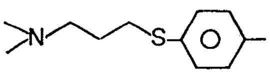 | $CH_3$ | OH | -H |
| O | 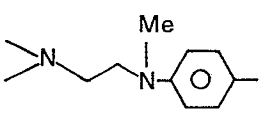 | $CH_3$ | OH | $-C{\equiv}C-H$ |
| O | 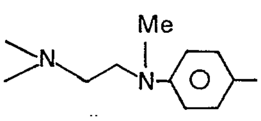 | $CH_3$ | OH | $-C{\equiv}C-CF_3$ |
| O | 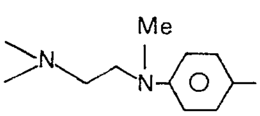 | $CH_3$ | OH | $-C{\equiv}C-CH_2CH_3$ |
| O | 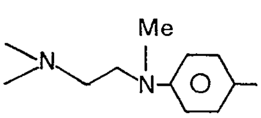 | $CH_3$ | OH | $-C{\equiv}C-Cl$ |
| O | 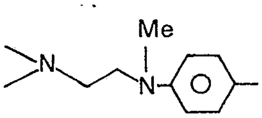 | $CH_3$ | OH | $-CH_2-C{\equiv}C-H$ |

| A | $R_1$ | $R_2$ | $R_3$ | $R_4$ |
|---|---|---|---|---|
| O | 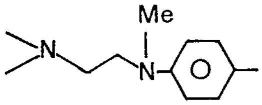 | $CH_3$ | $-C{\equiv}C-H$ | $-OH$ |
| O | 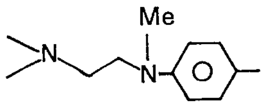 | $CH_3$ | 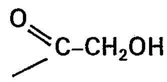 | $-H$ |
| O | 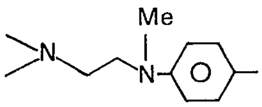 | $CH_3$ | 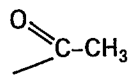 | $-CH_3$ |
| O | 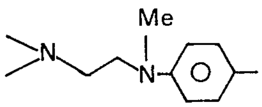 | $CH_2CH_3$ | OH | $-C{\equiv}C-H$ |
| O | 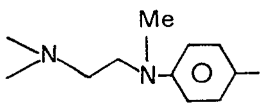 | $CH_2CH_3$ | OH | $-C{\equiv}C-CH_3$ |
| O | 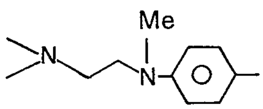 | $CH_2CH_3$ | OH | $-CH_2-C{\equiv}C-H$ |
| O | 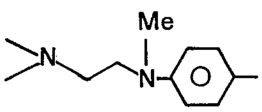 | $CH_2CH_3$ | $-C{\equiv}C-H$ | $-OH$ |
| O | 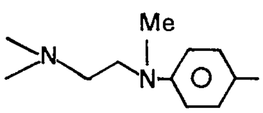 | $CH_2CH_3$ | 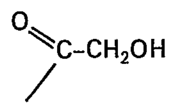 | $-H$ |
| O | 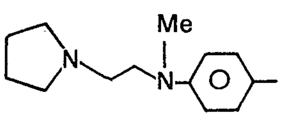 | $CH_3$ | OH | $-C{\equiv}C-H$ |
| O | 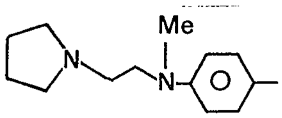 | $CH_3$ | OH | $-C{\equiv}C-CH_3$ |
| O | 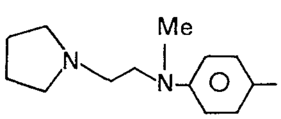 | $CH_3$ | OH | $-CH_2-C{\equiv}C-H$ |
| O | 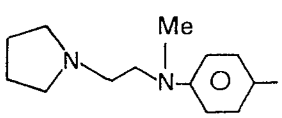 | $CH_3$ | $-C{\equiv}C-H$ | $-OH$ |
| O | 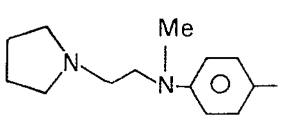 | $CH_3$ | 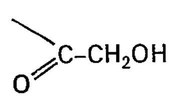 | $-H$ |

| A | $R_1$ | $R_2$ | $R_3$ | $R_4$ |
|---|---|---|---|---|
| O | 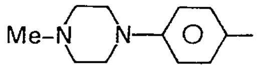 | $CH_3$ | 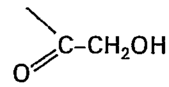 | –H |
| O | 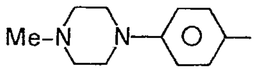 | $CH_3$ | –C≡C–H | –OH |
| O | 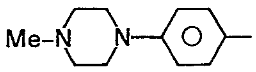 | $CH_3$ | OH | –C≡C–H |
| O | 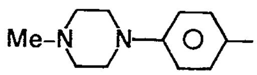 | $CH_3$ | OH | $-CH_2-C \equiv C-H$ |
| O | 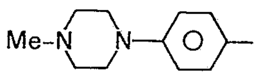 | $CH_3$ | OH | $-CH_2-CH=CH_2$ |
| O | 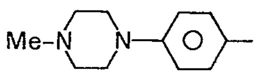 | $CH_3$ | OH | $-CH_2CN$ |
| O | 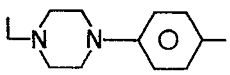 | $CH_3$ | OH | –C≡C–H |
| O | 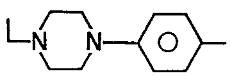 | $CH_3$ | OH | $-C \equiv C-CH_3$ |
| O | 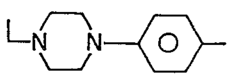 | $CH_3$ | OH | $-CH_2-C \equiv C-H$ |
| O | 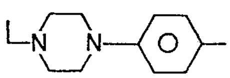 | $CH_3$ | OH | $-CH_2-CH=CH_2$ |
| O | 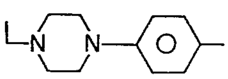 | $CH_3$ | OH | $-CH_2-CN$ |
| O | 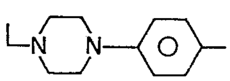 | $CH_3$ | –C≡C–H | –OH |
| O | 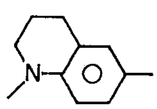 | $CH_3$ | –OH | –C≡C–H |
| O | 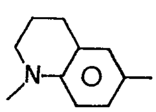 | $CH_3$ | –OH | $-C \equiv C-CH_3$ |
| O | 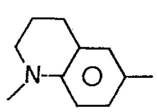 | $CH_3$ | –OH | $-CH_2-C \equiv C-H$ |

| A | $R_1$ | $R_2$ | $R_3$ | $R_4$ |
|---|---|---|---|---|
| O | 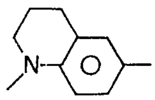 | $CH_3$ | $-C\equiv C-H$ | $-OH$ |
| O | 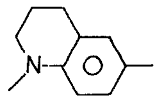 | $CH_3$ | 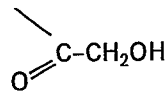 | $-H$ |
| O | 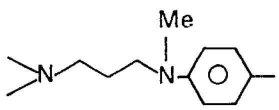 | $CH_3$ | 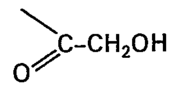 | $-H$ |
| O | 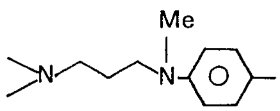 | $CH_3$ | $-C\equiv C-H$ | OH |
| O | 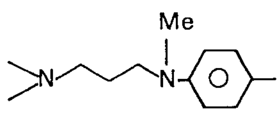 | $CH_3$ | OH | $-C\equiv C-H$ |
| O | 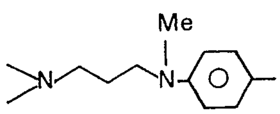 | $CH_3$ | OH | $-C\equiv C-CH_3$ |
| O | 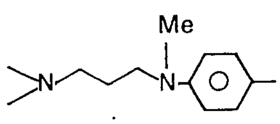 | $CH_3$ | OH | $-CH_2-C\equiv C-H$ |
| O | 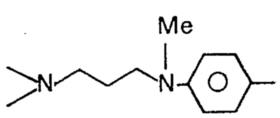 | $CH_3$ | OH | $-H$ |
| O | 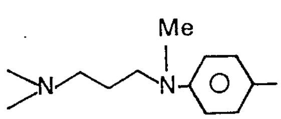 | $CH_3$ | OH | $-CH_3$ |
| O | 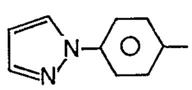 | $CH_3$ | OH | $-C\equiv C-H$ |
| O | 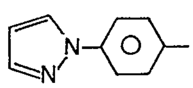 | $CH_3$ | OH | $-C\equiv C-CH_3$ |
| O | 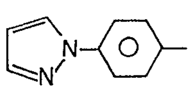 | $CH_3$ | OH | $-C\equiv C-CF_3$ |
| O | 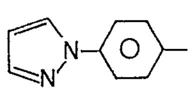 | $CH_3$ | OH | $-CH_2-C\equiv C-H$ |

| A | $R_1$ | $R_2$ | $R_3$ | $R_4$ |
|---|---|---|---|---|
| O | 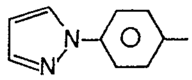 | $CH_3$ | $-C{\equiv}C-H$ | $-OH$ |
| O | 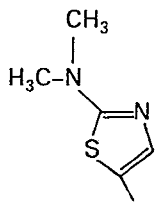 | $CH_3$ | OH | $-C{\equiv}C-H$ |
| O | 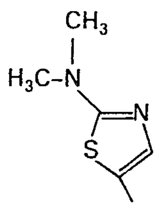 | $CH_3$ | OH | $-C{\equiv}C-CH_3$ |
| O | 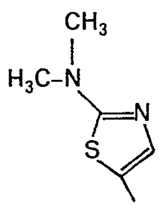 | $CH_3$ | OH | $-C{\equiv}C-CF_3$ |
| O | 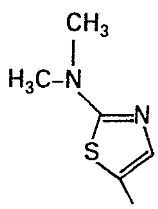 | $CH_3$ | OH | $-CH_2-C{\equiv}C-H$ |
| O | 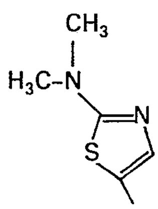 | $CH_3$ | $-C{\equiv}C-H$ | $-OH$ |
| O | 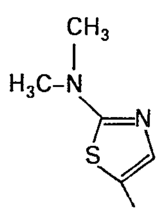 | $CH_3$ | $-C{\equiv}C-CH_3$ | $-OH$ |

B) Les produits de formule:

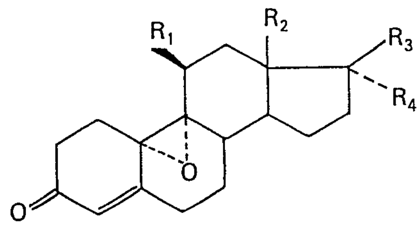

dans laquelle $R_1$, $R_2$, $R_3$ et $R_4$ ont les significations suivantes:

| $R_1$ | $R_2$ | $R_3$ | $R_4$ |
|---|---|---|---|
| 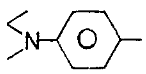 | $CH_3$ | OH | $-C{\equiv}C-H$ |
| 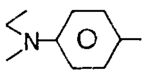 | $CH_3$ | OH | $-C{\equiv}C-CH_3$ |
| 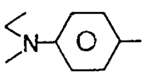 | $CH_3$ | OH | $-C{\equiv}C-CF_3$ |
| 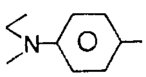 | $CH_3$ | OH | $-CH_2CH_3$ |
| 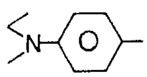 | $CH_3$ | OH | $-CH_2-C{\equiv}C-H$ |
| 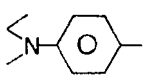 | $CH_3$ | $-C{\equiv}C-H$ | $-OH$ |
| 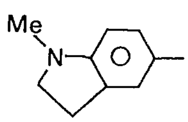 | $CH_3$ | OH | $-C{\equiv}C-H$ |
| 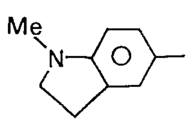 | $CH_3$ | OH | $-C{\equiv}C-CH_3$ |
| 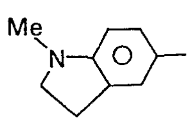 | $CH_3$ | OH | $-C{\equiv}C-CF_3$ |
| 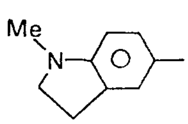 | $CH_3$ | OH | $-C{\equiv}C-CH_2CH_3$ |
| 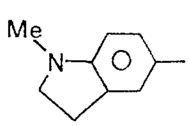 | $CH_3$ | OH | $-CH_2C{\equiv}C-H$ |

| $R_1$ | $R_2$ | $R_3$ | $R_4$ |
|---|---|---|---|
| 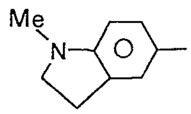 | $CH_3$ | OH | -H |
| 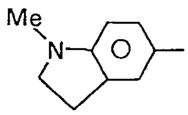 | $CH_3$ | OH | $-CH_2CH_3$ |
| 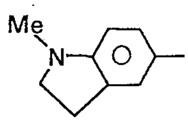 | $CH_3$ | -C≡C-H | -OH |
| 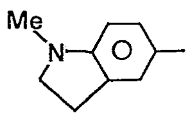 | $CH_3$ | 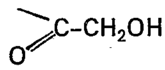 | -H |
| 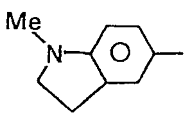 | $CH_3$ | 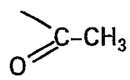 | $-CH_3$ |
| 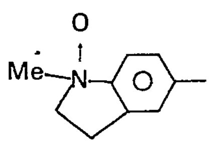 | $CH_3$ | 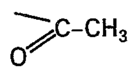 | $-CH_3$ |
| 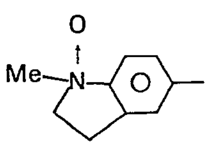 | $CH_3$ | OH | -C≡C-H |
| 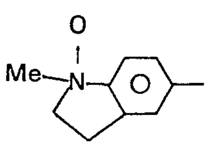 | $CH_3$ | OH | $-C≡C-CH_3$ |
| 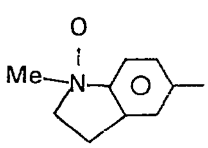 | $CH_3$ | OH | $-C≡C-CF_3$ |
| 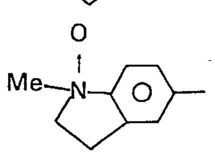 | $CH_3$ | OH | $-CH_2CH_3$ |
| 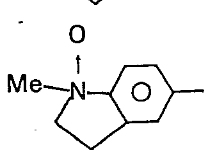 | $CH_3$ | OH | $-CH_2-C≡C-H$ |
| 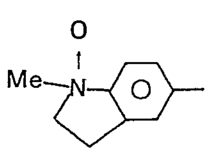 | $CH_3$ | -C≡C-H | -OH |

| $R_1$ | $R_2$ | $R_3$ | $R_4$ |
|---|---|---|---|
| 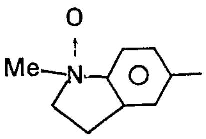 | $CH_3$ | OH | H |
| 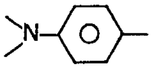 | $CH_3$ | 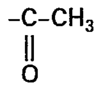 | $CH_3$ |
| 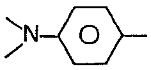 | $CH_3$ | OH | $-C\equiv C-H$ |
| 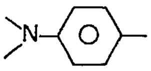 | $CH_3$ | OH | $-C\equiv C-CH_3$ |
| 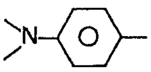 | $CH_3$ | OH | $-C\equiv C-CF_3$ |
| 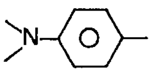 | $CH_3$ | OH | $-CH_2CH_3$ |
| 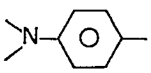 | $CH_3$ | OH | $-CH_2-C\equiv C-H$ |
| 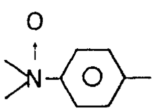 | $CH_3$ | OH | $-C\equiv C-H$ |
| 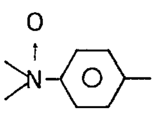 | $CH_3$ | OH | $-C\equiv C-CH_3$ |
| 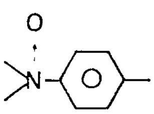 | $CH_3$ | OH | $-C\equiv C-CF_3$ |
| 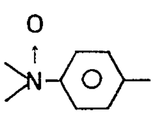 | $CH_3$ | OH | $-CH_2-CH_3$ |
| 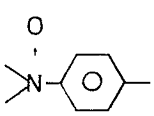 | $CH_3$ | OH | $-CH_2-C\equiv C-H$ |
| 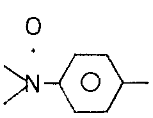 | $CH_3$ | OH | H |

| $R_1$ | $R_2$ | $R_3$ | $R_4$ |
|---|---|---|---|
| 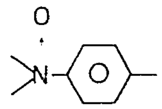 | $CH_3$ | OH | $CH_3$ |
| 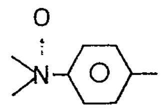 | $CH_3$ | -C≡C-H | OH |

On peut également préparer les époxydes correspondant aux autres produits figurant dans la liste A ci-dessus.

Les exemples suivants illustrent l'invention sans toutefois la limiter.

Exemple 1:

17β-hydroxy 17α-(prop-1-ynyl) 11β-(4-pyridyl) estra-4,9-dièn-3-one.

Stade A: 11β-(4-pyridyl) 3,3-[1,2-éthanediyl bis(oxy)]17α-[prop-1-ynyl] estr-9-en 5α 17β-diol.

On ajoute à 20°C, 100 $cm^3$ d'une solution de bromure de 4-chloropyridinyl magnésium dans le tétrahydrofuranne (solution 0,5–0,6 M préparée à partir de 15 g de 4-chloropyridine et de 6 g de magnésium) dans une solution renfermant 6,16 g de complexe diméthyl sulfure, bromure cuivreux dans 40 $cm^3$ de tétrahydrofuranne. On agite 20 minutes à la température ambiante sous atmosphère inerte et ajoute en 10 minutes une solution renfermant 3,7 g de 3,3-[1,2-éthanediyl bis (oxy)] 5α 10α-époxy 17α-(prop-1-ynyl)estr-9(11)-ène 17β-ol. On agite pendant une heure à la température ambiante et verse dans un mélange d'eau froide et de chlorure d'ammonium. On agite le mélange réactionnel pendant une demi-heure à la température ambiante et extrait à l'éther. On lave avec une solution saturée de chlorure de sodium, on sèche et concentre à sec sous pression réduite. On obtient 6 g d'un produit que l'on chromatographie sur silice en éluant par le mélange chlorure de méthylène-acétone 1:1 contenant 1 pour mille de triéthylamine. On isole ainsi 3,15 g de produit que l'on sèche sous un vide de 0,1 mm de mercure vers 60°C. On obtient ainsi le produit recherché $\alpha_D$ = -52°±1,5 (c = 1% $CHCl_3$).

Stade B: 17β-hydroxy 17α-(prop-1-ynyl) 11β-(4-pyridyl) estra 4,9-dien-3-one.

On agite pendant 3 heures à la température ambiante sous atmosphère inerte une solution renfermant 2,9 g du produit préparé au stade A, 14 $cm^3$ de méthanol et 7 $cm^3$ d'acide chlorhydrique 2N. On ajoute ensuite une solution renfermant 200 $cm^3$ d'éther et 90 $cm^3$ d'une solution saturée de carbonate acide de sodium. On agite pendant 15 minutes à la température ambiante, décante et extrait à l'éther. On lave les extraits avec une solution saturée de chlorure de sodium, puis l'on sèche et concentre à sec sous pression réduite. On obtient 2,3 g d'un produit que l'on chromatographie sur silice en éluant par le mélange chlorure de méthylène-acétone 6:4. On isole 1,7 g de produit que l'on sèche sous pression de 0,1 mm de mercure pendant 24 heures dont 8 heures à 80°C. On obtient ainsi le produit recherché /$\alpha$/$_D$ = +30°5∓1° (c = 1% $CHCl_3$).

De la même manière on a préparé le 17β-hydroxy, 17α-(prop-1-ynyl) 11β-(3-pyridyl) estra 4,9-dien 3-one (/$\alpha$/$_D$ = +14° c = 1% $CHCl_3$) et le 17β-hydroxy 17α-(prop-1-ynyl) 11β-(2-pyridyl) estra-4,9-dièn 3-one /$\alpha$/$_D$ = -2° c = 1% $CHCl_3$).

Exemple 2:

17β-hydroxy 11β[3-(N,N-diméthylamino) propyl] 17α-(prop-1-ynyl) estra-4,9-dien 3-one.

Stade A: 11β-[3-(N,N-diméthylamino) propyl] 3,3-[1,2-éthane diyl bis (oxy)] 17α-(prop-1-ynyl) estr-9-en 5α, 17β-diol.

On ajoute en 5 minutes à 0°C 12,33 g de complexe diméthyl sulfure bromure cuivreux dans 141 $cm^3$ du chlorure de 3-(N,N-diméthylamino) propyl magnésium, solution 0,85 M préparée à partir de 42 g de chloro 3-N,N-diméthylamino propane et 10,5 g de magnésium. On maintient sous agitation 25 minutes à 0°C et introduit goutte à goutte 3,70 g de 3,3-[1,2-éthanediyl bis (oxy)] 5α 10α époxy 17α-(1-propynyl) estr-9(11)-ène-17β-ol en solution dans 50 $cm^3$ de tétrahydrofuranne. On maintient le mélange réactionnel pendant 3 heures à 0°C sous agitation et le verse dans un mélange renfermant 40g de chlorure d'ammonium et 200 $cm^3$ d'eau glacée. On agite pendant 15 minutes à la température ambiante, puis extrait à l'éther. On lave avec une solution saturée de chlorure de sodium, on sèche et concentre à sec sous pression réduite. On obtient 4,6 g d'un produit que l'on chromatographie sur silice en éluant avec un mélange chlorure de méthylène-méthanol (8:2). On isole 2,55 g de produit. /$\alpha$/$_D$ = -86°±1,5° (c = 1% dans le chloroforme).

Stade B: 17β-hydroxy 11β [3-(N,N-diméthylamino) propyl]17α (prop-1-ynyl) estra 4,9-dien-3-one.

On agite à la température ambiante pendant 4 heures sous atmosphère inerte 2,4 g du produit préparé au stade A, 14 $cm^3$ de méthanol et 7 $cm^3$ d'acide chlorhydrique 2N. On ajoute ensuite 200 $cm^3$ d'éther isopropylique et 90 $cm^3$ d'une solution saturée de carbonate acide de sodium. On

agite une demi-heure à la température ambiante, décante et extrait à l'éther. On lave avec une solution saturée de chlorure de sodium et sèche. On concentre à sec sous pression réduite et on obtient 1,8 g d'un produit que l'on chromatographie sur silice en éluant par le mélange chloroforme-méthanol 8:2. On obtient ainsi 1,30 g d'un produit que l'on sèche à 30°-40°C environ sous pression réduite de 0,1 mm de mercure. On obtient ainsi 1,25 g de produit recherché /α/$_D$ = -114°+2,5° (c = 1% $CHCl_3$).

Exemple 3:

11β-[4-(N,N-diméthylamino éthyloxy) phényl] 17β-hydroxy 17α-(prop-1-ynyl) estra 4,9-dien-3-one.

Stade A: 3,3-éthane-diyl bis(oxy) 11β-[4-(N,N-diméthylamino éthyloxy) phényl] 17α-(prop-1-ynyl) estra-9-en 5α, 17β-diol.

a) magnesien de 4-(N,N-diméthylamino éthyloxy) bromobenzène.

On introduit goutte à goutte en 45 minutes une solution renfermant 24 g de 4-(N,N-diméthylamino éthyloxy) bromobenzène dans 90 cm³ de tétrahydrofuranne anhydre. On catalyse la réaction par addition de 0,2 cm³ de 1,2-dibromoéthane. L'introduction terminée, on agite encore une heure à 25°C. On obtient ainsi une solution 0,7M que l'on utilise telle quelle.

b) condensation.

On ajoute la solution préparée précédemment dans une solution renfermant 6,16 g de complexe diméthyl sulfure, bromure cuivreux dans 20 cm³ de tétrahydrofuranne. On agite 20 minutes à la température ambiante et ajoute, goutte à goutte, en quelques minutes, 3,7 g du 3,3-[1,2-(éthanediyl bis (oxy)] 5α, 10α-époxy 17α-(prop-1-ynyl) estr- 9(11)-ène 17β-ol dans 50 cm³ de tétrahydrofuranne. On agite ensuite pendant une heure sous atmosphère inerte, puis verse le mélange réactionnel dans une solution renfermant 15 g de chlorure d'ammonium dans 200 cm³ d'eau glacée. On extrait à l'éther et lave avec une solution aqueuse saturée de chlorure de sodium. On sèche et concentre sous pression réduite. On obtient ainsi 18,3 g d'une huile que l'on chromatographie sur alumine, élue au chloroforme et obtient ainsi 4,5 g du produit recherché.

/α/$_D$ = -44°±1,5° (c = 1% $CHCl_3$).

Stade B: 11β-[4-(N,N-diméthylamino éthyloxy) phényl] 17β-hydroxy 17α-(prop-1-ynyl) estra 4,9-dien-3-one.

On ajoute 9,5 cm³ d'acide chlorhydrique 2N dans une solution renfermant 4,5 g du produit préparé au stade A dans 20 cm³ de méthanol. On maintient la solution sous agitation pendant 2 heures à la température ambiante et ajoute 260 cm³ d'éther et 110 cm³ d'une solution saturée de carbonate acide de sodium. On maintient sous agitation pendant 15 minutes à la température ambiante, décante et extrait à l'éther. On sèche et on concentre à sec sous pression réduite. On obtient 3,3 g d'un produit que l'on chromatographie sur silice en éluant par le mélange chlorure de méthylène-méthanol (92,5:7,5). On obtient ainsi 1,8 g de produit attendu amorphe.

/α/$_D$ = +71° (c = 1% $CHCl_3$).

Exemple 4:

17β-hydroxy 11β-(4-diméthylamino phényl) 17α-(prop-1-ynyl) estra 4,9-dien-3-one.

Stade A: 11β-(4-diméthylamino phényl) 3,3-[1,2-éthanediyl bis (oxy)] 17α(prop-1-ynyl) estr 9-en-5α, 17β-diol.

On ajoute une solution renfermant 38 m/moles de bromure de p-diméthylamino phényl magnésium dans du tétrahydrofuranne à une suspension renfermant 4,1 g de complexe bromure cuivreux-diméthyl sulfure dans 20 cm³ de tétrahydrofuranne. On ajoute ensuite 2,45 g de 3,3-[1,2-éthanediyl bis (oxy)]5α, 10α époxy 17α-(prop-1-ynyl) estr 9(11)-ène 17β-ol en solution dans du tétrahydrofuranne. On maintient le mélange réactionnel sous agitation pendant 10 minutes, on hydrolyse avec 50 cm³ d'une solution saturée de chlorure d'ammonium. On décante, extrait à l'éther, lave la phase organique à l'eau et la sèche. On évapore les solvants sous pression réduite et l'on obtient 11 g de produit recherché brut que l'on chromatographie sur silice en éluant par le mélange cyclohexane-acétate d'éthyle 6:40. On obtient ainsi 1,8 g du produit recherché (11β) et 750 mg du produit 11α. On recristallise dans l'éther d'isopropyle et l'acétate d'éthyle F = 210°C /α/$_D$ = -66°5 (1% $CHCl_3$).

Stade B: 17β-hydroxy 11β-(4-diméthylamino phényl) 17α-(prop-1-ynyl) estra-4,9-dien-3-one.

On ajoute 2 cm³ d'une solution d'acide chlorhydrique concentré à une solution renfermant 1,53 g du produit préparé au stade A dans 60 cm³ de méthanol. On agite 30 minutes à la température ambiante, et ajoute 150 cm³ d'éther, puis 50 cm³ d'une solution aqueuse de soude N. On agite le milieu réactionnel pendant 15 minutes et l'on décante, sèche la phase organique. On évapore les solvants sous pression réduite et l'on obtient 1,4 g de produit brut que l'on purifie sur silice en éluant par le mélange cyclohexane-acétate d'éthyle (7:3). On obtient 0,932 g, du produit recherché à F = 150°C /α/$_D$ = +138,5° (c = 0,5% $CHCl_3$).

Exemple 5:

17β-hydroxy 17α-(prop-1-ynyl) 11β[(4-triméthylsilyl) phényl] estra-4,9-dien-3-one.

Stade A: 11β[(4-triméthylsilyl)phényl]3,3 [1,2-éthanediyl bis (oxy)] 17α-(prop-1-ynyl) estr-9-en-5α, 17β-diol.

On ajoute à -30°C sous atmosphère inerte dans 45 cm³ d'une solution 0,65 M de bromure de 4-triméthylsilyl phényl magnésium dans le tétrahydrofuranne, 200 mg de chlorure cuivreux, puis goutte à goutte, en maintenant la température à -20°C, une solution de 3,3 g de 3,3-[1,2-éthanediyl bis (oxy)] 5α, 10α-époxy 17α-(prop-1-ynyl) estr 9(11)-en 17β-ol dans 25 cm³ de tétrahydrofuranne. Après une heure, on hydrolyse au moyen d'une solution aqueuse de chlorure d'ammonium, extrait à l'éther, sèche et évapore les solvants sous pression réduite. On chromatographie sur silice en éluant par le mélange chlorure de méthylène-acétone (94:6) à 0,1% de triéthylamine. On isole 2,087 g du produit recherché que l'on purifie par recristallisation dans l'éther isopropylique puis dans l'acétate d'éthyle F = 226°C. $/\alpha/_D$ = -60°±1,5° (c = 0,9% $CHCl_3$).

Stade B: 17β-hydroxy 17α-(prop-1-ynyl) 11β [(4-triméthylsilyl) phenyl] estra-4,9-dien-3-one.

On ajoute 1,7 g de résine sulfonique Redex dans une solution renfermant 1,68 g du produit préparé au stade A dans 17 cm³ d'alcool à 90° bouillant. On chauffe au reflux pendant 30 minutes, essore la résine, la rince au chlorure de méthylène, et évapore le filtrat sous pression réduite. On reprend le résidu ainsi obtenu au chlorure de méthylène, sèche et chasse le solvant sous pression réduite. On chromatographie le résidu obtenu sur silice en éluant par le mélange benzène-acétate d'éthyle (85:15). On obtient ainsi 1,217 g du produit recherché fondant à 212°C. $/\alpha/_D$ = +94° (c = 0,9% $CHCl_3$).

De la même manière, on a préparé le 17β-hydroxy-17α-(prop-1-ynyl) 11β-[3-triméthylsilyl) phényl] estra-4,9-dien-3-one. $/\alpha/_D$ + 52,5°±2° (c = 1% $CHCl_3$).

Préparation: 3,3-[1,2-éthanediyl bis (oxy)] 17α-(prop-1-ynyl) estr-9(11)-en 5,10-époxy 17β-ol.

Stade A: 3,3-[1,2-éthanediyl bis (oxy)] 17α-(prop-1-ynyl) estra 5(10) 9(11)-diène 17β-ol.

On refroidit à 0°C, sous agitation, 207 cm³ d'une solution de bromure d'éthyle magnésium dans le tétrahydrofuranne à 1,15%, on fait barboter pendant 1 heure 30 minutes à 0°C, du propyne gaz préalablement séché sur chlorure de calcium. On laisse revenir à la température ambiante et agite encore 1 heure tout en maintenant le barbotage. On ajoute ensuite à 20 ~ 25°C en une demi-heure, une solution renfermant 30 g de 3,3-[1,2-éthanediyl bis (oxy)] estra 5(10) 9(11)-diène 17-one dans 120 cm³ de tétrahydrofuranne anhydre et une goutte de triéthylamine anhydre. On agite à température ambiante pendant 2 heures et verse dans un mélange d'eau distillée, de chlorure d'ammonium et de glace. On agite et extrait à l'éther éthylique trois fois. On lave la phase organique à l'eau, la sèche, et la concentre sous pression réduite. Le résidu est séché sous vide. On obtient 35,25 g du produit recherché.

Spectre RMN $CDCl_3$ ppm
0,83 H du méthyle en 18
1,85 H du méthyle en c≡ C $CH_3$
5,65 H du carbone en 11
4 H de l'éthylène cétal

Stade B: 3,3-[1,2-éthylènedioxy bis (oxy)] 17α-(prop-1-ynyl) estr 9(11)-en 5α, 10α-époxy 17β-ol.

On introduit sous agitation et barbotage d'azote 30 g du produit préparé au stade A dans 150 cm³ de chlorure de méthylène. On refroidit à 0°C puis ajoute en une seule fois 1,8 cm³ de sesquihydrate d'hexafluoroacétone, puis sous agitation 4,35 cm³ d'eau oxygénée à 85%. On maintient le mélange réactionnel sous agitation et barbotage d'azote à 0°C pendant 72 heures. On verse ensuite la solution réactionnelle dans un mélange renfermant 250 g de glace et 500 cm³ de thiosulfate de sodium 0,2N. On agite quelques instants puis extrait au chlorure de méthylène. On lave la phase organique à l'eau distillée, la sèche sur sulfate de soude en présence de pyridine, puis concentre sous pression réduite. On sèche le résidu sous pression réduite. On obtient 31,6 g d'un produit que l'on chromatographie sur silice, éluant benzène à acétate d'éthyle 90:10. On obtient ainsi le produit recherché.

Spectre RMN $CDCl_3$ ppm
0,82 H du méthyle en 18
1,83 H du méthyle du radical c ≡ C $-CH_3$
6,1 H du carbone en 11
3,92 H du cétal

Exemple 6:

17β-éthynyl 17α-hydroxy 11β-(4-diméthylaminophényl) estra 4,9-diène 3-one.

Stade A: 3,3-diméthoxy 5α-17α-hydroxy 11β-(4-diméthylaminophényl) 17β-éthynyl estr-9-ène.

On mélange sous gaz inerte 2,8 g de 3,3-diméthoxy 5α-10α-époxy 17β-éthynyl 17α-hydroxy estr-9(11)-ène, 56 cm³ de tétrahydrofuranne anhydre et 80 mg de chlorure cuivreux anhydre. On agite pendant 5 minutes à température ambiante puis place dans un bain d'eau glacée et ajoute goutte à goutte 33 cm³ d'une solution 0,95 M de bromure de (4-diméthylaminophényl) magnésium dans le tétrahydrofuranne. On laisse ensuite remonter à température ambiante.

A une suspension du complexe bromure de cuivre-sulfure de diméthyl (6,15 g) dans 30 cm³ de tétrahydrofuranne anhydre, on ajoute 63 cm³ de bromure de (4-diméthylaminophényl) magnésium de façon à ce que la température reste inférieure à 28,5°C. On laisse pendant 30 minutes sous agitation, puis ajoute goutte à goutte la solution obtenue ci-dessus. On maintient pendant 18 heures sous agitation à température ambiante, verse dans une solution saturée de chlorure d'ammonium, agite pendant 10 minutes, extrait au chloroforme, lave la phase organique à l'eau, la sèche et évapore le solvant. On chromatographie le résidu sur silice en éluant au mélange éther de pétrole-acétate d'éthyle (1:1) à 0,5 pour mille de triéthylamine, obtient 1,28 g de produit. On purifie à nouveau ce produit par chromatographie sur silice en éluant au même mélange, et obtient 0,84 g de produit attendu.

Stade B: 17β-éthynyl 17α-hydroxy 11β-(4-diméthylaminophényl) estra 4,9-dièn-3-one.

On mélange 0,76 g du produit obtenu au stade A avec 15 cm³ de méthanol et 1,6 cm³ d'acide chlorhydrique 2N. On agite pendant 1 heure et demie puis verse dans une solution aqueuse saturée de bicarbonate de sodium, extrait au chloroforme, sèche la phase organique et évapore le

solvant. On obtient 0,76 g de produit brut que l'on chromatographie sur silice en éluant au mélange éther de pétrole-acétate d'éthyle (1:1) puis en éluant au mélange éther éthylique-éther de pétrole (3:1). On obtient 0,435 g de produit attendu, que l'on cristallise dans l'éther isopropylique. F = 142°C.

$/\alpha/_D = +235{,}5° \pm 4{,}5°$ (c = 0,45% chloroforme).

Le produit de départ du stade A a été préparé comme suit:

Stade a: 3,3-diméthoxy 17α-hydroxy 17β-éthynyl estra 5(10) 9(11)-diène.

On agite pendant 5 minutes à température ambiante un mélange de 16,8 g de 3,3-diméthoxy 17β-hydroxy 17α-éthynyl estra 5(10) 9(11)-diène, 175 $cm^3$ de tétrahydrofuranne anhydre, 4,35 g de bromure de lithium puis refroidit à -60°C et ajoute 37 $cm^3$ d'une solution 1,35 M de butyllithium dans l'hexanne. On laisse pendant 30 minutes sous agitation puis ajoute 3,9 $cm^3$ de chlorure de méthane sulfonyle et laisse pendant 1 heure à -60°C sous agitation. On verse ensuite dans 500 $cm^3$ d'une solution aqueuse saturée de chlorure d'ammonium, agite pendant 10 minutes, extrait au chlorure de méthylène, sèche la phase organique, ajoute 2,5 $cm^3$ de pyridine puis évapore à sec sous pression réduite à 0°C. On ajoute 75 $cm^3$ de tétrahydrofuranne au résidu obtenu, puis 12,5 $cm^3$ d'eau renfermant 0,75 g de nitrate d'argent. On maintient pendant 18 heures à -30°C, puis pendant 4 heures à température ambiante. On verse dans 500 $cm^3$ d'une solution aqueuse demi-saturée de chlorure d'ammonium, contenant 5 g de cyanure de sodium. On agite pendant 30 minutes à 20°C, extrait au chloroforme, lave avec une solution aqueuse saturée de chlorure de sodium, sèche et évapore le solvant. On chromatographie sur silice en éluant au mélange éther de pétrole-acétate d'éthyle (9:1). On obtient 3 g de produit attendu. F ~ 150°C.

$/\alpha/_D = +125° \pm 2{,}5°$ (c = 1% chloroforme).

Stade b: 3,3-diméthoxy 5α-10α-époxy 17β-éthynyl 17α-hydroxy estr-9(11)-ène.

On mélange 2,6 g du produit obtenu au stade a, 12 $cm^3$ de chlorure de méthylène et une goutte de pyridine. On refroidit à 0°C, ajoute 0,12 $cm^3$ d'hexachloroacétone et 0,65 $cm^3$ d'eau oxygénée (200 volumes). Après une heure sous agitation, on ajoute 13 $cm^3$ de chloroforme puis poursuit l'agitation pendant 18 heures. On verse dans 100 $cm^3$ d'une solution saturée de thiosulfate de sodium, agite pendant 10 minutes, extrait au chloroforme, lave la phase organique avec une solution aqueuse saturée de chlorure de sodium, sèche et évapore le solvant. On obtient 2,8 g de produit attendu, utilisé tel quel pour le stade suivant. (Le produit renferme une faible proportion d'époxyde β.)

Exemple 7:

17β-hydroxy 17α-phényl 11β(4-diméthylaminophényl) estra 4,9-dièn-3-one.

Stade A: 3,3-[1,2-éthane diyl bis (oxy)] 11β-(4 diméthylamino-phényl) estr-9-ène 5α-hydroxy 17-one.

a) préparation du magnésien.

On mélange sous gaz inerte 29 g de tournure de magnésium et 50 $cm^3$ de tétrahydrofuranne anhydre. On introduit en 2 heures et demie en maintenant la température à 35°C±5°C, un mélange de 200 g de 4-diméthylamino bromo benzène dans 950 $cm^3$ de tétrahydrofuranne anhydre. On obtient ainsi une solution 0,8 M de magnésien attendu.

b) addition du magnésien.

On mélange sous gaz inerte 25 g de 3,3-[1,2-éthane diyl bis (oxy)] 5α-10α-époxy estr-9(11)-ène 17-one, 500 $cm^3$ de tétrahydrofuranne anhydre et 0,757 g de chlorure cuivreux. On refroidit à 0+5°C et ajoute goutte à goutte en 1 heure 15 minutes 284 $cm^3$ de la solution de magnésien préparée ci-dessus. On agite ensuite pendant 15 minutes, verse dans une solution saturée de chlorure d'ammonium, extrait à l'acétate d'éthyle, lave la phase organique avec une solution saturée de chlorure d'ammonium puis avec une solution saturée de chlorure de sodium. On sèche la phase organique et évapore à sec sous pression réduite. On obtient 46 g de produit brut que l'on chromatographie sur silice en éluant au mélange éther de pétrole-acétate d'éthyle (1:1) à 1 pour mille de triéthylamine, on obtient 17,76 g de produit attendu. F = 178°C.

Les fractions impures de produit obtenu sont à nouveau chromatographiées sur silice en éluant au mélange éther de pétrole-acétone (8:2) à 1 pour mille de triéthylamine. On obtient à nouveau 6,35 g de produit attendu. F = 176°C. Le produit ainsi obtenu est utilisé tel quel pour le stade suivant.

Stade B: 3,3-[1,2-éthane diyl bis (oxy)] 5α, 17β-dihydroxy 11β-(4-diméthylaminophényl) 17α-phényl estr-9-ène.

On ajoute en 30 minutes à +25°C à une solution de 33,3 $cm^3$ de phényllithium (1,5 M), une solution de 4,51 g du produit obtenu au stade A dans 45,1 $cm^3$ de tétrahydrofuranne anhydre. On agite pendant 4 heures à température ambiante, verse dans une solution aqueuse saturée de chlorure d'ammonium, extrait à l'éther, lave la phase organique avec une solution aqueuse saturée de chlorure de sodium, sèche et évapore le solvant. On obtient 5,6 g de produit brut que l'on chromatographie sur silice en éluant au mélange chlorure de méthylène-acétone (9:1) à 1 pour mille de triéthylamine. On obtient 1,16 g de produit attendu que l'on cristallise dans le mélange chlorure de méthylène-éther isopropylique. F = 240°C.

$/\alpha/_D = +53° \pm 2{,}5°$ (c = 0,5% chloroforme).

Stade C: 17β-hydroxy 17α-phényl 11β-(4-diméthylaminophényl) estra 4,9-dièn-3-one.

On mélange sous gaz inerte 1,5 g du produit obtenu au stade B, dans 45 $cm^3$ de méthanol. On refroidit à 0 +5°C et introduit 3 $cm^3$ d'acide chlorhydrique 2N. On agite pendant une heure à 0

+5°C, puis ajoute 90 $cm^3$ d'éther et 90 $cm^3$ d'une solution aqueuse 0,25 M de bicarbonate de sodium. On agite pendant 5 minutes, décante, extrait à l'éther, lave la phase organique avec une solution aqueuse saturée de chlorure de sodium, sèche et évapore le solvant. On obtient 1,30 g de produit que l'on purifie par chromatographie sur silice en éluant au mélange éther de pétrole-acétate d'éthyle (1:1). On obtient 0,93 g de produit attendu, que l'on cristallise dans le mélange chlorure de méthylène-éther isopropylique. F = 226°C. $/\alpha/_D$ = +151,5° (c = 0,4% chloroforme).

Le produit de départ du stade A a été préparé comme suit.

On mélange 11,18 g de 3,3-[1,2-éthane diyl bis (oxy)] estr-5(10) 9(11)-dièn-17-one et 56 $cm^3$ de chlorure de méthylène, ajoute 2 gouttes de pyridine, refroidit à 0°C et introduit 4,3 $cm^3$ de sesquihydrate d'hexafluoroacétone, puis ajoute 1,6 $cm^3$ d'eau oxygénée à 85%. On maintient sous agitation et sous gaz inerte à 0°C pendant 23 heures. On verse ensuite dans un mélange renfermant 200 $cm^3$ de solution 0,5 M de thiosulfate de sodium et 200 g de glace. On maintient pendant 30 minutes sous agitation puis on extrait au chlorure de méthylène renfermant une trace de pyridine. On lave la phase organique à l'eau, la sèche et évapore le solvant. On obtient 11,4 g de produit attendu, utilisé tel quel pour le stade suivant.

Exemple 8:

11β-(4-diméthylaminophényl) 17β-hydroxy 23-méthyl (17α) 19,21-dinorchola-4,9,23-trièn-20-yn-3-one.

Stade A: 3,3-[1,2-éthane diyl bis (oxy)] 11β-(4-diméthylaminophényl) 23-méthyl (17α) 19,21-dinorchola-9,23-dièn-20-yn-5α-17β-diol.

On mélange sous gaz inerte 4,5 g de terbutylate de potassium avec 90 $cm^3$ de tétrahydrofuranne anhydre. On refroidit à -10°C et ajoute 10,61 $cm^3$ de 2-méthyl 1-buten 3-yne. On agite pendant 15 minutes à -10°C, puis ajoute en 15 minutes une solution de 4,5 g de produit obtenu au stade A de l'exemple 7, dans 45 $cm^3$ de tétrahydrofuranne anhydre. On agite pendant 30 minutes à -10°C puis pendant 4 heures à 0 +5°C. On verse dans 500 $cm^3$ d'une solution aqueuse saturée de chlorure d'ammonium, extrait à l'acétate d'éthyle, lave la phase organique avec une solution aqueuse saturée de chlorure de sodium, sèche et évapore à sec. On obtient 5,56 g de produit attendu brut. F = 205°C. Le produit est utilisé tel quel pour la suite de la synthèse.

Le produit brut, chromatographié sur silice en éluant au mélange chlorure de méthylène-acétate d'éthyle (9:1) à 1 pour mille de triéthylamine puis recristallisé dans l'acétate d'éthyle, fond à 215°C.

Stade B: 11β-(4-diméthylaminophényl) 17β-hydroxy 23-méthyl (17α) 19,21-dinorchola 4,9,23-trièn-20-yn-3-one.

On mélange sous gaz inerte 5 g du produit obtenu au stade A avec 300 $cm^3$ de méthanol et 10 $cm^3$ d'acide chlorhydrique 2N. On agite pendant 15 minutes à 20°C, ajoute 300 $cm^3$ de chlorure de méthylène puis 300 $cm^3$ d'une solution aqueuse 0,25M de bicarbonate de sodium. Après 10 minutes sous agitation, on décante, extrait au chlorure de méthylène, lave la phase organique à l'eau, sèche et évapore à sec. On obtient 4,5 g de produit attendu brut que l'on chromatographie sur silice en éluant au mélange éther de pétrole-acétate d'éthyle (1:1). On obtient après recristallisation du produit dans l'oxyde de diisopropyle, 2,01 g de produit attendu. F = 185°C. $/\alpha/_D$ = +88,5°±1,5° (c = 1% chloroforme).

Exemple 9:

11β(4-diméthylaminophényl) 17β-méthoxy 23-méthyl (17α) 19,21-dinorchola-4,9,23-trièn-20-yn-3-one.

On mélange sous gaz inerte 4,5 g de tert-butylate de potassium dans 90 $cm^3$ de tétrahydrofuranne anhydre. On refroidit la suspension à -10°C puis ajoute, goutte à goutte, 10,61 $cm^3$ de 2-méthyl 1-butèn 3-yne. On agite pendant 15 minutes à -10°C, puis ajoute en 15 minutes, 4,5 g du produit obtenu au stade A de l'exemple 7, dans 45 $cm^3$ de tétrahydrofuranne anhydre. On agite pendant 30 minutes à -10°C, puis pendant 4 heures à 0 +5°C. On ajoute ensuite 7,5 $cm^3$ d'iodure de méthyle puis maintient pendant 30 minutes sous agitation dans un bain de glace. On verse ensuite le mélange dans 500 $cm^3$ d'acide chlorhydrique 0,1 N. On agite pendant 30 minutes à température ambiante, extrait à l'acétate d'éthyle, lave la phase organique par une solution aqueuse saturée de bicarbonate de sodium, puis par une solution aqueuse saturée de chlorure de sodium.

On sèche et évapore le solvant. On chromatographie le résidu sur silice en éluant au mélange chlorure de méthylène-acétate d'éthyle (95:5). On obtient 2,7 g de produit attendu, que l'on recristallise dans le méthanol.
F = 105°C.

Exemple 10:

21-chloro 17β-hydroxy 11β(4-diméthylamino-phényl) (17α)19-norpregna-4,9-dièn-20-yn 3-one.

Stade A: 21-chloro 3,3[1,2-éthane diyl bis (oxy)]11β(4-diméthylaminophényl) (17α)19-norpregna-9-èn 20-yn 5α, 17β-diol.

Préparation du lithien

On mélange sous gaz inerte 77,5 $cm^3$ d'une solution 1M de butyllithium dans l'hexanne avec 310 $cm^3$ d'éther éthylique anhydre. On refroidit à 0 +5°C et ajoute en 45 minutes une solution de 7 $cm^3$ de trichloréthylène dans 28 $cm^3$ d'éther éthylique anhydre. On agite pendant une heure en laissant revenir la température à 20°C.

Condensation

On refroidit à 0+5°C le mélange obtenu ci-dessus et y ajoute goutte à goutte, en trente minutes, une solution de 7 g du produit obtenu au stade A de l'exemple 7 dans 70 $cm^3$ de tétrahydrofuranne. On agite pendant trente minutes, à 0+5°C, puis laisse la température revenir à 20°C, verse lentement dans une solution aqueuse saturée de chlorure d'ammonium, décante, extrait au chlorure de méthylène, lave la phase organique à l'eau, la sèche et évapore le solvant. On obtient 8,5 g de produit brut (F = 220°C) que l'on introduit dans 42,5 $cm^3$ d'oxyde de diisopropyle. On agite pendant 30 minutes, essore et obtient 6,38 g de produit attendu. F = 230°C.

On peut purifier le produit par chromatographie sur silice en éluant au mélange benzène-acétate d'éthyle (7:3) à 1 pour mille de triéthylamine. Par dissolution de ce produit dans le chlorure de méthylène et addition d'oxyde de diisopropyle, on obtient un produit cristallisé fondant à 240°C.
$/\alpha/_D$ = −83,5°±1,5° (c = 1% chloroforme).

Stade B: 21-chloro 17β-hydroxy 11β(4-diméthylaminophényl) (17α)19-norpregna-4,9-dièn-20-yn 3-one.

On mélange sous gaz inerte 6,38 g du produit obtenu au stade précédent et 191,4 $cm^3$ d'éthanol à 95%. On ajoute 15 $cm^3$ d'acide chlorhydrique 2N, agite pendant 1 heure, ajoute 300 $cm^3$ de chlorure de méthylène puis 200 $cm^3$ d'une solution aqueuse 0,25 M de bicarbonate de sodium. On décante, extrait au chlorure de méthylène, lave la phase organique à l'eau, sèche et évapore le solvant. On obtient 6 g de produit brut que l'on chromatographie sur silice en éluant au mélange benzène–acétate d'éthyle (7:3). On obtient 3,95 g de produit attendu, que l'on cristallise dans l'acétate d'éthyle. F = 240°C.
$/\alpha/_D$ = +111°±2° (c = 1% chloroforme).

Exemple 11:
N-oxyde du 21-chloro 17β-hydroxy 11β-(4-diméthylaminophényl) (17α) 19-norpregna 4,9-dièn-20-yn-3-one.

On mélange sous gaz inerte 1,2 g du produit obtenu à l'exemple 10 dans 24 $cm^3$ de chlorure de méthylène. On refroidit à 0 +5°C et ajoute un mélange de 0,54 g d'acide métachloroperbenzoïque (à 85%) dans 10,8 $cm^3$ de chlorure de méthylène. On agite pendant 1 heure à 0 +5°C, verse dans une solution 0,2N de thiosulfate de sodium, extrait au chlorure de méthylène, lave la phase organique par une solution aqueuse saturée de bicarbonate de sodium puis à l'eau, sèche et évapore le solvant. On obtient 1,3 g de produit brut. On purifie ce produit par chromatographie sur silice en éluant au mélange chlorure de méthylène–méthanol (7:3). On obtient 1,15 g de produit attendu.
$/\alpha/_D$ = +47,5°±2,5° (c = 0,7% chloroforme).

Exemple 12:
N-oxyde du 21-chloro 9α-10α-époxy 17β-hydroxy 11β-(4-diméthylaminophényl) (17α) 19-norpregn-4-èn-20-yn-3-one.

On dissout 1,18 g de produit obtenu à l'exemple 10 dans 23,6 $cm^3$ de chlorure de méthylène, refroidit à 0 +5°C et ajoute en 15 minutes un mélange de 1,17 g d'acide métachloroperbenzoïque (à 85%) dans 23,4 $cm^3$ de chlorure de méthylène. On agite pendant 2 heures à 20°C, ajoute à nouveau 0,117 g d'acide métachloroperbenzoïque, agite encore pendant 1 heure, verse le mélange dans une solution 0,2N de thiosulfate de sodium, extrait au chlorure de méthylène, lave la phase organique avec une solution aqueuse saturée de bicarbonate de sodium puis à l'eau, sèche et évapore à sec. On obtient 1,14 g de produit brut. F = 220°C.

On purifie le produit par chromatographie sur silice en éluant au mélange chlorure de méthylène–méthanol (8:2) et obtient 1 g de produit attendu. F = 270°C.
$/\alpha/_D$ = +39,5°±2,5° (c = 0,5% chloroforme).

Exemple 13:
21-chloro 9α-10α-époxy 17β-hydroxy 11β-(4-diméthylaminophényl) (17α) 19-norpregn-4-èn-20-yn-3-one.

On mélange sous gaz inerte 0,63 g du produit obtenu à l'exemple 12 avec 6,3 $cm^3$ d'acide acétique. On ajoute 0,34 g de triphénylphosphine, agite pendant 45 minutes à température ambiante, verse dans l'eau, extrait au chlorure de méthylène, lave la phase organique à l'eau, la sèche et évapore le solvant. On obtient 0,9 g de produit que l'on chromatographie sur silice en éluant au mélange éther de pétrole–acétate d'éthyle (1:1). On recristallise le produit ainsi obtenu dans un mélange chlorure de méthylène-éther isopropylique et obtient 0,346 g de produit attendu. F = 265°C.
$/\alpha/_D$ = +45°±2° (c = 0,8% chloroforme).

Exemple 14:
17β-hydroxy 11β-(4-diméthylaminophényl) 21-phényl (17α) 19-norpregna-4,9-dièn-20-yn-3-one.

Stade A: 21-phényl 3,3-[1,2-éthane diyl bis (oxy)]11β-(4-diméthylamino phényl) 5α-17β-dihydroxy (17α) 19-norpregn-9-èn-20-yne.

On mélange sous gaz inerte 4,17 g de terbutylate de potassium dans 83 $cm^3$ de tétrahydrofuranne anhydre. On agite pendant 5 minutes puis refroidit à −10°C et ajoute goutte à goutte 4,5 $cm^3$ de phényl acétylène. On agite la suspension pendant 5 minutes puis ajoute goutte à goutte à −10°C, une solution de 4,17 g de produit obtenu au stage A de l'exemple 7 dans 41 $cm^3$ de tétrahydrofuranne anhydre. A la fin de l'introduction, on amène la température à 0°C, puis après 1 heure on verse le mélange dans une solution saturée de chlorure d'ammonium. On extrait à l'éther, lave la phase organique à l'aide d'une solution aqueuse saturée de chlorure de sodium, sèche, concentre à sec et obtient 4,7 g de produit que l'on chromatographie sur silice en éluant au mélange chlorure de méthylène–acétone (95:5). On obtient 3,71 g de produit attendu. F = 168°C.
$/\alpha/_D$ = −119,5°±2° (c = 1% chloroforme).

Stade B: 17β-hydroxy 11β-(4-diméthylaminophényl) 21-phényl (17α) 19-norpregna-4,9-dièn-20-yn-3-one.

On dissout 3,49 g de produit obtenu comme décrit au stade précédent, dans 68 $cm^3$ de méthanol puis ajoute 6,3 $cm^3$ d'acide chlorhydrique 2N. Après 30 minutes sous agitation, on verse dans un mélange de 180 $cm^3$ d'éther éthylique et 90 $cm^3$ d'une solution 0,25 M de bicarbonate de sodium. On agite pendant 5 minutes, décante, extrait à l'éther, lave les phases organiques avec une solution 0,25 M de bicarbonate de sodium puis avec une solution saturée de chlorure de sodium. On sèche, évapore le solvant et obtient 4,35 g de produit que l'on purufie par chromatographie sur silice en éluant au mélange chlorure de méthylène-acétone (95:5). On obtient 2,13 g de produit attendu, après cristallisation dans l'éther isopropylique.

$/\alpha/_D = +22{,}5° \pm 1°$ (c = 1% chloroforme).

Exemple 15:

17β-hydroxy 11β-(4-diméthylaminophényl) 17α-(propa-1,2-diényl) estra-4,9-dièn-3-one.

Stade A:. 11β-(4-diméthylaminophényl) 3,3-[1,2-éthane diyl bis (oxy)] 17α-(propa-1,2-diényl) estr-9-èn-5α-17β-diol et 11β-(4-diméthylaminophényl) 3,3-[1,2-éthane diyl bis (oxy)] 17α-(prop-2-ynyl) estr-9-èn-5α-17β-diol.

Préparation du lithien.

Dans 50 $cm^3$ de tétrahydrofuranne anhydre à 0+5°C, on fait barboter de l'Allène jusqu'à absorption de 2,1 g. On refroidit à -70°C et ajoute en 15 minutes 23,9 $cm^3$ d'une solution 1,3M de butyllithium dans l'hexanne. On agite le mélange obtenu pendant 15 minutes à -70°C.

Condensation.

A la solution de lithien obtenue ci-dessus, on ajoute à -70°C en 25 minutes une solution de 3,5 g du produit obtenu au stade A de l'exemple 7 dans 35 $cm^3$ de tétrahydrofuranne anhydre. On agite pendant 1 heure à -70°C, verse lentement dans une solution aqueuse saturée glacée, de chlorure d'ammonium. On extrait à l'éther, lave la phase organique avec une solution saturée de chlorure de sodium, sèche et évapore le solvant. On obtient 3,4 g de produit que l'on chromatographie sur silice en éluant au mélange éther de pétrole-acétate d'éthyle (1:1) à 1 pour mille de triéthylamine. On isole ainsi:

a) 1,73 g d'isomère 17α-(propa-1,2-diényl)
F = 178°C.
$/\alpha/_D = -32° \pm 2°$ (c = 0,7% chloroforme).

b) 1,5 g d'isomère 17α-(prop-2-ynyl)
F = 150°C.
$/\alpha/_D = -15° \pm 2°$ (c = 0,9% chloroforme).

Stade B: 17β-hydroxy 11β-(4-diméthylaminophényl) 17α-(propa-1,2-diényl) estra-4,9-dièn-3-one.

On mélange sous gaz inerte 1,73 g d'isomère 17α-(propa-1,2-dienyl) obtenu au stade A, 51,8 $cm^3$ d'éthanol à 95% et 3,5 $cm^3$ d'acide chlorhydrique 2N. On agite à 20°C pendant 1 heure, ajoute 50 $cm^3$ de chlorure de méthylène puis 50 $cm^3$ d'une solution 0,25 M de bicarbonate de sodium; on décante, extrait au chlorure de méthylène, lave à l'eau, sèche et évapore le solvant. On obtient 1,51 g de produit que l'on dissout dans 10 $cm^3$ de chlorure de méthylène à chaud. On y ajoute 15 $cm^3$ d'éther isopropylique, concentre et laisse au repos. On isole ainsi 1,23 g de produit attendu que l'on cristallise à nouveau dans le mélange chlorure de méthylène-éther isopropylique. On obtient finalement 1,11 g de produit attendu. F = 228°C.

$/\alpha/_D = +139{,}5° \pm 3°$ (c = 0,8% chloroforme).

Exemple 16:

17β-hydroxy 11β-(4-diméthylaminophényl) 17α-(prop-2-ynyl) estra-4,9-dièn-3-one.

On mélange 0,94 g d'isomère 17α-(prop-2-ynyl) obtenu au stade A de l'exemple 15, 28,2 $cm^3$ d'éthanol à 95% et 2 $cm^3$ d'acide chlorhydrique 2N. On agite à 20°C pendant 1 heure, ajoute 50 $cm^3$ de chlorure de méthylène et 50 $cm^3$ d'une solution 0,25 M de bicarbonate de sodium, agite pendant 5 minutes, décante et extrait au chlorure de méthylène. On lave la phase organique à l'eau, la sèche et évapore le solvant. On chromatographie le produit obtenu sur silice en éluant au mélange éther de pétroleacétate d'éthyle (1:1). On obtient ainsi 0,42 g de produit attendu amorphe.

$/\alpha/_D = +143° \pm 3°$ (c = 0,8% chloroforme).

Exemple 17:

17α-éthynyl 17β-hydroxy 11β-(4-diméthylaminophényl) estra-4,9-dièn-3-one.

Stade A: 17β-cyano 11β-(4-diméthylaminophényl) 3,3-[1,2-éthane diyl bis (oxy)] 17α-(triméthyl silyloxy) estr-9-èn-5α-ol.

On ajoute sous gaz inerte à température ambiante, à une suspension de 2,05 g de complexe bromure de cuivre-diméthyl sulfure dans 10 $cm^3$ de tétrahydrofuranne anhydre, une solution de 18 m/moles de bromure de (4-diméthylaminophényl) magnésium dans le tétrahydrofuranne anhydre, puis on agite pendant 20 minutes et ajoute 20 $cm^3$ de triéthylamine anhydre. On ajoute ensuite 0,95 g de 17β-cyano 17α-(triméthyl silyloxy) 3,3-[1,2-éthane diyl bis (oxy)] 5α-10α-époxy estr-9-(11)ène en solution dans le tétrahydrofuranne anhydre, agite pendant 15 heures à température ambiante, verse dans 50 $cm^3$ d'une solution saturée de chlorure d'ammonium, décante, extrait à l'éther, lave la phase organique à l'eau, sèche et évapore le solvant. On purifie le résidu par chromatographie sur silice en éluant au mélange benzène-acétate d'éthyle (8:2). On obtient 1,1 g de produit attendu que l'on recristallise dans l'éther isopropylique. F = 247°C.

$/\alpha/_D = -12{,}5°$ (c = 1% chloroforme).

Stade B: 17α-éthynyl 3,3-[éthane diyl bis (oxy)] 11β-)4-diméthylaminophényl) estr-9-èn-5α-17β-diol.

A 0,8 g de produit obtenu au stade A dans 8 $cm^3$ d'éthylène diamine, on ajoute 1 g de complexe acétylure de lithium éthylène diamine, puis maintient sous agitation et sous gaz inerte à ~ 50°C pendant 1 heure et demie. On refroidit à 20°C puis

verse dans une solution de chlorure d'ammonium. On extrait à l'éther et au chlorure de méthylène. On sèche la phase organique et évapore le solvant. On chromatographie le résidu sur silice en éluant au mélange benzène-acétate d'éthyle (7:3), recristallise le produit obtenu dans l'éther isopropylique et obtient 0,43 g de produit attendu. F = 199°C.

/α/$_D$ = -43°±1,5° (c = 1% chloroforme).

Stade C: 17α-éthynyl 17β-hydroxy 11β-(4-diméthylaminophényl) estra-4,9-dièn-3-one.

A une solution de 0,25 g de produit obtenu au stade B, dans 6 cm³ de méthanol, on ajoute 1 cm³ d'acide chlorhydrique 2N. On agite pendant 40 minutes à 20°C, verse dans de l'eau contenant 2,5 cm³ d'hydroxyde de sodium 1N, extrait à l'éther, sèche la phase organique et évapore le solvant. On chromatographie le résidu sur silice en éluant au mélange benzène-acétate d'éthyle (7:3), et obtient 0,25 g de produit attendu.

Analyse: $C_{28}H_{33}NO_2$ (415,54)

| | | | |
|---|---|---|---|
| Calculé: | C% 80,92 | H% 8,00 | N% 3,37 |
| Trouvé: | C% 80,7 | H% 8,1 | N% 3,1 |

Exemple 18:

17α-éthynyl 17β-hydroxy 11β-(4-diméthylaminophényl) estra 4,9-dièn-3-one.

Stade A: 11β-(4-diméthylaminophényl) 3,3-[1,2-éthane diyl bis (oxy)] 5α-17β-dihydroxy 17α-éthynyl estr-9-ène.

On dissout sous gaz inerte 6 g de produit obtenu au stade A de l'exemple 7, dans 180 cm³ de tétrahydrofuranne, puis ajoute 12,25 g du complexe acétilure de lithium-éthylène diamine. On porte la température à 55°C, agite pendant 4 heures, refroidit, puis verse dans 600 cm³ d'une solution saturée glacée de chlorure d'ammonium. On extrait à l'éther, lave la phase organique avec une solution saturée de chlorure de sodium, sèche et évapore le solvant. On purifie le résidu obtenu par chromatographie sur silice en éluant au mélange benzène-acétate d'éthyle (7:3) à 1 pour mille de triéthylamine et obtient 4,5 g de produit attendu que l'on peut recristalliser dans le mélange chlorure de méthylène-oxyde de diisopropyle. F = 202°C.

/α/$_D$ = -47,5°±1,5° (c = 1% chloroforme)

Stade B: 17α-éthynyl 17β-hydroxy 11β-(4-diméthylaminophényl) estra 4,9-dièn-3-one.

On mélange 2 g du produit obtenu au stade A dans 50 cm³ d'éthanol à 95%. On ajoute à la suspension 5 cm³ d'acide chlorhydrique 2N. On maintient sous agitation pendant 1 heure à 20°C, ajoute 100 cm³ d'éther éthylique puis 100 cm³ d'une solution 0,25 M de bicarbonate de sodium. On décante, extrait à l'éther, lave la phase organique avec une solution aqueuse saturée de chlorure de sodium, sèche et évapore à sec. On chromatographie le résidu obtenu sur silice en éluant au mélange éther de pétrole-acétate d'éthyle (6:4) et isole 1,52 g de produit attendu que l'on peut recristalliser dans l'oxyde de diisopropyle. F = 172°C.

/α/$_D^{20}$ = +182°±2,5° (c = 1% chloroforme).

Exemple 19:

17β-hydroxy 11β-(3-diméthylaminophényl) 17α-(prop-1-ynyl) estra-4,9-dièn-3-one.

Stade A: 11β-(3-diméthylaminophényl) 3,3-[1,2-éthane diyl bis (oxy)] 17α-(prop-1-ynyl) estr-9-èn-5α-17β-diol.

Préparation du magnésien.

On mélange sous gaz inerte 1,46 g de magnésium et 5 cm³ de tétrahydrofuranne anhydre. On introduit en 45 minutes en maintenant la température vers 50°C, 10 g de métabromodiméthylaniline dans 45 cm³ de tétrahydrofuranne anhydre. (La réaction a été amorcée par addition de dibromométhane.) On maintient pendant 1 heure sous agitation et obtient une solution 0,95 M du magnésien attendu.

Condensation.

On mélange sous gaz inerte 3,7 g de 3,3-[1,2-éthylène dioxy bis (oxy)] 17α-(prop-1-ynyl) estr-9 (11) èn-5α-10α-époxy 17β-ol, 74 cm³ de tétrahydrofuranne anhydre et 99 mg de chlorure cuivreux. On refroidit à 0 +5°C, puis ajoute en 30 minutes 42,2 cm³ de la solution de magnésien obtenue ci-dessus. On agite pendant 30 minutes à 0 +5°C, verse dans une solution aqueuse saturée de chlorure d'ammonium, extrait à l'éther, lave la phase organique avec une solution aqueuse saturée de chlorure de sodium, sèche et évapore le solvant. On chromatographie le résidu sur silice en éluant au mélange chlorure de méthylène-acétone (9:1) à 1 pour mille de triéthylamine. On obtient 3,5 g de produit attendu. F = 262°C.

/α/$_D$ = -64°±1,5° (c = 1% chloroforme).

On isole également 0,66 g de l'isomère 5βOH correspondant. F = 210°C.

/α/$_D$ = +32,5°±1° (c = 0,8% chloroforme).

Stade B: 17β-hydroxy 11β-(3-diméthylaminophényl) 17α-(prop-1-ynyl) estra 4,9-dièn-3-one.

On mélange sous gaz inerte 3,3 g du produit obtenu au stade A avec 100 cm³ de méthanol, refroidit à 0 +5°C et ajoute 10 cm³ d'acide chlorhydrique 2N. On agite pendant 1 heure à 0 +5°C, ajoute 200 cm³ d'oxyde de diéthyle, puis 200 cm³ d'une solution 0,25 M de bicarbonate de sodium. On agite pendant 5 minutes, décante, extrait à l'oxyde de diéthyle, lave la phase organique avec une solution saturée de chlorure de sodium, sèche et évapore le solvant. On obtient 3 g de produit que l'on chromatographie sur silice en éluant au mélange benzène-acétate d'éthyle (7:3). On isole 1,43 g de produit attendu amorphe.

/α/$_D$ = +43°±2,5° (c = 1% chloroforme).

Exemple 20:

N-oxyde de 17β-hydroxy 11β-(4-diméthylaminophényl) 17α-(prop-1-ynyl) estra 4,9-dièn-3-one.

On mélange 1,5 g de produit obtenu à

l'exemple 4, avec 30 cm³ de chlorure de méthylène. On refroidit à 0 +5°C et ajoute en 10 minutes une solution de 0,71 g d'acide métachloroperbenzoïque (à 85%) dans 14,2 cm³ de chlorure de méthylène. On agite à 0 +5°C pendant 1 heure, verse dans 100 cm³ une solution 0,2N de thiosulfate de sodium, décante, extrait au chlorure de méthylène, lave la phase organique avec une solution 0,5 M de bicarbonate de sodium, sèche et évapore le solvant. On dissout le résidu dans 20 cm³ de chlorure de méthylène et ajoute 20 cm³ d'oxyde de diisopropyle. On amorce la cristallisation, laisse au repos, essore les cristaux formés et les sèche. On obtient 1,4 g de produit attendu. F = 210°C.
$/\alpha/_D$ = +73,5°±2° (c = 1% chloroforme).

Exemple 21:
11β-(4-diméthylaminophényl) 17β-hydroxy estra 4,9-dièn-3-one.

On mélange 1 g du produit obtenu au stade A de l'exemple 7 dans 20 cm³ de tétrahydrofuranne à 10% d'eau. Après dissolution, on ajoute 106 mg de borohydrure de sodium, agite pendant 1 heure, verse dans 200 cm³ d'eau, extrait au chlorure de méthylène, lave la phase organique avec une solution saturée de chlorure de sodium, sèche et évapore le solvant. On obtient 1,3 g de produit 5α-17β-dihydroxy.

On introduit 0,63 g de produit obtenu ci-dessus dans un mélange de 12 cm³ de méthanol et 2,4 cm³ d'acide chlorhydrique 2N. On agite pendant 1 heure 30 à température ambiante, verse dans une solution de bicarbonate de sodium, extrait à l'éther, lave la phase organique avec une solution saturée de chlorure de sodium, sèche et évapore le solvant. On chromatographie le résidu sur silice en éluant au mélange éther de pétrole–acétate d'éthyle (6:4). On triture le résidu dans l'éther de pétrole, l'essore et obtient 0,38 g de produit attendu. F = 130°C.
$/\alpha/_D$ = +277°±5° (c = 0,5% chloroforme).

Exemple 22:
17β-hydroxy 11β-(4-diméthylaminophényl) 17α-(prop-2-ényl) estra 4,9-dièn-3-one.

Stade A: 3,3-[1,2-éthane diyl bis (oxy)] 11β-(4-diméthylaminophényl) 17α-(prop-2-ényl) estr-9-ène 5α-17β-diol.

Dans 55,5 cm³ d'une solution 0,7 M de bromure d'allyl magnésium dans l'éther, on introduit sous gaz inerte à 20°C en 15 minutes, une solution de 3,5 g du produit obtenu au stade A de l'exemple 7 dans 35 cm³ de tétrahydrofuranne. On agite à 20°C pendant 1 heure, verse dans une solution aqueuse saturée de chlorure d'ammonium, extrait à l'éther, lave la phase organique avec une solution aqueuse saturée de chlorure de sodium, sèche et évapore le solvant. On dissout le résidu obtenu dans 10 cm³ de chlorure de méthylène. On y ajoute 15 cm³ d'oxyde de diisopropyle, concentre, puis laisse au repos. On essore les cristaux formés, les rince à l'oxyde de diisopropyle, les sèche et obtient 2,76 g de produit attendu. F = 198°C.

Analyse: $C_{31}H_{43}NO_4$ (493,69)

| | | | |
|---|---|---|---|
| Calculé: | C% 74,42 | H% 8,78 | N% 2,83 |
| Trouvé: | C% 74,0 | H% 8,7 | N% 2,9 |

Stade B: 17β-hydroxy 11β-(4-diméthylaminophényl) 17α-(prop-2-ényl) estra 4,9-dièn-3-one.

On met en suspension 2,2 g du produit obtenu au stade A dans 66 cm³ de méthanol, puis ajoute 4,5 cm³ d'acide chlorhydrique 2N. On agite pendant 30 minutes à 20°C, ajoute 132 cm³ d'oxyde de diéthyle, puis 132 cm³ d'une solution aqueuse 0,25 M de bicarbonate de sodium. On décante, extrait à l'oxyde de diéthyle, lave avec une solution aqueuse saturée de chlorure de sodium, sèche et évapore le solvant. On chromatographie le résidu sur silice en éluant au mélange benzène–acétate d'éthyle (7:3), reprend le produit obtenu dans un mélange de 15 cm³ d'oxyde de diisopropyle et 7,5 cm³ de chlorure de méthylène, concentre puis laisse au repos. On essore et rince à l'oxyde de diisopropyle les cristaux obtenus, et obtient 1,365 g de produit attendu. F = 182°C.
$/\alpha/_D$ = +206,5°±3° (c = 1% chloroforme).

Exemple 23:
17β-hydroxy 11β-[4-(N,N-diméthylaminométhyl) phényl]17α-(prop-1-ynyl) estra 4,9-dièn-3-one.

Stade A: 3,3-[1,2-éthane diyl bis (oxy)] 11β-[4-(N,N-diméthyl-aminométhyl) phényl] 17α-(prop-1-ynyl) estr-9-èn-5α-17β-diol.

Préparation du magnésien.

On mélange sous gaz inerte 5,5 g de magnésium et 10 cm³ de tétrahydrofuranne anhydre. On introduit en 1 heure 30 minutes en maintenant la température à +45°/+50°C, 42,8 g de 4-(N,N-diméthylaminométhyl) bromobenzène dans 190 cm³ de tétrahydrofuranne anhydre. La réaction a été amorcée par addition de dibromoéthane. Après la fin d'introduction, on maintient sous agitation pendant 1 heure. On obtient ainsi la solution de magnésien attendue 0,85 M.

Addition sur l'époxyde.

On mélange sous gaz inerte 10 g de 3,3-[1,2-éthane diyl bis (oxy)] 17α-(prop-1-ynyl) estr-9(11)-èn-5α-10α-époxy 17β-ol, 200 cm³ de tétrahydrofuranne anhydre et 0,27 g de chlorure cuivreux. On refroidit à 0 +5°C et introduit en 1 heure 127 cm³ de la solution de magnésien préparée ci-dessus. On agite ensuite 15 minutes, verse dans une solution aqueuse saturée de chlorure d'ammonium, extrait à l'éther, lave la phase organique avec une solution aqueuse saturée de chlorure de sodium, sèche et évapore le solvant. On chromatographie le résidu sur silice en éluant au mélange chlorure de méthylène–méthanol (9:1) à 1 pour mille de triéthylamine. On obtient 10,1 g de produit que l'on cristallise par dissolution dans le chlorure de méthylène et addition de quelques cm³ de méthanol puis d'oxyde de diisopropyle. Après concentration et maintien au repos pendant 6 heures, on essore le produit obtenu et obtient 7,37 g de produit attendu. F = 186°C.
/α/$_D$ = -63°±2,5° (c = 0,5% chloroforme).

Stade B: 17β-hydroxy 11β-[4-(N,N-diméthyl-aminométhyl) phényl]17α-(prop-1-ynyl) estra 4,9-dièn-3-one.

On mélange sous gaz inerte 7,37 g du produit obtenu au stade A dans 147,4 cm³ de méthanol et 15 cm³ d'acide chlorhydrique 2N. On agite à 20°C pendant 1 heure, ajoute 300 cm³ d'oxyde de diéthyle et 300 cm³ d'une solution aqueuse 0,25 M de bicarbonate de sodium, décante, extrait à l'oxyde de diéthyle, lave la phase organique avec une solution aqueuse saturée de chlorure de sodium, sèche et évapore le solvant. On recristallise le produit obtenu en le dissolvant dans un mélange d'oxyde de diisopropyle et de chlorure de méthylène, puis en concentrant la solution et en laissant au repos. On essore et sèche les cristaux formés. On obtient ainsi 3,74 g de produit attendu. F = 190°C.
/α/$_D$ = +84,5°±2° (c = 0,8% chloroforme).

Exemple 24:

17β-hydroxy 11β-(4-pyrrolidinylphényl) 17α-(prop-1-ynyl) estra 4,9-dièn-3-one.

Stade A: 3,3-[1,2-éthane diyl bis (oxy)] 11β-(4-pyrrolidinylphényl) 17α-(prop-1-ynyl) estr-9-èn- 5α-17β-diol.

Préparation du magnésien.

On mélange sous gaz inerte 4 g de magnésium et 10 cm³ de tétrahydrofuranne anhydre. On introduit en 1 heure en maintenant la température à 45-50°C, 34 g de 4-pyrrolidinyl bromobenzène dans 140 cm³ de tétrahydrofuranne anhydre. La réaction a été amorcée par addition de dibromoéthane. On obtient ainsi une solution 1 M de magnésien attendu.

Condensation sur l'époxyde.

On mélange sous gaz inerte 8 g de 3,3-[1,2-éthane diyl bis (oxy)]5α-10α-époxy 17α-(prop-1-ynyl) estr-9 (11) èn-17β-ol, 160 cm³ de tétrahydrofuranne anhydre et 216 mg de chlorure cuivreux. On refroidit à 0 +5°C et introduit en 1 heure 30 minutes 86,4 cm³ de la solution de magnésien préparée ci-dessus. On agite pendant 1 heure, verse dans une solution aqueuse saturée de chlorure d'ammonium, extrait à l'oxyde de diéthyle, lave la phase organique avec une solution aqueuse saturée de chlorure d'ammonium puis avec une solution aqueuse saturée de chlorure de sodium, sèche et évapore le solvant. On purifie le résidu par chromatographie sur silice en éluant au mélange chlorure de méthylène-acétone (95:5) à 1 pour mille de triéthylamine. On obtient ainsi 8,3 g de produit attendu que l'on recristallise dans un mélange chlorure de méthylène-éther isopropylique. F = 185°C.
/α/$_D$ = -67°±1,5° (c = 1% chloroforme).

Stade B: 17β-hydroxy 11β-(4-pyrrolidinyl-phényl) 17α-(prop-1-ynyl) estra 4,9-dièn-3-one.

On dissout 6,4 g de produit obtenu au stade A dans 128 cm³ de méthanol puis ajoute 13 cm³ d'acide chlorhydrique 2N. On agite à 20°C pendant 1 heure, puis ajoute 256 cm³ d'oxyde de diéthyle et 256 cm³ d'une solution aqueuse 0,25 M de bicarbonate de sodium. On décante, extrait à l'oxyde de diéthyle, lave la phase organique avec une solution aqueuse 0,25 M de bicarbonate de sodium, puis avec une solution aqueuse saturée de chlorure de sodium, sèche et évapore le solvant. On chromatographie le résidu sur silice en éluant au mélange éther de pétrole-acétate d'éthyle (1:1) et obtient 5,25 g de produit attendu que l'on recristallise dans un mélange chlorure de méthylène-oxyde de diisopropyle. F = 190°C.
/α/$_D$ = +120°±2,5° (c = 1,2% chloroforme).

Exemple 25:

17β-hydroxy 11β-(4-diméthylaminophényl) 17α-éthényl estra 4,9-dièn-3-one.

Stade A: 3,3-[1,2-éthane diyl bis (oxy)] 11β-(4-diméthylaminophényl) 17α-éthényl estr-9-èn-5α-17β-diol.

On mélange 3 g de produit obtenu au stade B de l'exemple 17 avec 60 cm³ de pyridine anhydre et ajoute 0,6 g de palladium à 5% sur carbonate de calcium. On fait passer un courant d'hydrogène dans le mélange à température ambiante pendant 1 heure. On essore le catalyseur, évapore le filtrat à sec, reprend le résidu au toluène et évapore de nouveau à sec. On obtient ainsi 2,94 g de produit attendu, utilisé tel quel pour la suite de la synthèse. F = 181°C. Le produit peut être recristallisé dans un mélange chlorure de méthylène-oxyde de diisopropyle. F = 182°C.
/α/$_D$ = -6,5°±2° (c = 0,7% chloroforme).

Stade B: 17β hydroxy 11β-(4-diméthylamino-phényl) 17α-éthényl estra 4,9-dièn-3-one.

On mélange sous gaz inerte 2,94 g du produit obtenu au stade A avec 60 cm³ de méthanol puis ajoute 6,2 cm³ d'acide chlorhydrique 2N. On agite la solution à 20°C pendant 1 heure, ajoute 120 cm³ d'éther et 120 cm³ d'une solution aqueuse à 0,25 M de bicarbonate de sodium, maintient sous agitation pendant 10 minutes, décante et extrait à l'éther. On lave la phase organique avec une solution aqueuse 0,25 M de bicarbonate de sodium puis avec une solution aqueuse saturée de chlorure de sodium. On sèche et évapore le solvant. On obtient 2,65 g de produit que l'on chromatographie sur silice en éluant au mélange benzène-acétate d'éthyle (7:3) puis que l'on cristallise dans un mélange oxyde de diisopropyle-chlorure de méthylène. On obtient finalement 1,51 g de produit attendu. F = 150°C.
/α/$_D$ = +243°±3° (c = 0,8% chloroforme).

Exemple 26:

17β-hydroxy 11β-(4-diéthylaminophényl) 17α-(prop-1-ynyl) estra 4,9-dièn-3-one.

Stade A: 3,3-[1,2-éthane diyl bis (oxy)] 11β-

(4-diéthylaminophényl) 17α-(prop-1-ynyl) estr-9-èn 5α-17β-diol.

Formation du magnésien.

On mélange sous gaz inerte 3,9 g de magnésium dans 10 $cm^3$ de tétrahydrofuranne. On ajoute goutte à goutte 34,2 g de 4-(N,N-diéthylamino) bromobenzène dans 110 $cm^3$ de tétrahydrofuranne en maintenant la température à environ 35°C. On obtient une solution 1M du magnésien attendu.

Condensation.

On dissout 7,4 g de 3,3-[1,2 éthane diyl bis (oxy)] 5α-10α-époxy 17α-(prop-1-ynyl) estr-9 (11) ène 17β-ol dans 150 $cm^3$ de tétrahydrofuranne anhydre. On ajoute 0,25 g de chlorure cuivreux. On agite à 0 +5°C sous gaz inerte et ajoute lentement 80 $cm^3$ de la solution de magnésien préparée ci-dessus. On maintient pendant 17 heures sous agitation à 20°C, verse dans une solution aqueuse de chlorure d'ammonium, extrait à l'éther, lave la phase organique avec une solution aqueuse de bicarbonate de sodium, sèche et évapore le solvant. On empâte le résidu à l'éther de pétrole puis le traite au charbon actif dans l'éther et le recristallise dans l'éther isopropylique. On obtient ainsi 4 g de produit attendu.

$/\alpha/_D = -61° \pm 2{,}5°$ (c = 0,7% chloroforme).

Stade B: 17β-hydroxy 11β-(4-diéthylamino-phényl) 17α-(prop-1-ynyl) estra 4,9-dièn-3-one.

A une solution de 3,12 g de produit obtenu au stade A dans 45 $cm^3$ de méthanol, on ajoute 8 $cm^3$ d'acide chlorhydrique 2N et agite à 20°C sous gaz inerte pendant 45 minutes. On verse dans l'eau, neutralise par addition d'hydroxyde de sodium 2N, extrait au chlorure de méthylène, sèche et évapore le solvant. On chromatographie le résidu sur silice en éluant au mélange benzène-acétate d'éthyle (1:1) et obtient 1,34 g de produit attendu.

$/\alpha/_D = +144{,}5° \pm 3°$ (c = 0,8% chloroforme).

Analyse: $C_{31}H_{39}NO_2$ (457,63)

| | | | |
|---|---|---|---|
| Calculé: | C% 81,36 | H% 8,59 | N% 3,06 |
| Trouvé: | C% 81,7 | H% 8,8 | N% 2,09 |

Le 4-(N,N-diéthylamino) bromobenzène utilisé au départ du stade A a été préparé comme suit.

A une solution de 86 g de N,N-diéthylaniline dans 400 $cm^3$ d'acide acétique, on ajoute goutte à goutte 93 g de brome. Après la fin de l'introduction, on verse dans un mélange eau-glace, extrait au chlorure de méthylène, lave la phase organique avec une solution aqueuse de bicarbonate de sodium, la sèche et évapore le solvant. On obtient 125 g de produit attendu. Eb: 0,6 = 97°C.

Exemple 27:

17β-hydroxy 11β-[4-]méthyl (3-méthylbutyl) amino[phényl] 17α-(prop-1-ynyl) estra 4,9-dièn-3-one.

Stade A: 3,3-[1,2-éthane diyl bis (oxy)] 11β-[4-] méthyl(3-méthylbutyl) amino [phényl]-17α-(prop-1-ynyl) estr-9-èn 5α-17β-diol.

Préparation du magnésien.

On mélange sous gaz inerte 4,12 g de magnésium et 10 $cm^3$ de tétrahydrofuranne. On introduit quelques $cm^3$ de N-méthyl N-(3-méthylbutyl) 4-bromo benzènamine en solution dans le tétrahydrofuranne et amorce la réaction par addition de 0,2 $cm^3$ de 1,2-dibromoéthane. On ajoute ensuite en 40 minutes le reste de la solution de N-méthyl N-(3-méthylbutyl) 4-bromo benzènamine dans le tétrahydrofuranne anhydre (32,6 g dans 90 $cm^3$). On laisse ensuite revenir à température ambiante puis maintient sous agitation pendant 1 heure. On obtient ainsi une solution 0,9 M du magnésien attendu.

Condensation.

On mélange 8 g de 3,3-[1,2-éthane diyl bis (oxy)] 5α-10α-époxy 17α-(prop-1-ynyl) estr-9 (11) èn 17β-ol avec 90 $cm^3$ de tétrahydrofuranne anhydre et 3,77 g de chlorure cuivreux. On agite pendant 20 minutes à +5°C sous gaz inerte, puis ajoute 100 $cm^3$ de la solution de magnésien préparée ci-dessus. On verse ensuite le mélange dans une solution aqueuse de chlorure d'ammonium, extrait à l'éther additionné de triéthylamine puis au chlorure de méthylène additionné de triéthylamine. On lave les phases organiques réunies par une solution aqueuse saturée de chlorure de sodium, sèche et évapore à sec. On obtient 31,2 g de produit attendu, utilisé tel quel pour le stade suivant. On peut purufier le produit par chromatographie sur silice en éluant au mélange chlorure de méthylène-acétone-triéthylamine (96,5:4,5:0,5).

$/\alpha/_D = -59{,}5° \pm 2{,}5°$ (c = 0,7% chloroforme).

Stade B: 17β-hydroxy 11β-[4-]méthyl (3-méthylbutyl) amino[phényl] 17α-(prop-1-ynyl) estra 4,9-dièn-3-one.

On dissout 26 g de produit obtenu au stade A dans 200 $cm^3$ de méthanol puis ajoute 52 $cm^3$ d'acide chlorhydrique 2N. Après 1 heure sous agitation, on verse le mélange dans une solution aqueuse de bicarbonate de sodium, extrait à l'éther, puis au chlorure de méthylène, lave les phases organiques réunies avec une solution aqueuse saturée de chlorure de sodium, sèche et évapore le solvant. On purifie le produit par chromatographie sur silice en éluant au mélange toluène-acétate d'éthyle (92:8) et obtient 3,23 g de produit attendu.

$/\alpha/_D = +125° \pm 3{,}5°$ (c = 0,6% chloroforme).

Analyse: $C_{33}H_{43}NO_2$ (485,71)

| | | | |
|---|---|---|---|
| Calculé: | C% 81,6 | H% 8,92 | N% 2,88 |
| Trouvé: | C% 81,4 | H% 9,0 | N% 2,7 |

L'amine utilisée au départ du stade A a été préparée comme suit.

Stade A: N-méthyl N-(3-méthylbutyl) aniline.

On mélange 86 g de N-méthyl aniline, 500 $cm^3$ de benzène anhydre et 81 g de triéthylamine anhydre. On ajoute goutte à goutte 121 g de bromure d'isoamyle, porte au reflux pendant 100 heures. On filtre le mélange, lave le filtrat à l'eau, sèche et évapore le solvant. On distille le résidu et obtient 90 g de produit attendu. Eb: 18 = 132°C.

Stade B: N-méthyl N-(3-méthylbutyl) 4-bromo aniline.

On mélange 64 g de produit obtenu au stade a avec 300 $cm^3$ d'acide acétique, puis ajoute goutte à goutte en 1 heure à environ 15°C, 58 g de brome en solution dans 60 $cm^3$ d'acide acétique. On porte la température à 80°C, agite pendant 8 heures, verse ensuite dans l'eau glacée, extrait au chlorure de méthylène, lave la phase organique avec une solution de bicarbonate de sodium puis à l'eau, sèche et évapore le solvant. On distille le résidu et obtient 70 g de produit attendu. Eb: 0,5 = 119°C.

Exemple 28:

17β-hydroxy 11β-[4-(N,N-diméthylaminoéthylthio) phényl] 17α-(prop-1-ynyl) estra 4,9-dièn-3-one.

Stade A: 3,3-[1,2-éthane diyl bis (oxy)] 11β-[4-(N,N-diméthylaminoéthylthio) phényl] 17α-(prop-1-ynyl) estr-9-èn 5α-17β-diol.

Préparation du magnésien.

On mélange sous gaz inerte 2 g de magnésium et 15 $cm^3$ de tétrahydrofuranne anhydre. On introduit ensuite en 45 minutes en laissant la température s'élever jusqu'à 56°C une solution de 20 g de 4-(N,N-diméthylaminoéthylthio) 1-bromo benzène dans 40 $cm^3$ de tétrahydrofuranne anhydre. On a amorcé la réaction par addition de 1,2-dibromoéthane. On laisse ensuite revenir à 20°C puis maintient sous agitation pendant 45 minutes sous gaz inerte. On obtient ainsi une solution 1,05 M du magnésien attendu.

Condensation.

On refroidit à -20°C sous gaz inerte 38 $cm^3$ de la solution de magnésien obtenue ci-dessus. On y ajoute 1,730 g de chlorure cuivreux, maintient sous agitation pendant 20 minutes puis ajoute 5 g de 3,3-[1,2-éthane diyl bis (oxy)] 5α-10α-époxy 17α-(prop-1-ynyl) estr-9(11)èn 17β-ol dans 50 $cm^3$ de tétrahydrofuranne anhydre. On maintient à 20°C sous gaz inerte pendant 2 heures 45 minutes. On verse le mélange dans 600 $cm^3$ d'eau glacée, contenant 60 g de chlorure d'ammonium. On maintient sous agitation pendant 45 minutes, décante, extrait la phase aqueuse par de l'oxyde de diéthyle additionné de triéthylamine, lave les phases organiques réunies par une solution aqueuse saturée de chlorure de sodium, sèche et évapore le solvant. On chromatographie le résidu sur alumine en éluant au mélange chlorure de méthylène-acétone (95:5) et obtient 10,3 g de produit attendu.

Spectre IR.

Absorption à 3600 $cm^{-1}$ (OH), 2240 $cm^{-1}$ (C≡C) 1705 et 1670 $cm^{-1}$ (CO et CO conjugué) 1615 et 1490 $cm^{-1}$ (bandes aromatiques).

Stade B: 17β-hydroxy 11β-[4-(N,N-diméthylaminoéthylthio) phényl] 17α-(prop-1-ynyl) estra 4,9-dièn-3-one.

On mélange sous gaz inerte 10,3 g de produit obtenu au stade A avec 72 $cm^3$ de méthanol puis ajoute 20,6 $cm^3$ d'acide chlorhydrique 2N. On maintient sous agitation à 20°C pendant 1 heure 15 minutes, neutralise par addition d'une solution aqueuse saturée de bicarbonate de sodium, ajoute 200 $cm^3$ d'oxyde de diéthyle, décante, extrait à l'oxyde de diéthyle, lave les phases organiques réunies par une solution aqueuse saturée de chlorure de sodium, sèche et concentre à sec. On chromatographie le résidu sur silice en éluant au mélange chlorure de méthylène-méthanol (9:1) et obtient 3 g de produit attendu que l'on cristallise par empâtage dans l'oxyde de diisopropyle. F = 145°C.

$/\alpha/_D = +125° \pm 2°$ (c = 1% chloroforme).

L'amine utilisée au départ du stade A a été préparée comme suit.

On dissout 20 g de soude en pastilles dans 500 $cm^3$ d'éthanol. On dissout par ailleurs 23,5 g de chlorhydrate de chloroéthyldiméthylamine dans 75 $cm^3$ d'éthanol, puis ajoute 160 $cm^3$ de la solution de soude préparée ci-dessus. On dissout par ailleurs 30 g de parabromothiophénol dans 100 $cm^3$ d'éthanol puis ajoute 160 $cm^3$ de la solution de soude préparée ci-dessus. On ajoute ensuite en 2 minutes à 20°C, la solution d'amine preparée ci-dessus. On porte au reflux pendant 3 heures, évapore le solvant, ajoute de l'eau, extrait au chlorure de méthylène, lave la phase organique avec une solution aqueuse 0,1 N de soude puis à l'eau, sèche et évapore le solvant. On distille le résidu et obtient 35,5 g de produit attendu. Eb: 0,1 = 110°C.

Exemple 29:

11β-(4-diméthylaminophényl) 17β-hydroxy 21-(triméthylsilyl) (17α) 19-norpregna 4,9-dièn-20-yn-3-one.

Stade A: 11β-(4-diméthylaminophényl) 3,3-[1,2-éthane diyl bis (oxy)] 21-(triméthylsilyl) (17α) 19-norpregn-9-èn-20-yn 5α-17β-diol.

On mélange sous gaz inerte 13 $cm^3$ d'une solution 1,6 M de bromure d'éthyl magnésium dans le tétrahydrofuranne, avec 13 $cm^3$ de tétrahydrofuranne anhydre. On agite pendant 5 minutes à 0 +5°C puis ajoute goutte à goutte 3,4 $cm^3$ de triméthylsilyl acétylène. On laisse remonter la température à 20°C et poursuit l'agitation pendant 20 minutes, puis introduit goutte à goutte une solution de 1,12 g du produit obtenu au stade A de l'exemple 7 dans 10 $cm^3$ de tétrahydrofuranne anhydre. On maintient pendant 16 heures à température ambiante sous agitation, verse dans une solution aqueuse de chlorure d'ammonium, agite pendant 10 minutes à température ambiante, extrait au chlorure de méthylène, lave la phase organique avec une solution aqueuse saturée de chlorure de sodium, sèche et évapore le solvant. On chromatographie le résidu sur silice en éluant au mélange éther de pétrole-acétate d'éthyle (6:4) et obtient 680 mg de produit attendu.

$/\alpha/_D$ = −76,5°±3° (c = 0,5% chloroforme).

Stade B: 11β-(4-diméthylaminophényl) 17β-hydroxy 21-(triméthylsilyl) (17α) 19-norpregna 4,9-dièn-20-yn-3-one.

On mélange 562 mg du produit obtenu au stade A avec 15 $cm^3$ de méthanol et 1 $cm^3$ d'acide chlorhydrique 2N. On maintient sous agitation à température ambiante pendant 40 minutes, verse dans une solution aqueuse de bicarbonate de sodium, extrait à l'éther, lave la phase organique avec une solution aqueuse saturée de chlorure de sodium, sèche et évapore le solvant. On chromatographie le résidu sur silice en éluant au mélange éther de pétrole–acétate d'éthyle (6:4) et obtient 364 mg de produit attendu.
$/\alpha/_D$ = +97,5°±3° (c = 0,35% chloroforme).

Analyse: $C_{31}H_{41}NO_2Si$ (487,76)

| | | | |
|---|---|---|---|
| Calculé: | C% 76,33 | H% 8,47 | N% 2,87 |
| Trouvé: | C% 76,4 | H% 8,7 | N% 2,8 |

Exemple 30:

N-oxyde de 17β-hydroxy 11β-[4-(N,N-diméthylaminométhyl) phényl] 17α-(prop-1-ynyl) estra 4,9-dièn-3-one.

On dissout 1,4 g de produit obtenu à l'exemple 23 dans 28 $cm^3$ de chlorure de méthylène puis introduit en 15 minutes à 0 +5°C une solution de 0,64 g d'acide métachloroperbenzoïque dans 12,8 $cm^3$ de chlorure de méthylène. On agite pendant 1 heure à 0 +5°C puis verse dans une solution aqueuse 0,2 N de thiosulfate de sodium, décante, extrait au chlorure de méthylène, lave par une solution aqueuse de bicarbonate de sodium, sèche et évapore à sec. On chromatographie le résidu sur silice en éluant au mélange chlorure de méthylène–méthanol (8:2) et obtient 1,28 g de produit attendu que l'on dissout dans un mélange chlorure de méthylène-oxyde de diisopropyle. On essore les cristaux formés, les sèche et obtient 1,075 g de produit attendu. F = 215°C.
$/\alpha/_D$ = +74,5°±2,5° (c = 0,7% chloroforme).

Exemple 31:

Hémifumarate du 17β-hydroxy 11β-[4-(N,N-diméthylaminométhyl) phényl] 17α-(prop-1-ynyl) estra 4,9-dièn-3-one.

On mélange 1,44 g du produit obtenu à l'exemple 23 dans 2,88 $cm^3$ d'éthanol puis ajoute un mélange de 0,378 g d'acide fumarique dans 4,54 $cm^3$ d'éthanol. On agite la suspension pendant 30 minutes à 60°C, laisse la température revenir à 20°C, et maintient sous agitation. On évapore le solvant, reprend le résidu à l'éther, essore, sèche et obtient 1,70 g de produit attendu. F = 160°C. $/\alpha/_D$ = +70,5°±2,5° (c = 0,8% chloroforme).

Exemple 32:

17β-hydroxy 11β-[4-(N,N-dipropylamino) phényl] 17α-(prop-1-ynyl) estra 4,9-dièn-3-one.

Stade A: 3,3-[1,2-éthane diyl bis (oxy)] 11β-[4-(N,N-dipropylamino) phényl] 17α-(prop-1-ynyl) estr-9-èn 5α-17β-diol.

Préparation du magnésien.

On mélange sous gaz inerte 5 g de magnésium avec 15 $cm^3$ de tétrahydrofuranne anhydre. On ajoute goutte à goutte une solution de 52 g de 4-bromo N,N-dipropylaniline dans 110 $cm^3$ de tétrahydrofuranne en maintenant la température à 40°C. On obtient ainsi une solution 1,1 M de magnésien attendu.

Condensation.

On mélange sous gaz inerte une solution de 5,5 g de 3,3-[1,2-éthane diyl bis (oxy)] 5α-10α-époxy 17α-(prop-1-ynyl) estr-9(11)-èn 17β-ol obtenu au stade A de l'exemple 7 avec 200 mg de chlorure cuivreux. On agite à 0 +5°C, puis ajoute en 15 minutes 50 $cm^3$ de la solution de magnésien obtenue ci-dessus. On agite ensuite pendant 1 heure à 20°C, verse dans une solution aqueuse saturée de chlorure d'ammonium, extrait à l'éther, sèche la phase organique et évapore le solvant. On chromatographie le résidu sur silice en éluant au mélange toluène–acétate d'éthyle (7:3) et obtient 6,3 g de produit attendu.
$/\alpha/_D$ = −56°±2° (c = 0,8% chloroforme).

Analyse: $C_{35}H_{49}NO_4$ (547,75)

| | | | |
|---|---|---|---|
| Calculé: | C% 76,74 | H% 9,02 | N% 2,56 |
| Trouvé: | C% 76,6 | H% 89,2 | N% 2,5 |

Stade B: 17β-hydroxy 11β-[4-(N,N-dipropylamino) phényl] 17α-(prop-1-ynyl) estra 4,9-dièn-3-one.

A une solution de 5,83 g de produit obtenu au stade A dans 80 $cm^3$ de méthanol on ajoute 10 $cm^3$ d'acide chlorhydrique 2N et agite à 20°C pendant 50 minutes. On neutralise par addition de soude N, évapore le solvant sous pression réduite et reprend le résidu au chlorure de méthylène. On lave la phase organique à l'eau, sèche et évapore le solvant. On chromatographie le résidu sur silice en éluant au mélange toluène-acétate d'éthyle (75-25) et obtient 3,81 g de produit attendu.

Spectre IR (chloroforme)

Absorption à 3600 $cm^{-1}$ (OH), 1654 $cm^{-1}$ (C=O), 1610-1595-1558 et 1517 $cm^{-1}$ (Δ4,9+bandes aromatiques), 2240 $cm^{-1}$ (C≡C).

Les produits ci-après constituent des exemples d'autres produits pouvant être obtenus par le procédé de l'invention:

– la 11β-[4-(N-éthyl N-méthylamino) phényl] 17β-hydroxy 17α-(prop-1-ynyl) estra 4,9-dièn-3-one (F = 174°C; $\alpha_D$ = +149°±2,5° (c = 1% $CHCL_3$)
– la 17β-hydroxy 11β-[N-méthyl 2,3-dihydro 1H-indol-5-yl]17α-(prop-1-ynyl) estra 4,9-dièn-3-one (F = 176°C; $\alpha_D$ = +133°±3° - c = 0,8% $CHCl_3$);
– le 11β-(4-diméthylaminophényl) 3-hydroxyimino 17α-)prop-1-ynyl) estra 4,9-dièn-17β-ol (isomère Z) (F = 260°C; $\alpha_D$ = 141°±3,5° - c = 0,8% $CHCl_3$);
– le 11β-(4-diméthylaminophényl) 3-hydroxyimino 17α-(prop-1-ynyl) estra 4,9-dièn-17β-ol (isomère E) (F = 220°C; $\alpha_D$ = +164°±3,5° - c = 0,8% $CHCl_3$);

– le N-oxyde de 17β-hydroxy 11β-(4-pyrrolidylphényl) 17α-(prop-1-ynyl) estra 4,9-dièn-3-one (F = 220°C; $\alpha_D$ = +88°±2,5° - c = 0,75% $CHCl_3$);

– la 17β-hydroxy 11β-[4-N-méthyl N-(1-méthyléthyl) aminophényl]17α-(prop-1-ynyl) estra 4,9-dièn-3-one ($\alpha_D$ = +140°±3,5° - c = 0,5% $CHCl_3$;

– le N-oxyde de 11β-[4-(N,N-diméthylamino-éthyloxy) phényl] 17β-hydroxy 17α-(prop-1-ynyl)estra 4,9-dièn-3-one ($\alpha_D$ = +60,5° - c = 1,2% $CHCl_3$);

– le N-oxyde de 17β-hydroxy 11β-[(N-méthyl) 2,3-dihydro 1H-indol-5-yl/]17α-(prop-1-ynyl) estra 4,9-dièn-3-one ($\alpha_D$ = +103°±2,5° - c = 0,8% $CHCl_3$);

– la 17β-hydroxy 11β-[4-(N-méthyl N-triméthylsilylméthyl) aminophényl] 17α-(prop-1-ynyl) estra 4,9-dièn-3-one;

– la 17β-hydroxy 11β-[4-(N-méthyl N-diméthylaminoéthyl) aminophényl] 17α-(prop-1-ynyl) estra 4,9-dièn-3-one;

– la 17β-hydroxy 11β-[4-(N-méthyl pipérazin-1-yl) phényl] 17α-(prop-1-ynyl) estra 4,9-dièn-3-one;

– la 17-hydroxyimino 11β-(4-diméthylaminophényl) estra 4,9-dièn-3-one. /α/$_D$ = +207,5° ±3,5° (c = 1% $CHCl_3$);

– le 3(E)-hydroxyimino 17-hydroxyimino 11β-(4-diméthylaminophényl) estra 4,9-dièn-3-one ($\alpha_D$ = +195°±3° - c = 1% $CHCl_3$);

– le 3(Z)-hydroxyimino 17-hydroxyimino 11β-(4-diméthylaminophényl) estra 4,9-dièn-3-one ($\alpha_D$ = +163°±2,5° - c = 0,6% $CHCl_3$).

## Revendications

1. Les composés de formule (II):

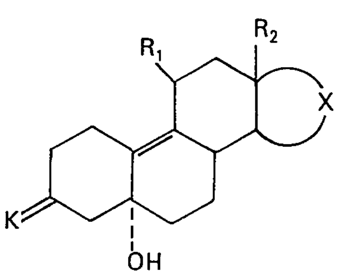

(II)

dans laquelle K représente un groupement cétonique bloqué sous forme de cétal, de thiocétal, d'oxime ou de méthyloxime, $R_1$ représente un radical organique renfermant de 1 à 18 atomes de carbone, contenant au moins un atome d'azote, de phosphore ou de silicium, l'atome immédiatement adjacent au carbone en 11 étant un atome de carbone, $R_2$ représente un radical hydrocarboné renfermant de 1 à 8 atomes de carbone, X représente le reste d'un cycle pentagonal ou hexagonal éventuellement substitué et éventuellement porteur d'insaturation.

2. Les produits de formule (II) telle que définie à la revendication 1, répondant à la formule (V):

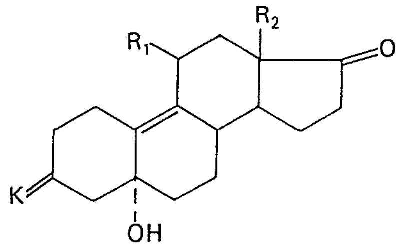

(V)

dans laquelle K, $R_1$ et $R_2$ conservent la même signification que dans la revendication 1, ou à la formule (II'):

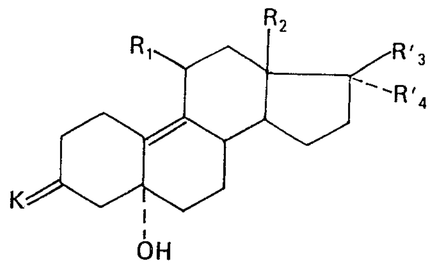

(II')

dans laquelle $R_1$, $R_2$ et K sont définis comme dans la revendication 1, $R'_3$ représente un radical hydroxy ou un radical $OR_e$, dans lequel $R_e$ représente le reste $alc_4$ d'un groupement éther ou $COalc_5$ d'un groupement ester $alc_4$ et $alc_5$ étant définis comme à la revendication 1 et $R'_4$ représente un atome d'hydrogène ou un radical alkényle ou alkynyle comportant de 2 à 8 atomes de carbone.

3. Les produits de formule (II) tels que définis à la revendication 1 ou 2, pour lesquels $R_2$ représente un radical méthyle.

4. Les produits de formule (II) tels que définis à la revendication 1 ou 3, pour lesquels X représente le reste d'un cycle de formule:

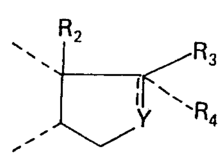

dans lequel $R_2$ conserve la même signification que dans la revendication 1, 2 ou 3, le trait pointillé en 16–17 symbolise la présence éventuelle d'une double liaison, Y représente un radical

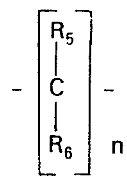

dans lequel n représente le nombre 1 ou 2, $R_5$ représente un atome d'hydrogène, un radical alkyle renfermant de 1 à 8 atomes de carbone, un radical alkényle ou alkynyle renfermant de 2 à 8 atomes de carbone, un radical aryle renfermant de 6 à 14 atomes de carbone ou un radical aralkyle renfermant de 7 à 15 atomes de carbone, $R_6$,

identique ou différent de $R_5$, peut prendre l'une des valeurs indiquées pour $R_5$ et peut également représenter un radical hydroxyle, $R_3$ et $R_4$ identiques ou différents, représentent soit un atome d'hydrogène, soit un radical OH, $Oalc_4$, O-CO-$alc_5$, $alc_4$ et $alc_5$ représentant un radical alkyle renfermant de 1 à 8 atomes de carbone ou aralkyle renfermant de 7 à 15 atomes de carbone, soit un radical alkényle ou alkynyle renfermant de 2 à 8 atomes de carbone, soit un radical

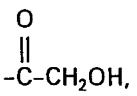

soit un radical $-COCH_2OCOalc_6$, dans lequel $alc_6$ représente un radical alkyle renfermant de 1 à 8 atomes de carbone éventuellement substitué ou un radical aralkyle renfermant de 7 à 15 atomes de carbone, soit un radical $CO-CO_2H$, ou $CO-CO_2-alc_7$ dans lequel $alc_7$ représente un radical alkyle renfermant de 1 à 8 atomes de carbone, soit un radical

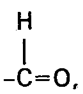 soit un radical 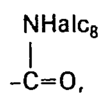

dans lequel $alc_8$ représente un radical alkyle renfermant de 1 à 8 atomes de carbone ou un radical aralkyle renfermant de 7 à 15 atomes de carbone, soit un radical -C≡N, soit $R_3$ et $R_4$ forment ensemble un radical

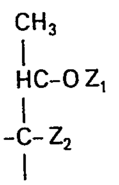

dans lequel $Z_1$ représente un atome d'hydrogène, un radical alkyle ou un radical acyle renfermant de 1 à 8 atomes de carbone et $Z_2$ un radical alkyle renfermant de 1 à 8 atomes de carbone.

5. Les produits de formule (II) tels que définis à la revendication 4, pour lesquels le cycle D ne porte pas d'insaturation éthylénique, $R_5$ et $R_6$ représentent un atome d'hydrogène et n est égal à 1.

6. Les produits de formule (II) tels que définis à l'une quelconque des revendications 1 à 5, pour lesquels $R_1$ représente un radical hydrocarboné renfermant de 1 à 18 atomes de carbone et contenant au moins un atome d'azote.

7. Les produits de formule (II) tels que définis à la revendication 6, pour lesquels $R_1$ représente un radical alkyle primaire, secondaire ou tertiaire, renfermant de 1 à 8 atomes de carbone, comportant un ou plusieurs hétéroatomes choisis dans le groupe constitué par l'oxygène, l'azote et le soufre, dont au moins un atome d'azote ou substitué par un hétérocycle comportant au moins un atome d'azote.

8. Les produits de formule (II) tels que définis à la revendication 6, pour lesquels $R_1$ représente un radical hétérocyclique comportant au moins un atome d'azote, éventuellement substitué par un radical alkyle renfermant de 1 à 8 atomes de carbone.

9. Les produits de formule (II) tels que définis à la revendication 6, pour lesquels $R_1$ représente un radical aryle ou aralkyle portant une fonction amine

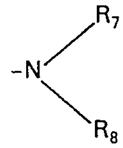 ,

dans laquelle $R_7$ et $R_8$ représentent un radical alkyle renfermant de 1 à 8 atomes de carbone ou un radical alkyle primaire, secondaire ou tertiaire renfermant de 1 à 8 atomes de carbone, comportant un ou plusieurs hétéroatomes choisi dans le groupe constitué par l'oxygène, l'azote et le soufre, dont au moins un atome d'azote, ou substitué par un hétérocycle comportant au moins un atome d'azote.

10. Les produits de formule (II) tels que définis à l'une quelconque des revendications 1 à 9, pour lesquels $R_1$ représente un radical 2,3 ou 4-pyridyle,

un radical 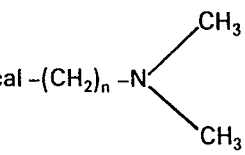 (⩾ 3) ,

un radical 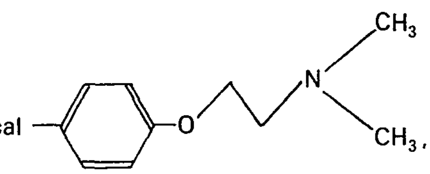

un radical 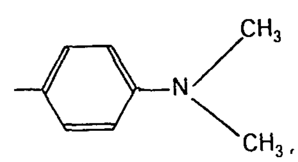

un radical 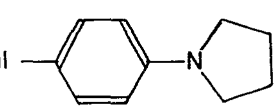 ou

un radical 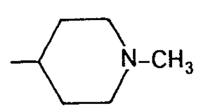

11. Les produits de formule (II), tels que définis à l'une quelconque des revendications 1 à 10, dans lesquels $R_1$ comporte un atome d'azote oxydé.

12. L'un quelconque des produits de formule (II) dont les noms suivent:

- le 11β-[4-(triméthylsilyl) phényl] 3,3-[1,2-éthane diyl bis (oxy)]17α-(prop-1-ynyl) estr-9-èn 5α, 17β-diol,
- le 11β-(4-pyridyl) 3,3-[1,2-éthane diyl bis (oxy)] 17α-(prop-1-ynyl) estr-9-èn 5α, 17β-diol,
- le 11β-[3-(N,N-diméthylamino) propyl] 3,3-[1,2-éthane diyl bis (oxy)] 17α-(prop-1-ynyl) estr-9-èn 5α, 17β-diol,
- le 3,3-[éthane diyl bis (oxy)] 11β-[4-(N,N-diméthylaminoéthyloxy) phényl] 17α-(prop-1-ynyl) estr-9-èn 5α, 17β-diol,
- le 21-chloro 3,3-[1,2-éthane diyl bis (oxy)] 11β-(4-diméthylaminophényl) (17α) 19-norpregn-9-èn-20-yn 5α, 17β-diol,
- le 11β-(4-diméthylaminophényl) 3,3-[1,2-éthane diyl bis (oxy)]17α-(prop-2-ynyl) estr-9-èn 5α, 17β-diol.

13. Procédé de préparation des produits de formule (II) tels que définis à l'une quelconque des revendications 1 à 12, caractérisé en ce que l'on soumet un produit de formule générale (III):

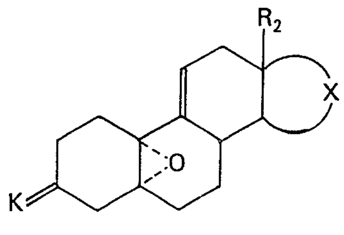

(III)

à l'action d'un composé choisi dans le groupe constitué par les composés de formule $(R_1)_2$ Cu Li, de formule $R_1$Mg Hal et de formule $R_1$Li, dans laquelle $R_1$ conserve la même signification que dans la revendication 1 et Hal représente un atome d'halogène, le cas échéant, en présence d'halogénure cuivreux, pour obtenir le composé de formule (II) correspondant.

14. Procédé selon la revendication 13, caractérisé en ce que le produit de départ, répondant à la formule (II'),

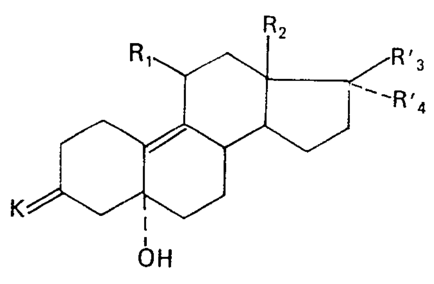

(II')

dans lequel $R_1$, $R_2$ et K sont définis comme dans la revendication 1, $R'_3$ représente un radical hydroxy ou un radical $OR_e$, dans lequel $R_e$ représente le reste $alc_4$ d'un groupement éther ou $COalc_5$ d'un groupement ester, $alc_4$ et $alc_5$ étant définis comme à la revendication 1 et $R'_4$ représente un atome d'hydrogène ou un radical alkényle ou alkynyle comportant de 2 à 8 atomes de carbone est préparé en soumettant un produit de formule (IV):

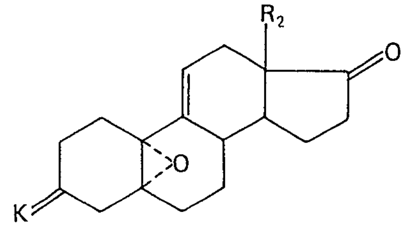

(IV)

à l'action d'un produit choisi dans le groupe constitué par les composés de formule $(R_1)_2$ Cu Li, de formule $R_1$Mg Hal et de formule $R_1$Li, dans laquelle $R_1$ et Hal sont définis comme à la revendication 13, le cas échéant, en présence d'halogénure cuivreux, pour obtenir le produit de formule (V):

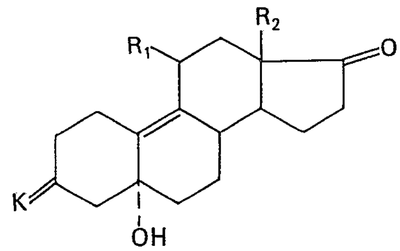

(V)

que l'on soumet soit à l'action d'un agent de réduction, pour obtenir le produit 17-hydroxy correspondant, soit à l'action d'un magnésien approprié, pour obtenir le produit 17β-hydroxy 17α-substitué correspondant, soit à l'action d'un dérivé organométallique tel qu'un lithien ou un dérivé de potassium, pour obtenir le produit 17β-hydroxy 17α-substitué correspondant, soit à l'action d'un agent de cyanuration, pour obtenir le produit 17β-cyano 17α-hydroxy correspondant, dont on protège la fonction hydroxy, puis à l'action d'un dérivé organométallique tel que décrit précédemment, pour obtenir le produit 17β-hydroxy 17α-substitué correspondant, et que, le cas échéant, l'on soumet l'un ou l'autre des produits 17-hydroxy obtenus ci-dessus, à l'action d'un agent d'estérification ou d'éthérification, et que, le cas échéant, l'on soumet l'un ou l'autre des produits 17-substitués obtenus ci-dessus, dans lesquels le substituant en 17 comporte une triple liaison, à l'action d'un agent de réduction, pour obtenir l'éthylénique correspondant.

15. La 3,3-[1,2-éthane diyl bis (oxy)] 5α, 10α-époxy 17α-[1-propynyl] estr-9(11)-èn 17β-ol.

16. Les composés de formule (V):

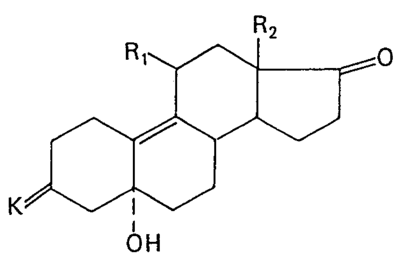

(V)

dans laquelle K et $R_2$ conservent la même signification que dans la revendication 1 et $R_1$ représente un radical:

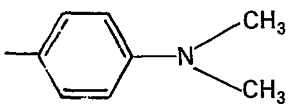

**Revendications pour l'Autriche**

1. Procédé de préparation des produits de formule (II):

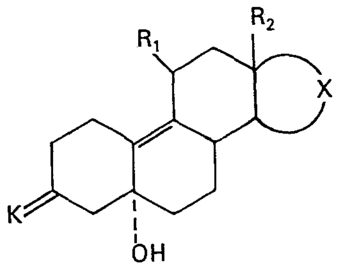

(II)

dans laquelle K représente un groupement cétonique bloqué sous forme de cétal, de thiocétal, d'oxime ou de méthyloxime, $R_1$ représente un radical organique renfermant de 1 à 18 atomes de carbone, contenant au moins un atome d'azote, de phosphore ou de silicium, l'atome immédiatement adjacent au carbone en 11 étant un atome de carbone, $R_2$ représente un radical hydrocarboné renfermant de 1 à 8 atomes de carbone, X représente le reste d'un cycle pentagonal ou hexagonal éventuellement substitué et éventuellement porteur d'insaturation, caractérisé en ce que l'on soumet un produit de formule (III):

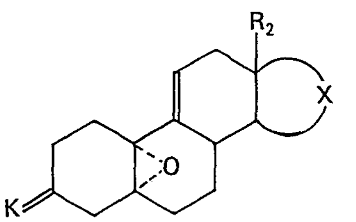

(III)

à l'action d'un produit choisi dans le groupe constitué par les produits de formule $(R_1)_2$ Cu Li, de formule $R_1$Mg Hal et de formule $R_1$Li, dans laquelle $R_1$ conserve la même signification que dans la revendication 1 et Hal représente un atome d'halogène, le cas échéant, en présence d'halogénure cuivreux, pour obtenir le produit de formule (II) correspondant.

2. Procédé selon la revendication 1, caractérisé en ce que le produit de formule (II), répondant à la formule (II'),

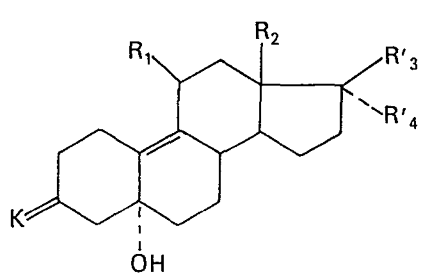

(II')

dans lequel $R_1$, $R_2$ et K sont définis comme dans la revendication 1, $R''_3$ représente un radical hydroxy ou un radical $OR_e$, dans lequel $R_e$ représente le reste $alc_4$ d'un groupement éther ou $COalc_5$ d'un groupement ester, $alc_4$ et $alc_5$ étant définis comme à la revendication 1 et $R'_4$ représente un atome d'hydrogène ou un radical alkényle ou alkynyle comportant de 2 à 8 atomes de carbone est préparé en soumettant un produit de formule (IV):

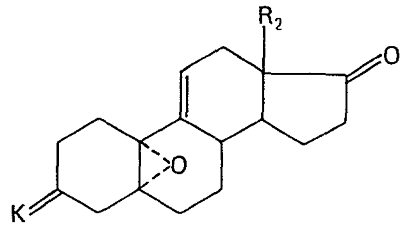

(IV)

à l'action d'un produit choisi dans le groupe constitué par les produits de formule $(R_1)_2$ Cu Li, de formule $R_1$Mg Hal et de formule $R_1$Li, dans laquelle $R_1$ et Hal sont définis comme à la revendication 1, le cas échéant, en présence d'halogénure cuivreux, pour obtenir le produit de formule (V):

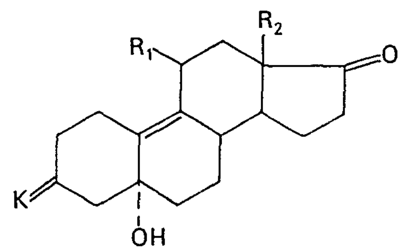

(V)

que l'on soumet soit à l'action d'un agent de réduction, pour obtenir le produit 17-hydroxy correspondant, soit à l'action d'un magnésien approprié, pour obtenir le produit 17β-hydroxy 17α-substitué correspondant, soit à l'action d'un dérivé organométallique tel qu'un lithien ou un dérivé de potassium, pour obtenir le composé 17β-hydroxy 17α-substitué correspondant, soit à l'action d'un agent de cyanuration, pour obtenir le produit 17β-cyano 17α-hydroxy correspondant, dont on protège la fonction hydroxy, puis à l'action d'un dérivé organométallique tel que décrit précédemment, pour obtenir le produit 17β-hydroxy 17α-substitué correspondant, et que, le cas échéant, l'on soumet l'un ou l'autre des produits 17-hydroxy obtenus ci-dessus, à l'action d'un agent d'estérification ou d'éthérification, et que, le cas échéant, l'on soumet l'un ou l'autre des produits 17-substitués obtenus ci-dessus, dans lesquels le substituant en 17 comporte une triple liaison, à l'action d'un agent de réduction, pour obtenir l'éthylénique correspondant.

3. Procédé selon la revendication 1, caractérisé en ce que l'on utilise au départ un produit de formule (III), dans laquelle $R_2$ représente un radical méthyle.

4. Procédé selon la revendication 1 ou 3, caractérisé en ce que l'on utilise au départ un produit

de formule (III), dans laquelle X représente le reste d'un cycle de formule

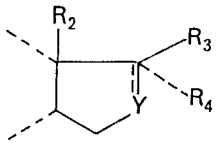

dans lequel $R_2$ conserve la même signification que dans la revendication 1 ou 3, le trait pointillé en 16–17 symbolise la présence éventuelle d'une double liaison, Y représente un radical

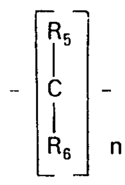

dans lequel n représente le nombre 1 ou 2, $R_5$ représente un atome d'hydrogène, un radical alkyle renfermant de 1 à 8 atomes de carbone, un radical alkényle ou alkynyle renfermant de 2 à 8 atomes de carbone un radical aryle renfermant de 6 à 14 atomes de carbone, ou un radical aralkyle renfermant de 7 à 15 atomes de carbone, $R_6$, identique ou différent de $R_5$, peut prendre l'une des valeurs indiquées pour $R_5$ et peut également représenter un radical hydroxyle, $R_3$ et $R_4$ identiques ou différents, représentent soit un atome d'hydrogène, soit un radical OH, $Oalc_4$, O-CO-$alc_5$, $alc_4$ et $alc_5$ représentant un radical alkyle renfermant de 1 à 8 atomes de carbone ou aralkyle renfermant de 7 à 15 atomes de carbone, soit un radical alkényle ou alkynyle renfermant de 2 à 8 atomes de carbone, soit un radical

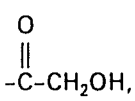

soit un radical $-COCH_2OCOalc_6$, dans lequel $alc_6$ représente un radical alkyle renfermant de 1 à 8 atomes de carbone éventuellement substitué ou un radical aralkyle renfermant de 7 à 15 atomes de carbone, soit un radical $CO-CO_2H$, ou $CO-CO_2-alc_7$ dans lequel $alc_7$ représente un radical alkyle renfermant de 1 à 8 atomes de carbone, soit un radical

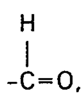, soit un radical 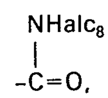,

dans lequel $alc_8$ représente un radical alkyle renfermant de 1 à 8 atomes de carbone ou un radical aralkyle renfermant de 7 à 15 atomes de carbone, soit un radical $-C{\equiv}N$, soit $R_3$ et $R_4$ forment ensemble un radical

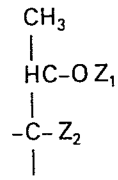

dans lequel $Z_1$ représente un atome d'hydrogène, un radical alkyle ou un radical acyle renfermant de 1 à 8 atomes de carbone et $Z_2$ un radical alkyle renfermant de 1 à 8 atomes de carbone.

5. Procédé selon l'une quelconque des revendications 1, 3 ou 4, caractérisé en ce que l'on utilise au départ un composé de formule (III), dans laquelle le cycle D ne porte pas d'insaturation éthylénique, $R_5$ et $R_6$ représentent un atome d'hydrogène et n est égal à 1.

6. Procédé selon l'une quelconque des revendications 1 et 3 à 5, caractérisé en ce que l'on utilise au départ un composé de formule (III), dans laquelle $R_1$ représente un radical hydrocarboné renfermant de 1 à 18 atomes de carbone et contenant au moins un atome d'azote.

7. Procédé selon la revendication 6, caractérisé en ce que l'on utilise au départ un composé de formule (III), dans laquelle $R_1$ est choisi dans le groupe constitué par un radical alkyle primaire, secondaire ou tertiaire, renfermant de 1 à 8 atomes de carbone, comportant un ou plusieurs hétéroatomes choisis dans le groupe constitué par l'oxygène, l'azote et le soufre, dont au moins un atome d'azote, ou substitué par un hétérocycle comportant au moins un atome d'azote, un radical hétérocyclique comportant au moins un atome d'azote, éventuellement substitué par un radical alkyle renfermant de 1 à 8 atomes de carbone, un radical aryle ou aralkyle portant une fonction amine

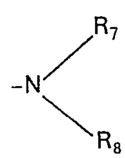,

dans laquelle $R_7$ et $R_8$ représentent un radical alkyle renfermant de 1 à 8 atomes de carbone ou un radical alkyle primaire, secondaire ou tertiaire renfermant de 1 à 8 atomes de carbone, comportant un ou plusieurs hétéroatomes choisi dans le groupe constitué par l'oxygène, l'azote et le soufre, dont au moins un atome d'azote, ou substitué par un hétérocycle comportant au moins un atome d'azote, un radical 2, 3 ou 4-pyridine, un radical

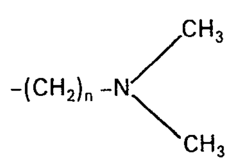 $(\geq 3)$,

un radical 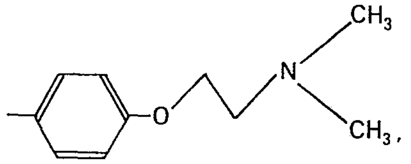

un radical 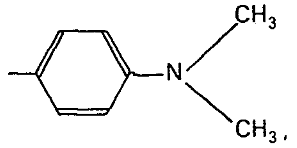

un radical 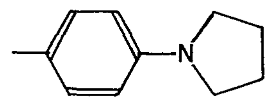 ou

un radical 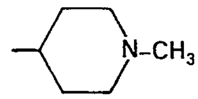

8. Procédé selon l'une quelconque des revendications 1 à 7, caractérisé en ce que l'on prépare l'un quelconque des composés de formule (II) dont les noms suivent:

- le 11β-[4-(triméthylsilyl) phényl] 3,3-[1,2-éthane diyl bis (oxy)]17α-(prop-1-ynyl) estr-9-èn 5α, 17β-diol,
- le 11β-(4-pyridyl) 3,3-[1,2-éthane diyl bis (oxy)] 17α-(prop-1-ynyl) estr-9-èn 5α, 17β-diol,
- le 11β-[3-(N,N-diméthylamino) propyl] 3,3-[1,2-éthane diyl bis (oxy)] 17α-(prop-1-ynyl) estr-9-èn 5α, 17β-diol,
- le 11β-(4-diméthylaminophényl) 3,3-[1,2-éthane diyl bis (oxy)] 17α-(prop-1-ynyl) estr-9-èn 5α, 17β-diol,
- le 3,3-[éthane diyl bis (oxy)] 11β [4-(N,N-diméthylaminoéthyloxy) phényl] 17α-(prop-1-ynyl) estr-9-èn 5α, 17β-diol,
- le 21-chloro 3,3-[1,2-éthane diyl bis (oxy)] 11β-(4-diméthylaminophényl) (17α) 19-norpregn-9-èn-20-yn 5α, 17β-diol,
- le 11β-(4-diméthylaminophényl) 3,3-[1,2-éthane diyl bis (oxy)]17α-(prop-2-ynyl) estr-9-èn 5α, 17β-diol.

9. Procédé de préparation d'un produit de formule (V):

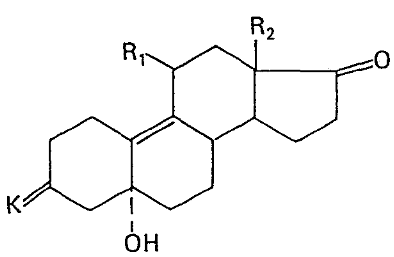

(V)

dans laquelle K et $R_2$ sont définis comme dans la revendication 1 et $R_1$ représente un radical

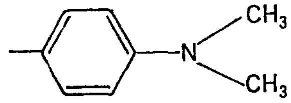

caractérisé en ce que l'on soumet un produit de formule (IV):

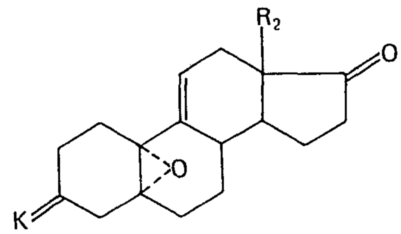

(IV)

à l'action d'un produit de formule

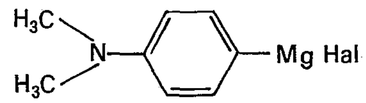

dans laquelle Hal est défini comme à la revendication 1.

**Patentansprüche für die Vertragsstaaten BE, CH, DE, FR, GB, IT, LI, LU, NL, SE**

1. Verbindungen der Formel (II)

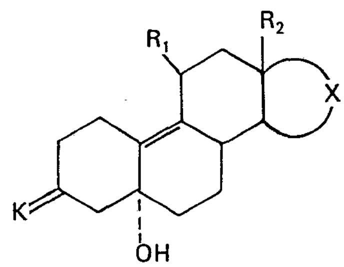

(II)

worin K eine in Form des Ketals, Thioketals, Oxims oder Methyloxims blockierte Ketogruppe bedeutet, $R_1$ einen organischen Rest mit 1 bis 18 Kohlenstoffatomen bedeutet, der zumindest ein Stickstoff-, Phosphor- oder Siliciumatom enthält, wobei das dem Kohlenstoffatom in 11-Stellung unmittelbar benachbarte Atom ein Kohlenstoffatom ist, $R_2$ einen Kohlenwasserstoffrest mit 1 bis 8 Kohlenstoffatomen bedeutet, X den Rest eines pentagonalen oder hexagonalen Rings darstellt, der gegebenenfalls substituiert ist und gegebenenfalls eine Unsättigung enthält.

2. Produkte der Formel (II) gemäss Anspruch 1 der Formel (V)

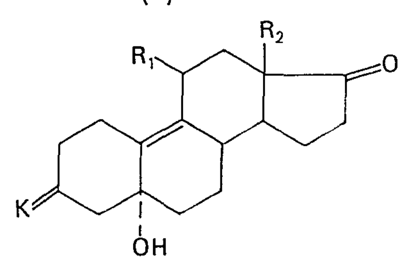

(V)

worin K, $R_1$ und $R_2$ die in Anspruch 1 angegebene Bedeutung besitzen, oder der Formel (II')

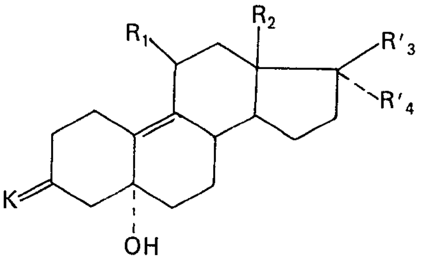

(II')

worin $R_1$, $R_2$ und K wie in Anspruch 1 definiert sind, $R'_3$ eine Hydroxygruppe oder einen Rest $OR_e$ bedeutet, wobei $R_e$ den Rest $alc_4$ einer Ethergruppe oder $COalc_5$ einer Estergruppe darstellt, wobei $alc_4$ und $alc_5$ wie in Anspruch 1 definiert sind, und $R'_4$ ein Wasserstoffatom oder einen Alkenyl- oder Alkinylrest mit 2 bis 8 Kohlenstoffatomen bedeutet.

3. Produkte der Formel (II) gemäss Anspruch 1 oder 2, worin $R_2$ eine Methylgruppe bedeutet.

4. Produkte der Formel (II) gemäss Anspruch 1 oder 3, worin X den Rest eines Rings der Formel darstellt:

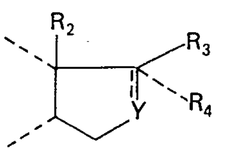

worin $R_2$ die in Anspruch 1, 2 oder 3 angegebene Bedeutung besitzt, die gestrichelte Linie in 16-17-Stellung die etwaige Anwesenheit einer Doppelbindung symbolisiert, Y einen Rest

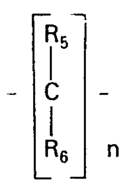

darstellt, worin n die Zahl 1 oder 2 bedeutet, $R_5$ ein Wasserstoffatom, einen Alkylrest mit 1 bis 8 Kohlenstoffatomen, einen Alkenyl- oder Alkinylrest mit 2 bis 8 Kohlenstoffatomen, einen Arylrest mit 6 bis 14 Kohlenstoffatomen oder einen Aralkylrest mit 7 bis 15 Kohlenstoffatomen bedeutet, $R_6$, das mit $R_5$ identisch oder von diesem verschieden ist, eine der für $R_5$ angegebenen Bedeutungen besitzen kann und auch einen Hydroxylrest bedeuten kann, $R_3$ und $R_4$, die gleich oder verschieden sind, entweder ein Wasserstoffatom oder einen Rest OH, $Oalc_4$, $O-CO-alc_5$, wobei $alc_4$ und $alc_5$ einen Alkylrest mit 1 bis 8 Kohlenstoffatomen oder einen Aralkylrest mit 7 bis 15 Kohlenstoffatomen bedeuten, oder einen Alkenyl- oder Alkinylrest mit 2 bis 8 Kohlenstoffatomen oder einen Rest

O
||
$-C-CH_2OH$,

oder einen Rest $-COCH_2OCOalc_6$, worin $alc_6$ einen Alkylrest mit 1 bis 8 Kohlenstoffatomen, der gegebenenfalls substituiert ist, oder einen Aralkylrest mit 7 bis 15 Kohlenstoffatomen bedeutet, oder einen Rest der Formel $CO-CO_2H$ oder $CO-CO_2alc_7$, worin $alc_7$ einen Alkylrest mit 1 bis 8 Kohlenstoffatomen bedeutet, oder einen Rest

H
|
$-C=O$, oder einen Rest

$NHalc_8$
|
$-C=O$,

worin $alc_8$ einen Alkylrest mit 1 bis 8 Kohlenstoffatomen oder einen Aralkylrest mit 7 bis 15 Kohlenstoffatomen bedeutet, oder einen Rest $-C\equiv N$ bedeuten, oder $R_3$ und $R_4$ gemeinsam einen Rest

$CH_3$
|
$HC-OZ_1$
|
$-C-Z_2$
|

bilden, worin $Z_1$ ein Wasserstoffatom, einen Alkylrest oder einen Acylrest mit 1 bis 8 Kohlenstoffatomen darstellt und $Z_2$ einen Alkylrest mit 1 bis 8 Kohlenstoffatomen bedeutet.

5. Produkte der Formel (II) gemäss Anspruch 1, worin der Ring D keine ethylenische Unsättigung enthält, $R_5$ und $R_6$ ein Wasserstoffatom darstellen und n 1 ist.

6. Produkte der Formel (II) gemäss einem der Ansprüche 1 bis 5, worin $R_1$ einen Kohlenwasserstoffrest mit 1 bis 18 Kohlenstoffatomen, der zumindest ein Stickstoffatom enthält, bedeutet.

7. Produkte der Formel (II) gemäss Anspruch 6, worin $R_1$ eine primäre, sekundäre oder tertiäre Alkylgruppe mit 1 bis 8 Kohlenstoffatomen darstellt, die eine oder mehrere Heteroatome, ausgewählt unter Sauerstoff, Stickstoff und Schwefel, enthält, von denen zumindest eines ein Stickstoffatom ist, oder die durch einen Heterocyclus mit zumindest einem Stickstoffatom substituiert ist.

8. Produkte der Formel (II) gemäss Anspruch 6, worin $R_1$ einen heterocyclischen Rest mit zumindest einem Stickstoffatom bedeutet, der gegebenenfalls durch eine Alkylgruppe mit 1 bis 8 Kohlenstoffatomen substituiert ist.

9. Produkte der Formel (II) gemäss Anspruch 6, worin $R_1$ eine Aryl- oder Aralkylgruppe mit einer Aminfunktion

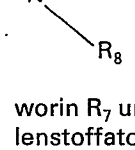,

worin $R_7$ und $R_8$ eine Alkylgruppe mit 1 bis 8 Kohlenstoffatomen darstellen, oder eine primäre, sekundäre oder tertiäre Alkylgruppe mit 1 bis 8 Kohlenstoffatomen, mit einem oder mehreren Heteroatomen, ausgewählt unter Sauerstoff, Stickstoff und Schwefel, bedeutet, von denen zumindest eines ein Stickstoffatom ist oder die durch einen Heterocyclus mit zumindest einem Stickstoffatom substituiert ist.

10. Produkte der Formel (II) gemäss einem der Ansprüche 1 bis 9, worin $R_1$ einen 2-, 3- oder 4-Pyridylrest,

einen Rest 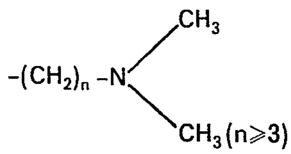 ,

einen Rest: 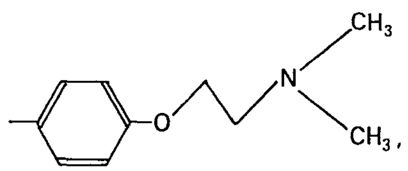

einen Rest 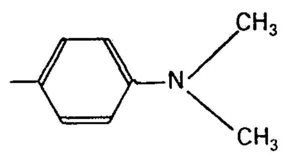

einen Rest 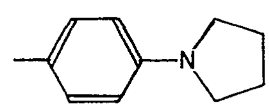 oder

einen Rest 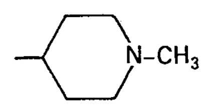

bedeutet.

11. Die Produkte der Formel (II) gemäss einem der Ansprüche 1 bis 10, worin $R_1$ ein oxidiertes Stickstoffatom enthält.

12. Eines der Produkte der Formel (II) mit den folgenden Bezeichnungen:

11β-[4-(Trimethylsilyl)-phenyl]-3,3-[1,2-ethandiyl-bis-(oxy)]-17α-(prop-1-inyl)-östr-9-en-5α,17β-diol,

11β-(4-Pyridyl)-3,3-[1,2-ethandiyl-bis-(oxy)]-17α-(prop-1-inyl)-östr-9-en-5α,17β-diol,

11β-[3-(N,N-Dimethylamino)-propyl]-3,3-[1,2-ethan-diyl-bis-(oxy)]-17α-(prop-1-inyl)-östr-9-en-5α,17β-diol,

11β-(4-Dimethylaminophenyl)-3,3-[1,2-ethan-diyl-bis-(oxy)]-17α-(prop-1-inyl)-östr-9-en-5α,17β-diol,

3,3-[Ethandiyl-bis-(oxy)]-11β-[4-(N,N-dimethyl-aminoethyloxy)-phenyl]-17α-(prop-1-inyl)-östr-9-en-5α,17β-diol,

21-Chlor-3,3-[1,2-ethan-diyl-bis-(oxy)]-11β-(4-dimethylaminophenyl)-(17α)19-nor-pregn-9-en-20-in-5α,17β-diol,

11β-(4-Dimethylaminophenyl)-3,3-[1,2-ethan-diyl-bis-(oxy)]-17α-(prop-2-inyl)-östr-9-en-5α,17β-diol.

13. Verfahren zur Herstellung der Produkte der Formel (II) gemäss einem der Ansprüche 1 bis 12, dadurch gekennzeichnet, dass man ein Produkt der allgemeinen Formel (III)

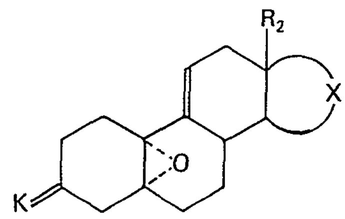

(III)

der Einwirkung einer Verbindung, ausgewählt unter den Verbindungen der Formel $(R_1)_2$ Cu Li, der Formel $R_1$Mg Hal und der Formel $R_1$Li, worin $R_1$ die in Anspruch 1 angegebene Bedeutung besitzt und Hal ein Halogenatom bedeutet, gegebenenfalls in Anwesenheit von Kupfer(I)-halogenid unterzieht, um die entsprechende Verbindung der Formel (II) zu erhalten.

14. Verfahren gemäss Anspruch 13, dadurch gekennzeichnet, dass das Ausgangsprodukt, das der Formel (II′)

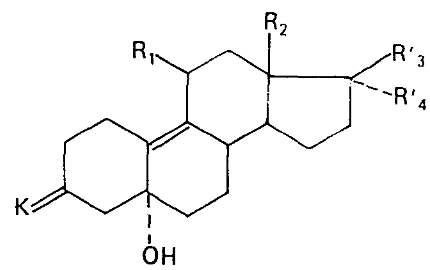

(II′)

entspricht, worin $R_1$, $R_2$ und K wie in Anspruch 1 definiert sind, $R'_3$ eine Hydroxygruppe oder einen Rest $OR_e$ bedeutet, wobei $R_e$ den Rest $alc_4$ einer Ethergruppe oder $COalc_5$ einer Estergruppe darstellt, wobei $alc_4$ und $alc_5$ wie in Anspruch 1 definiert sind, und $R'_4$ ein Wasserstoffatom oder eine Alkenyl- oder Alkinylgruppe mit 2 bis 8 Kohlenstoffatomen bedeutet, hergestellt wird, indem man ein Produkt der Formel (IV)

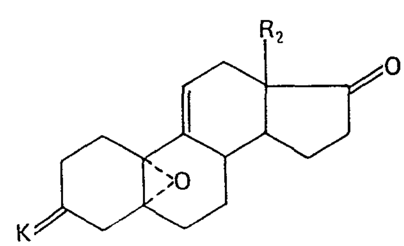

(IV)

der Einwirkung eines Produkts, ausgewählt unter den Verbindungen der Formel $(R_1)_2$ Cu Li, der Formel $R_1$Mg Hal und der Formel $R_1$Li, worin $R_1$ und Hal wie in Anspruch 13 definiert sind, gegebenenfalls in Anwesenheit von Kupfer(I)-halogenid unterzieht, um das Produkt der Formel (V)

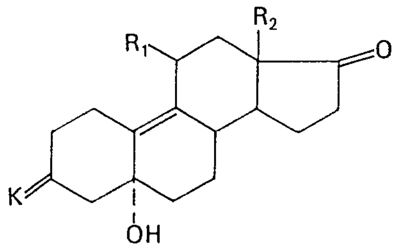

(V)

zu erhalten, das man entweder der Einwirkung eines Reduktionsmittels unterzieht, um das entsprechende 17-Hydroxy-Produkt zu erhalten, oder der Einwirkung einer geeigneten Magnesiumverbindung unterzieht, um das entsprechende 17β-Hydroxy-17α-substituierte Produkt zu erhalten, oder der Einwirkung einer organometallischen Verbindung, wie einer Lithiumverbindung oder eines Kaliumderivats, unterzieht, um das entsprechende 17β-Hydroxy-17α-substituierte Produkt zu erhalten, oder der Einwirkung eines Cyanurierungsmittels unterzieht, um das entsprechende 17β-Cyano-17α-hydroxy-Produkt zu erhalten, von dem man die Hydroxyfunktion schützt, danach der Einwirkung eines organometallischen Derivats, wie vorstehend beschrieben, unterzieht, um das entsprechende 17β-Hydroxy-17α-substituierte Produkt zu erhalten, und gegebenenfalls das eine oder andere der vorstehend erhaltenen 17-Hydroxy-Produkte der Einwirkung eines Veresterungs- oder Veretherungsmittels unterzieht, und gegebenenfalls das eine oder andere der vorstehend erhaltenen 17-substituierten Produkte, worin der Substituent in 17-Stellung eine Dreifachbindung enthält, der Einwirkung eines Reduktionsmittels unterzieht, um die entsprechende ethylenische Verbindung zu erhalten.

15. 3,3-[1,2-Ethan-diyl-bis-(oxy)]-5α,10α-epoxy-17α-(1-propinyl)-östr-9(11)-en-17β-ol.

16. Verbindungen der Formel (V)

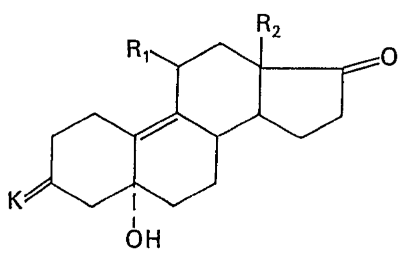

(V)

worin K und $R_2$ die in Anspruch 1 angegebene Bedeutung besitzen und $R_1$ einen Rest

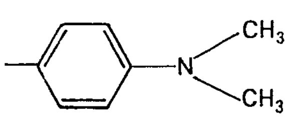

darstellt.

**Patentansprüche für den Vertragsstaat AT**

1. Verfahren zur Herstellung der Produkte der Formel (II)

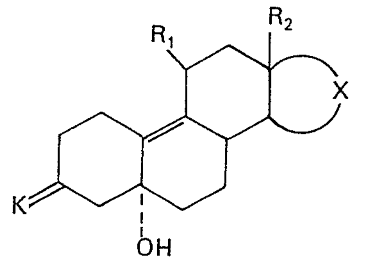

(II)

worin K eine in Form des Ketals, Thioketals, Oxims oder Methyloxims blockierte Ketogruppe bedeutet, $R_1$ einen organischen Rest mit 1 bis 18 Kohlenstoffatomen bedeutet, der zumindest ein Stickstoff-, Phosphor- oder Siliciumatom enthält, wobei das dem Kohlenstoffatom in 11-Stellung unmittelbar benachbarte Atom ein Kohlenstoffatom ist, $R_2$ einen Kohlenwasserstoffrest mit 1 bis 8 Kohlenstoffatomen bedeutet, X den Rest eines pentagonalen oder hexagonalen Rings darstellt, der gegebenenfalls substituiert ist und gegebenenfalls eine Unsättigung enthält, dadurch gekennzeichnet, dass man ein Produkt der Formel (III)

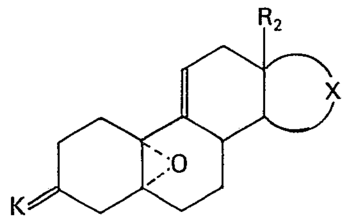

(III)

der Einwirkung eines Produkts, ausgewählt unter den Produkten der Formel $(R_1)_2$ Cu Li, der Formel $R_1$Mg Hal und der Formel $R_1$Li, worin $R_1$ die in Anspruch 1 angegebene Bedeutung besitzt und Hal ein Halogenatom darstellt, gegebenenfalls in Anwesenheit von Kupfer(I)-halogenid unterzieht, um das entsprechende Produkt der Formel (II) zu erhalten.

2. Verfahren gemäss Anspruch 1, dadurch gekennzeichnet, dass das Produkt der Formel (II), das der Formel (II′) entspricht

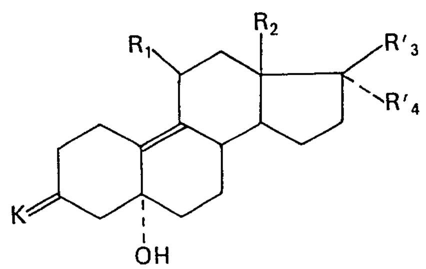

(II′)

worin $R_1$, $R_2$ und K wie in Anspruch 1 definiert sind, $R''_3$ eine Hydroxygruppe oder einen Rest $OR_e$ bedeutet, worin $R_e$ den Rest $alc_4$ einer Ethergruppe oder $COalc_5$ einer Estergruppe darstellt, wobei $alc_4$ und $alc_5$ wie in Anspruch 1 definiert sind, und $R'_4$ ein Wasserstoffatom oder eine Alkenyl- oder Alinylgruppe mit 2 bis 8 Kohlenstoffatomen darstellt, hergestellt wird, indem man ein Produkt der Formel (IV)

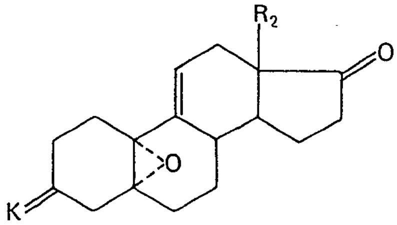

(IV)

der Einwirkung eines Produkts, ausgewählt unter den Produkten der Formel $(R_1)_2$ Cu Li, der Formel $R_1$Mg Hal und der Formel $R_1$Li, worin $R_1$ und Hal wie in Anspruch 1 definiert sind, gegebenenfalls in Anwesenheit eines Kupfer(I)-halogenids unterzieht, um das Produkt der Formel (V)

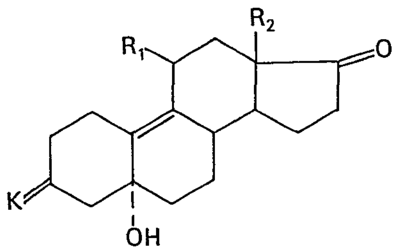

(V)

zu erhalten, das man entweder der Einwirkung eines Reduktionsmittels unterzieht, um das entsprechende 17-Hydroxy-Produkt zu erhalten, oder der Einwirkung einer geeigneten Magnesiumverbindung unterzieht, um das entsprechende 17β-Hydroxy-17α-substituierte Produkt zu erhalten, oder der Einwirkung eines organometallischen Derivats, wie einer Lithiumverbindung oder eines Kaliumderivats, unterzieht, um die entsprechende 17β-Hydroxy-17α-substituierte Verbindung zu erhalten, oder der Einwirkung eines Cyanurierungsmittels unterzieht, um das entsprechende 17β-Cyano-17α-hydroxy-Produkt zu erhalten, von dem man die Hydroxyfunktion schützt, danach der Einwirkung eines organometallischen Derivats, wie vorstehend beschrieben, unterzieht, um das entsprechende 17β-Hydroxy-17α-substituierte Produkt zu erhalten, und gegebenenfalls das eine oder andere der vorstehend erhaltenen 17-Hydroxy-Produkte der Einwirkung eines Veresterungs- oder Veretherungsmittels unterzieht, und gegebenenfalls das eine oder andere der vorstehend erhaltenen 17-substituierten Produkte, worin der Substituent in 17-Stellung eine Dreifachbindung enthält, der Einwirkung eines Reduktionsmittels unterzieht, um das entsprechende ethylenische Produkt zu erhalten.

3. Verfahren gemäss Anspruch 1, dadurch gekennzeichnet, dass man von einem Produkt der Formel (III) ausgeht, worin $R_2$ eine Methylgruppe bedeutet.

4. Verfahren gemäss Anspruch 1 oder 3, dadurch gekennzeichnet, dass man von einem Produkt der Formel (III) ausgeht, worin X den Rest eines Rings der Formel

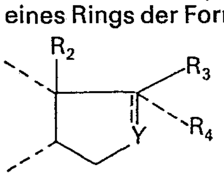

bedeutet, worin $R_2$ die in Anspruch 1 oder 3 angegebene Bedeutung hat, die gestrichelte Linie in 16-17-Stellung die etwaige Anwesenheit einer Doppelbindung symbolisiert, Y einen Rest

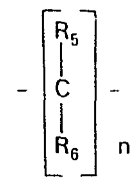

darstellt, worin n die Zahl 1 oder 2 bedeutet, $R_5$ ein Wasserstoffatom, einen Alkylrest mit 1 bis 8 Kohlenstoffatomen, einen Alkenyl- oder Alkinylrest mit 2 bis 8 Kohlenstoffatomen, einen Arylrest mit 6 bis 14 Kohlenstoffatomen oder einen Aralkylrest mit 7 bis 15 Kohlenstoffatomen bedeutet, $R_6$, das mit $R_5$ identisch oder von diesem verschieden ist, eine der für $R_5$ angegebenen Bedeutungen besitzen kann und auch einen Hydroxylrest bedeuten kann, $R_3$ und $R_4$, die gleich oder verschieden sind, entweder ein Wasserstoffatom oder einen Rest OH, $Oalc_4$, $O-CO-alc_5$, wobei $alc_4$ und $alc_5$ einen Alkylrest mit 1 bis 8 Kohlenstoffatomen oder einen Aralkylrest mit 7 bis 15 Kohlenstoffatomen bedeuten, oder einen Alkenyl- oder Alkinylrest mit 2 bis 8 Kohlenstoffatomen oder einen Rest

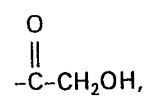

oder einen Rest $-COCH_2OCOalc_6$, worin $alc_6$ einen Alkylrest mit 1 bis 8 Kohlenstoffatomen, der gegebenenfalls substituiert ist, oder einen Aralkylrest mit 7 bis 15 Kohlenstoffatomen bedeutet, oder einen Rest der Formel $CO-CO_2H$ oder $CO-CO_2alc_7$, worin $alc_7$ einen Alkylrest mit 1 bis 8 Kohlenstoffatomen bedeutet, oder einen Rest

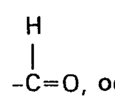 oder einen Rest 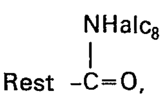

worin $alc_8$ einen Alkylrest mit 1 bis 8 Kohlenstoffatomen oder einen Aralkylrest mit 7 bis 15 Kohlenstoffatomen bedeutet, oder einen Rest $-C{\equiv}N$ bedeuten, oder $R_3$ und $R_4$ gemeinsam einen Rest

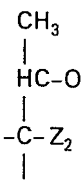

bilden, worin $Z_1$ ein Wasserstoffatom, einen Alkylrest oder einen Acylrest mit 1 bis 8 Kohlenstoffatomen darstellt und $Z_2$ einen Alkylrest mit 1 bis 8 Kohlenstoffatomen bedeutet.

5. Verfahren gemäss einem der Ansprüche 1, 3 oder 4, dadurch gekennzeichnet, dass man von

einer Verbindung der Formel (III) ausgeht, worin der Ring D keine ethylenische Unsättigung enthält, $R_5$ und $R_6$ ein Wasserstoffatom bedeuten und n 1 ist.

6. Verfahren gemäss einem der Ansprüche 1 und 3 bis 5, dadurch gekennzeichnet, dass man von einer Verbindung der Formel (III) ausgeht, worin $R_1$ einen Kohlenwasserstoffrest mit 1 bis 18 Kohlenstoffatomen, der zumindest ein Stickstoffatom enthält, bedeutet.

7. Verfahren gemäss Anspruch 6, dadurch gekennzeichnet, dass man von einer Verbindung der Formel (III) ausgeht, worin $R_1$ ausgewählt ist unter einer primären, sekundären oder tertiären Alkylgruppe mit 1 bis 8 Kohlenstoffatomen, die ein oder mehrere Heteroatome, ausgewählt unter Sauerstoff, Stickstoff und Schwefel, enthält, von denen zumindest eines ein Stickstoffatom ist oder die substituiert ist durch einen Heterocyclus mit zumindest einem Stickstoffatom, einem heterocyclischen Rest mit zumindest einem Stickstoffatom, der gegebenenfalls durch eine Alkylgruppe mit 1 bis 8 Kohlenstoffatomen substituiert ist, einem Aryl- oder Aralkylrest mit einer Aminfunktion

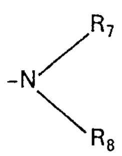 ,

worin $R_7$ und $R_8$ eine Alkylgruppe mit 1 bis 8 Kohlenstoffatomen bedeuten, oder einer primären, sekundären oder tertiären Alkylgruppe mit 1 bis 8 Kohlenstoffatomen mit einem oder mehreren Heteroatomen, ausgewählt unter Sauerstoff, Stickstoff und Schwefel, von denen zumindest eines ein Stickstoffatom ist, oder die durch einen Heterocyclus mit zumindest einem Stickstoffatom substituiert ist, einem 2-, 3- oder 4-Pyridinrest, einem Rest

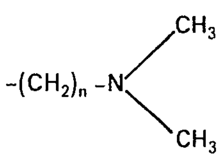 (≥ 3) ,

einen Rest 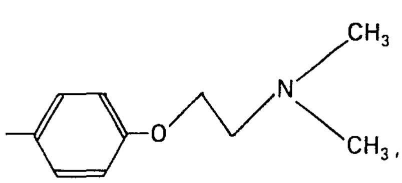,

einen Rest 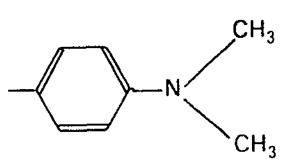,

einen Rest 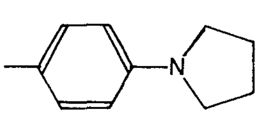 oder

einen Rest 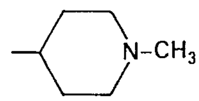

8. Verfahren gemäss einem der Ansprüche 1 bis 7, dadurch gekennzeichnet, dass man eine der Verbindungen der Formel (II) mit den folgenden Bezeichnungen herstellt:

11β-[4-(Trimethylsilyl)-phenyl]-3,3-[1,2-ethandiyl-bis-(oxy)]-17α-(prop-1-inyl)-östr-9-en-5α,17β-diol,

11β-(4-Pyridyl)-3,3-[1,2-ethandiyl-bis-(oxy)]-17α-(prop-1-inyl)-östr-9-en-5α,17β-diol,

11β-[3-(N,N-Dimethylamino)-propyl]-3,3-[1,2-ethan-diyl-bis-(oxy)]-17α-(prop-1-inyl)-östr-9-en-5α,17β-diol,

11β-(4-Dimethylaminophenyl)-3,3-[1,2-ethandiyl-bis-(oxy)]-17α-(prop-1-inyl)-östr-9-en-5α,17β-diol,

3,3-[Ethandiyl-bis-(oxy)]-11β-[4-(N,N-dimethylaminoethyloxy)-phenyl]-17α-(prop-1-inyl)-östr-9-en-5α,17β-diol,

21-Chlor-3,3-[1,2-ethandiyl-bis-(oxy)]-11β-(4-dimethylaminophenyl)-(17α)19-nor-pregn-9-en-20-in-5α,17β-diol,

11β-(4-Dimethylaminophenyl)-3,3-[1,2-ethandiyl-bis-(oxy)]-17α-(prop-1-inyl)-östr-9-en-5α,17β-diol.

9. Verfahren zur Herstellung eines Produkts der Formel (V)

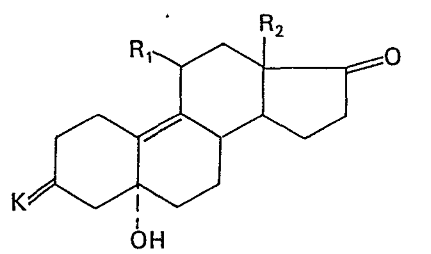

(V)

worin K und $R_2$ wie in Anspruch 1 definiert sind und $R_1$ einen Rest

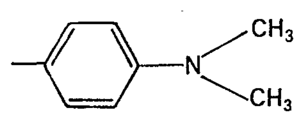

bedeutet, dadurch gekennzeichnet, dass man ein Produkt der Formel (IV)

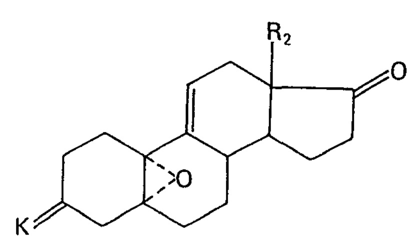

(IV)

der Einwirkung eines Produktes der Formel

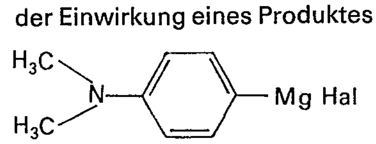

unterzieht, worin Hal wie in Anspruch 1 definiert ist.

**Claims for the Contracting States BE, CH, DE, FR, GB, IT, LI, LU, NL, SE**

1. Compounds with the formula (II):

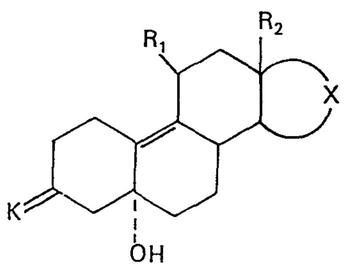

(II)

in which K represents a ketone group blocked in the ketal, thioketal, oxime or methyloxime form, $R_1$ represents an organic radical containing from 1 to 18 carbon atoms, containing at least one nitrogen, phosphorus or silicon atom, the atom immediately adjacent to the carbon at position 11 being a carbon atom, $R_2$ represents a hydrocarbon radical containing from 1 to 8 carbon atoms, X represents the residue of a pentagonal or hexagonal ring, possibly substituted and possibly having an unsaturation.

2. Products with the formula (II) as defined in Claim 1, answering to the formula (V):

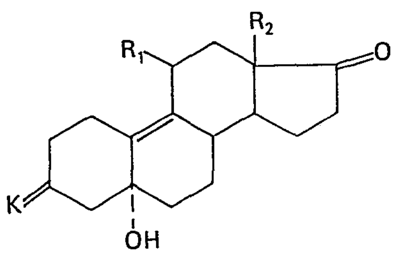

(V)

in which K, $R_1$ and $R_2$ retain the same significance as in Claim 1, or to the formula (II'):

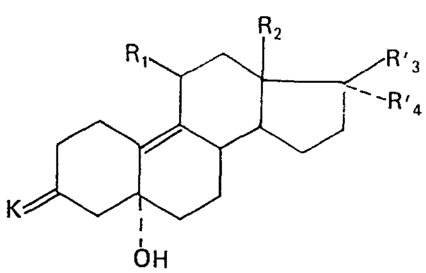

(II')

in which $R_1$, $R_2$ and K are defined as in Claim 1, $R'_3$ represents a hydroxy radical or a radical $OR_e$, in which $R_e$ represents the residue $alk_4$ of an ether group or the residue $COalk_5$ of an ester group, $alk_4$ and $alk_5$ being defined as in Claim 1 and $R'_4$ represents a hydrogen atom or an alkenyl or alkynyl radical containing from 2 to 8 carbon atoms.

3. Products with the formula (II) as defined in Claim 1 or 2, for which $R_2$ represents a methyl radical.

4. Products with the formula (II) as defined in Claim 1 or 3, for which X represents the residue of a ring with the formula:

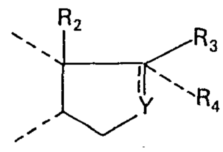

in which $R_2$ retains the same significance as in Claim 1, 2 or 3, the broken line at 16–17 symbolizes the possible presence of a double bond, Y represents a

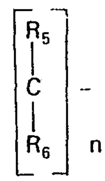

radical in which n represents the numeral 1 or 2, $R_5$ represents a hydrogen atom, an alkyl radical containing from 1 to 8 carbon atoms, an alkenyl or alkynyl radical containing from 2 to 8 carbon atoms, an aryl radical containing from 6 to 14 carbon atoms or an aralkyl radical containing from 7 to 15 carbon atoms, $R_6$ identical to or different from $R_5$, can take one of the values indicated for $R_5$ and can equally represent a hydroxyl radical, $R_3$ and $R_4$, identical or different, represent either a hydrogen atom, or an OH, $Oalk_4$, or $O-CO-alk_5$ radical, $alk_4$ and $alk_5$ representing an alkyl radical containing from 1 to 8 carbon atoms, or an aralkyl radical containing from 7 to 15 carbon atoms, or an alkenyl or alkynyl radical containing from 2 to 8 carbon atoms, or a radical

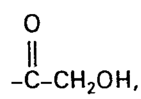

or a radical $-COCH_2OCOalk_6$, in which $alk_6$ represents a possibly substituted alkyl radical containing from 1 to 8 carbon atoms or an aralkyl radical containing from 7 to 15 carbon atoms, or a $CO-CO_2H$ radical, or $CO-CO_2-alk_7$ in which $alk_7$ represents an alkyl radical containing from 1 to 8 carbon atoms, or a radical

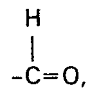 , or a radical 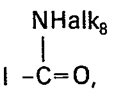

in which $alk_8$ represents an alkyl radical containing from 1 to 8 carbon atoms or an aralkyl radical containing from 7 to 15 carbon atoms, or a radical $-C{\equiv}N$, or $R_3$ and $R_4$ together form a radical

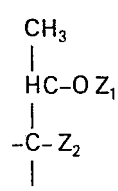

in which $Z_1$ represents a hydrogen atom, an alkyl radical or an acyl radical containing from 1 to 8 carbon atoms and $Z_2$ represents an alkyl radical containing from 1 to 8 carbon atoms.

5. Products with the formula (II) as defined in Claim 4, for which the ring D does not have an ethylene unsaturation, $R_5$ and $R_6$ represent a hydrogen atom and n is 1.

6. Products with the formula (II) as defined in any one of the Claims 1 to 5, for which $R_1$ represents a hydrocarbon radical containing from 1 to 18 carbon atoms and containing at least one nitrogen atom.

7. Products with the formula (II) as defined in Claim 6, for which $R_1$ represents a primary, secondary or tertiary alkyl radical, containing from 1 to 8 carbon atoms, including one or more heteroatoms chosen from the group constituted by oxygen, nitrogen and sulphur, of which at least one is a nitrogen atom or substituted by a heterocycle including at least one nitrogen atom.

8. Products with the formula (II) as defined in Claim 6, for which $R_1$ represents a heterocyclic radical including at least one nitrogen atom, possibly substituted by an alkyl radical containing from 1 to 8 carbon atoms.

9. Products with the formula (II) as defined in Claim 6, for which $R_1$ represents an aryl or an aralkyl radical carrying an amine function

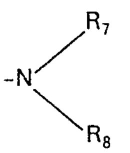 ,

in which $R_7$ and $R_8$ represent an alkyl radical containing from 1 to 8 carbon atoms or a primary, secondary or tertiary alkyl radical containing from 1 to 8 carbon atoms, carrying one or more heteroatoms chosen from the group constituted by oxygen, nitrogen and sulphur, of which at least one is a nitrogen atom, or substituted by a heterocycle carrying at least one nitrogen atom.

10. Products with the formula (II) as defined in any one of the Claims 1 to 9, for which $R_1$ represents a 2-, 3- or 4-pyridyl radical,

a $-(CH_2)_n$ 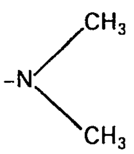 radical, ($n \geqslant 3$),

a radical: 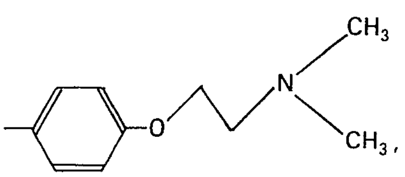

a radical: 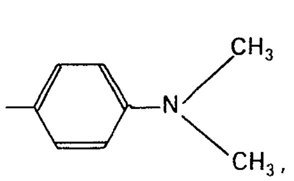

a radical: 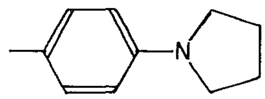 or

a radical: 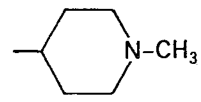

11. Products with the formula (II), as defined in any one of the Claims 1 to 10, in which $R_1$ includes an oxided nitrogen atom.

12. Any one of the products with the formula (II), the names of which follow:

- 11β-[4-(trimethylsilyl)-phenyl]-3,3-[1,2-ethanediyl-bis(oxy)]-17α-(prop-1-ynyl)-estr-9-en-5α,17β-diol,
- 11β-(4-pyridyl)-3,3-[1,2-ethanediyl-bis(oxy)]-17α-(prop-1-ynyl)-estr-9-en-5α,17β-diol,
- 11β-[3-(N,N-dimethylamino)-propyl]-3,3-[1,2-ethanediyl-bis-(oxy)]-17α-(prop-1-ynyl)-estr-9-en-5α,17β-diol,
- 11β-(4-dimethylaminophenyl)-3,3-[1,2-ethanediyl-bis(oxy)]-17α-(prop-1-ynyl)-estr-9-en-5α,17β-diol,
- 3,3-[ethanediyl-bis(oxy)]-11β-[4-(N,N-dimethylaminoethyloxy)-phenyl]-17α-(prop-1-ynyl)-estr-9-en-5α,17β-diol,
- 21-chloro-3,3-[1,2-ethanediyl-bis(oxy)]-11β-(4-dimethylaminophenyl]-(17α)-19-nor-pregn-9-en-20-yn-5α,17β-diol,
- 11β-(4-dimethylaminophenyl)-3,3-[1,2-ethanediyl-bis(oxy)]-17α-(prop-2-ynyl)-estr-9-en-5α,17β-diol.

13. Preparation process for products with the formula (II) as defined in any one of the Claims 1 to 12, characterized in that a product with the general formula (III):

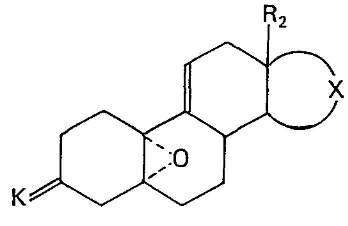

(III)

is submitted to the action of a compound chosen from the group constituted by the compounds with the formula $(R_1)_2$ Cu Li, with the formula $R_1$Mg Hal and with the formula $R_1$Li, in which $R_1$ retains the same significance as in Claim 1 and Hal represents a halogen atom, if the case arises, in the presence of a cuprous halide, so as to obtain the corresponding compound with the formula (II).

14. Process according to Claim 13, characterized in that the starting product, answering to the formula (II′),

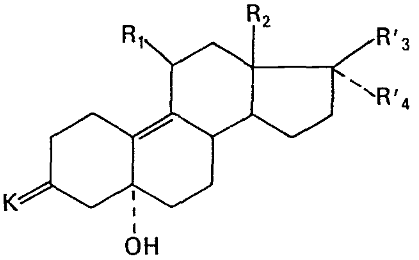

(II')

in which $R_1$, $R_2$ and K are defined as in Claim 1, $R'_3$ represents a hydroxy radical or an $OR_e$ radical, in which $R_e$ represents the residue $alk_4$ of an ether group or the residue $COalk_5$ of an ester group, $alk_4$ and $alk_5$ being defined as in Claim 1 and $R'_4$ represents a hydrogen atom or an alkenyl or alkynyl radical containing from 2 to 8 carbon atoms, is prepared by submitting a product with the formula (IV):

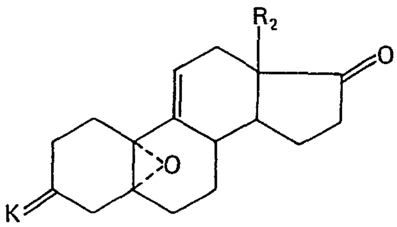

(IV)

to the action of a product chosen from the group constituted by the compounds with the formula $(R_1)_2$ Cu Li, with the formula $R_1$Mg Hal and with the formula $R_1$Li, in which $R_1$ and Hal are defined as in Claim 13, if necessary, in the presence of a cuprous halide, so as to obtain the compound with the formula (V):

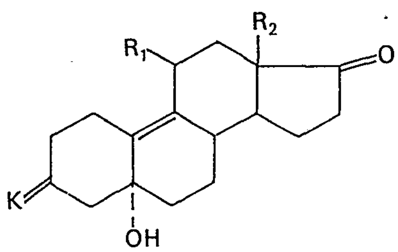

(V)

which is submitted either to the action of a reduction agent, so as to obtain the corresponding 17-hydroxy product, or to the action of an appropriate magnesium compound, so as to obtain the corresponding 17β-hydroxy-17α-substituted product, or to the action of an organo-metallic derivative such as a lithium or potassium derivative, so as to obtain the corresponding 17β-hydroxy-17α-substituted product, or to the action of a cyaniding agent, so as to obtain the corresponding 17β-cyano-17α-hydroxy product, of which the hydroxy function is protected, then to the action of an organo-metallic derivative as previously described, so as to obtain the corresponding 17β-hydroxy-17α-substituted product, and that, if the case arises, one or other of the 17-hydroxy products obtained above, is submitted to an esterification or etherification agent, and that, if the case arises, one or other of the 17-substituted products obtained above, in which the substituent at 17 includes a triple bond, is submitted to the action of a reducing agent, so as to obtain the corresponding ethylene compound.

15. 3,3-[1,2-ethanediyl-bis(oxy)]-5α,10α,-epoxy-17α-[1-propynyl]-estr-9(11)-en-17β-ol.

16. Compounds with the formula (V):

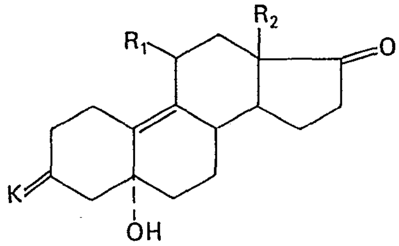

(V)

in which K and $R_2$ retain the same significance as in Claim 1 and $R_1$ represents a radical:

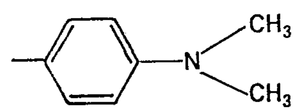

**Claims for the Contracting State AT**

1. Preparation process for products with the formula (II):

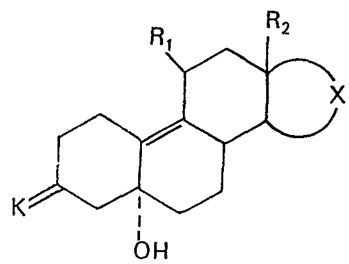

(II)

in which K represents a ketone group blocked in the ketal, thioketal, oxime or methyloxime form, $R_1$ represents an organic radical containing from 1 to 18 carbon atoms, containing at least one nitrogen, phosphorus or silicon atom, the atom immediately adjacent to the carbon at position 11 being a carbon atom, $R_2$ represents a hydrocarbon radical containing from 1 to 8 carbon atoms, X represents the residue of a pentagonal or hexagonal ring, possibly substituted and possibly having an unsaturation, characterized in that a product with the formula (III):

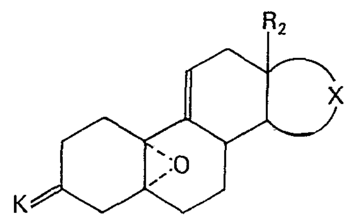

(III)

is submitted to the action of a product chosen from the group constituted by the products with the formula $(R_1)_2$ Cu Li, with the formula $R_1$Mg Hal and with the formula $R_1$Li, in which $R_1$ retains the same significance as in Claim 1 and Hal represents a halogen atom, if the case arises, in the presence of a cuprous halide, so as to obtain the corresponding product with the formula (II).

2. Process according to Claim 1, characterized in that the product with the formula (II), answering to the formula (II′),

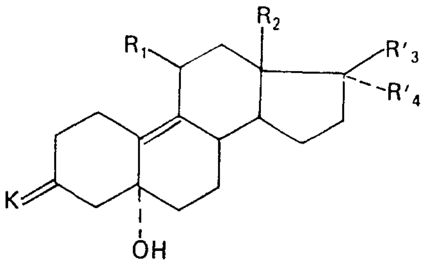

(II′)

in which $R_1$, $R_2$ and K are defined as in Claim 1, $R'_3$ represents a hydroxy radical or an $OR_e$ radical, in which $R_e$ represents the residue $alk_4$ of an ether group or the residue $COalk_5$ of an ester group, $alk_4$ and $alk_5$ being defined as in Claim 1 and $R'_4$ represents a hydrogen atom or an alkenyl or alkynyl radical containing from 2 to 8 carbon atoms, is prepared by submitting a product with the formula (IV):

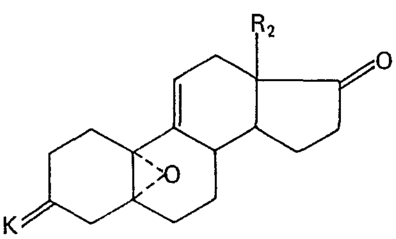

(IV)

to the action of a product chosen from the group constituted by the products with the formula $(R_1)_2$ Cu Li, with the formula $R_1$Mg Hal and with the formula $R_1$Li, in which $R_1$ and Hal are defined as in Claim 1, if necessary, in the presence of a cuprous halide, so as to obtain the product with the formula (V):

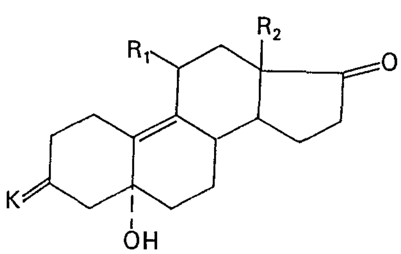

(V)

which is submitted either to the action of a reduction agent, so as to obtain the corresponding 17-hydroxy product, or to the action of an appropriate magnesium compound, so as to obtain the corresponding 17β-hydroxy-17α-substituted product, or to the action of an organo-metallic derivative such as a lithium or potassium derivative, so as to obtain the corresponding 17β-hydroxy-17α-substituted compound, or to the action of a cyaniding agent, so as to obtain the corresponding 17β-cyano-17α-hydroxy product, of which the hydroxy function is protected, then to the action of an organo-metallic derivative as previously described, so as to obtain the corresponding 17β-hydroxy-17α-substituted product, and that, if the case arises, one or other of the 17-hydroxy products obtained above, is submitted to an esterification or etherification agent, and that, if the case arises, one or other of the 17-substituted products obtained above, in which the substituent at 17 includes a triple bond, is submitted to the action of a reducing agent, so as to obtain the corresponding ethylene compound.

3. Process according to Claim 1, characterized in that at the start, a product with the formula (III) is used, in which $R_2$ represents a methyl radical.

4. Process according to Claim 1 or 3, characterized in that at the start, a product with the formula (III) is used, in which X represents the residue of a ring with the formula:

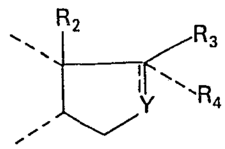

in which $R_2$ retains the same significance as in Claim 1 or 3, the broken line at 16–17 symbolizes the possible presence of a double bond, Y represents a

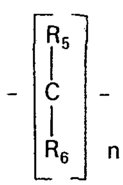

radical in which n represents the numeral 1 or 2, $R_5$ represents a hydrogen atom, an alkyl radical containing from 1 to 8 carbon atoms, an alkenyl or alkynyl radical containing from 2 to 8 carbon atoms, an aryl radical containing from 6 to 14 carbon atoms or an aralkyl radical containing from 7 to 15 carbon atoms, $R_6$ identical to or different from $R_5$, can take one of the values indicated for $R_5$ and can equally represent a hydroxyl radical, $R_3$ and $R_4$, identical or different, represent either a hydrogen atom, or an OH, $Oalk_4$, or O-CO-$alk_5$ radical, $alk_4$ and $alk_5$ representing an alkyl radical containing from 1 to 8 carbon atoms, or an aralkyl radical containing from 7 to 15 carbon atoms, or an alkenyl or alkynyl radical containing from 2 to 8 carbon atoms, or a radical

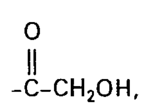

or a radical $-COCH_2OCOalk_6$, in which $alk_6$ represents a possibly substituted alkyl radical containing from 1 to 8 carbon atoms or an aralkyl radical containing from 7 to 15 carbon atoms, or a $CO-CO_2H$ radical, or $CO-CO_2$-alk$_7$ in which alk$_7$ represents an alkyl radical containing from 1 to 8 carbon atoms, or a radical

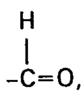 or a radical 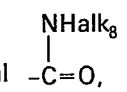

in which alk$_8$ represents an alkyl radical containing from 1 to 8 carbon atoms or an aralkyl radical containing from 7 to 15 carbon atoms, or a radical $-C{\equiv}N$, or $R_3$ and $R_4$ together form a radical

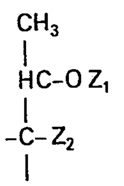

in which $Z_1$ represents a hydrogen atom, an alkyl radical or an acyl radical containing from 1 to 8 carbon atoms and $Z_2$ represents an alkyl radical containing from 1 to 8 carbon atoms.

5. Process according to any one of the Claims 1, 3 or 4, characterized in that at the start, a compound with the formula (III) is used, in which the ring D does not have an ethylene unsaturation, $R_5$ and $R_6$ represent a hydrogen atom and n is 1.

6. Process according to any one of the Claims 1 and 3 to 5, characterized in that at the start, a compound with the formula (III) is used, in which $R_1$ represents a hydrocarbon radical containing from 1 to 18 carbon atoms and containing at least one nitrogen atom.

7. Process according to Claim 6, characterized in that at the start, a compound with the formula (III) is used, in which $R_1$ is chosen from the group constituted by a primary, secondary or tertiary alkyl radical, containing from 1 to 8 carbon atoms, including one or more hetero-atoms chosen from the group constituted by oxygen, nitrogen and sulphur, of which at least one is a nitrogen atom or substituted by a heterocycle including at least one nitrogen atom, a heterocyclic radical including at least one nitrogen atom, possibly substituted by an alkyl radical containing from 1 to 8 carbon atoms, an aryl or an aralkyl radical carrying an amine function

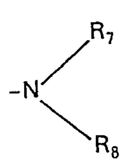 ,

in which $R_7$ and $R_8$ represent an alkyl radical containing from 1 to 8 carbon atoms or a primary, secondary or tertiary alkyl radical containing from 1 to 8 carbon atoms, including one or more heteroatoms chosen from the group constituted by oxygen, nitrogen and sulphur, of which at least one is a nitrogen atom, or substituted by a heterocycle including at least one nitrogen atom, a 2-, 3- or 4-pyridine radical,

a 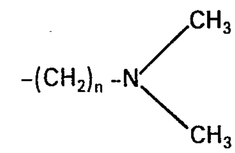 radical, (n⩾3),

a radical: 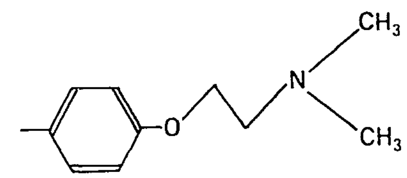

a radical: 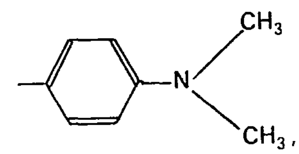

a radical: 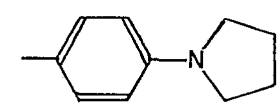 or

a radical: 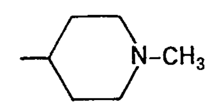

8. Process according to any one of the Claims 1 to 7, characterized in that any one of the compounds with the formula (II) is prepared, the names of which follow:

- 11β-[4-(trimethylsilyl)-phenyl]-3,3-[1,2-ethanediyl-bis(oxy)]-17α-(prop-1-ynyl)-estr-9-en-5α,17β-diol,
- 11β-(4-pyridyl)-3,3-[1,2-ethanediyl-bis(oxy)]-17α-(prop-1-ynyl)-estr-9-en-5α,17β-diol,
- 11β-[3-(N,N-dimethylamino)-propyl]-3,3-[1,2-ethanediyl-bis-(oxy)]-17α-(prop-1-ynyl)-estr-9-en-5α,17β-diol,
- 11β-(4-dimethylaminophenyl)-3,3-[1,2-ethanediyl-bis(oxy)]-17α-(prop-1-ynyl)-estr-9-en-5α,17β-diol,
- 3,3-[ethanediyl-bis(oxy)]-11β-[4-(N,N-dimethylaminoethyloxy)-phenyl]-17α-(prop-1-ynyl)-estr-9-en-5α,17β-diol,
- 21-chloro-3,3-[1,2-ethanediyl-bis(oxy)]-11β-(4-dimethylaminophenyl)-(17α)-19-nor-pregn-9-en-20-yn-5α,17β-diol,
- 11β-(4-dimethylaminophenyl)-3,3-[1,2-ethanediyl-bis(oxy)]-17α-(prop-2-ynyl)-estr-9-en-5α,17β-diol.

9. Preparation process for a product with the formula (V):

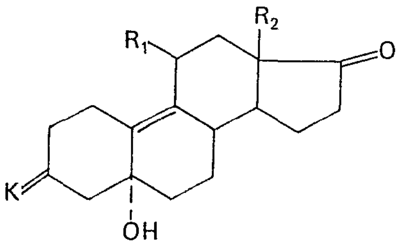

(V)

in which K and $R_2$ retain the same significance as in Claim 1 and $R_1$ represents a radical:

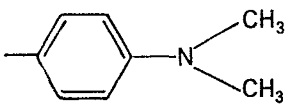

characterized in that a product with the formula (IV):

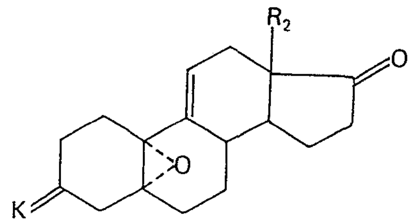

(IV)

is submitted to the action of a product with the formula

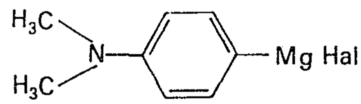

in which Hal is defined as in Claim 1.